# FASCICULE DE BREVET EUROPEEN

(11) **EP 3 233 806 B1**
(45) Date de publication et mention de la délivrance du brevet: **27.10.2021**
(21) Numéro de dépôt: 15832885.6
(22) Date de dépôt: 17.12.2015
(51) Int. Cl.: C07D 231/12, C07D 333/24, C07D 233/64, C07D 249/08, C07D 261/08, C07D 263/32, C07D 271/06, C07D 271/10, C07D 207/337, C07D 277/30, C07D 285/12, A61K 8/49, A61Q 19/00, A61K 31/381, A61K 31/40, A61K 31/4164, A61K 31/421, A61K 31/4245, A61P 17/00, A61P 35/00

(54) **COMPOSÉS HÉTÉROCYCLIQUES, LEUR PROCÉDÉ DE SYNTHÈSE ET LEUR UTILISATION EN MÉDECINE AINSI QU'EN COSMÉTIQUE**
HETEROCYCLISCHE VERBINDUNGEN, VERFAHREN ZU IHRER HERSTELLUNG UND VERWENDUNG DAVON IN DER MEDIZIN UND KOSMETIK
HETEROCYCLIC COMPOUNDS, PROCESS FOR THEIR PREPARATION AND USE THEREOF IN MEDICINE AS WELL AS IN COSMETICS

(30) Priorité: 19.12.2014 FR 1463033
(43) Date de publication de la demande: 25.10.2017
(73) Titulaire: Galderma Research & Development, 06410 Biot (FR)
(72) Inventeur: PORTAL, Thibaud, 06650 Opio (FR)
(74) Mandataire: Cabinet Becker et Associés
(86) Numéro de dépôt international: PCT/FR2015/053581
(87) Numéro de publication internationale: WO 2016/097626

(56) Documents cités:
- EP-A1- 1 092 711
- EP-A2- 0 382 077
- WO-A1-98/08830
- US-A1- 2011 275 823
- GARATTINI E ET AL: "ST1926, a novel and orally active retinoid-related molecule inducing apoptosis in myeloid leukemia cells: modulation of intracellular calcium homeostasis", BLOOD, AMERICAN SOCIETY OF HEMATOLOGY, US, vol. 103, no. 1, 1 janvier 2004 (2004-01-01), pages 194-207, XP002498487, ISSN: 0006-4971, DOI: 10.1182/BLOOD-2003-05-1577 [extrait le 2003-09-04]
- DANIELE SIMONI ET AL.: "Novel terphenyls and 3,5-diaryl isoxazole derivatives endowed with growth supporting and antiapoptotic properties", JOURNAL OF MEDICINAL CHEMISTRY., vol. 51, no. 15, 7 décembre 2008 (2008-12-07), pages 4796-4803, XP002756731, USAMERICAN CHEMICAL SOCIETY. WASHINGTON. ISSN: 0022-2623
- GARCÍA-RODRÍGUEZ JOSÉ ET AL: "Inhibition of I[kappa]B kinase-[beta] and I[kappa]B kinase-[alpha] by heterocyclic adam", BIOORGANIC & MEDICINAL CHEMISTRY, vol. 22, no. 4, 10 janvier 2014 (2014-01-10), pages 1285-1302, XP028606349, ISSN: 0968-0896, DOI: 10.1016/J.BMC.2014.01.006
- DUCHET L ET AL: "Synthesis of 3,5-disubstituted 1,2,4-oxadiazoles using ionic liquid-phase organic synthesis (IoLiPOS) methodology", TETRAHEDRON, ELSEVIER SCIENCE PUBLISHERS, AMSTERDAM, NL, vol. 66, no. 4, 23 janvier 2010 (2010-01-23), pages 986-994, XP026815260, ISSN: 0040-4020 [extrait le 2009-11-24]
- CHIHIRO M ET AL: "NOVEL THIAZOLE DERIVATIVES AS INHIBITORS OF SUPEROXIDE PRODUCTION BY HUMAN NEUTROPHILS: SYNTHESIS AND STRUCTURE-ACTIVITY RELATIONSHIPS", JOURNAL OF MEDICINAL CHEMISTRY, AMERICAN CHEMICAL SOCIETY, US, vol. 38, no. 2, 1 janvier 1995 (1995-01-01), pages 353-358, XP002024855, ISSN: 0022-2623, DOI: 10.1021/JM00002A017
- YOSHIZUMI T ET AL: "Synthesis of 2,5-diaryloxazoles through van Leusen reaction and copper-mediated direct arylation", TETRAHEDRON LETTERS, PERGAMON, GB, vol. 50, no. 26, 1 juillet 2009 (2009-07-01), pages 3273-3276, XP026120479, ISSN: 0040-4039, DOI: 10.1016/J.TETLET.2009.02.039 [extrait le 2009-02-10]
- ALI RAOOF ET AL.: "Toxoflavins and deazaflavins as the first reported selective small molecule inhibitors of tyrosyl-DNA Phosphodiesterase II", JOURNAL OF MEDICINAL CHEMISTRY., vol. 56, no. 16, 2013, pages 6352-6370, XP002756732, USAMERICAN CHEMICAL SOCIETY. WASHINGTON. ISSN: 0022-2623

## Description

### DOMAINE TECHNIQUE

L'invention se rapporte, à titre de produits industriels nouveaux et utiles, à de nouveaux composés, ligands modulateurs des récepteurs RARs. Elle se rapporte également à des compositions les contenant, leurs procédés de préparation et à leur utilisation dans des compositions pharmaceutiques destinées à un usage en médecine humaine ou vétérinaire, ou bien encore dans des compositions cosmétiques et à l'utilisation non thérapeutique de ces dernières.

Les composés ayant une activité de type rétinoïde (vitamine A et ses dérivés) sont largement décrits dans la littérature, comme ayant des activités dans les processus de prolifération et différentiation cellulaire. Ces propriétés confèrent à cette classe de composés, un fort potentiel dans le traitement ou la prévention de nombreuses pathologies, et plus particulièrement en dermatologie et les cancers. Beaucoup d'effets biologiques des rétinoïdes sont médiés par la modulation des récepteurs nucléaires de l'acide rétinoique (RAR).

Les récepteurs RARs activent la transcription en se liant à des éléments de séquences d'ADN, appelés les éléments de réponse des RAR Element (RARE), sous forme d'un hétérodimère avec les récepteurs X des rétinoïdes (appelés les RXRs).

Trois sous-types de RARs humains ont été identifiés et décrits : les RARα, RARβ et RARγ.

L'art antérieur contient un grand nombre de composés chimiques ligands des récepteurs de type RAR. Parmi les documents de l'art antérieur, on peut citer par exemple le brevet US 6,150,413 qui décrit des composés triaromatiques, le brevet US 6,214,878 qui décrit des composés stilbéniques, ou encore le brevet US 6,218,128 qui décrit une famille de molécules bicycliques ou tricycliques. EP 1 092 711 qui décrit des régulateurs des récepteurs de l'acide rétinoïque comprenant un dérivé 1,3-azole. Garattini et al. décrivent des modulateurs de l'acide rétinoïque comprenant un cycle isoxazolyle. EP 0 382 077 décrit des composés pouvant présenter une activité de type rétinoïde, en particulier le composé 58 correspondant à un dérivé oxadiazole.

La Demanderesse a inventé de nouveaux composés modulateurs des récepteurs de l'acide rétinoique. La portée de la présente invention est définie dans les revendications 1-11.

Ainsi, la présente invention se rapporte à de nouveaux composés répondant à la formule générale (I) suivante, 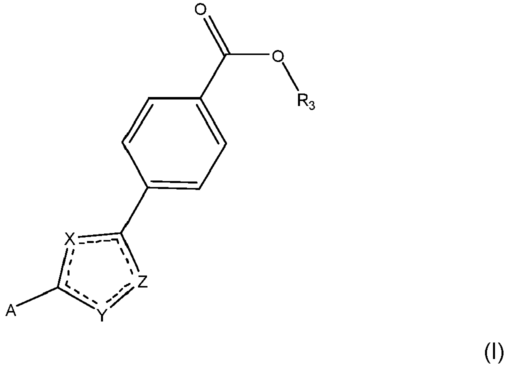 ainsi qu'à leur procédé de synthèse et à leur utilisation dans des compositions pharmaceutiques destinées à un usage en médecine humaine ou vétérinaire, ou éventuellement en cosmétique.

Les composés de la présente invention agissent comme modulateurs des différents sous-types des récepteurs nucléaires de l'acide rétinoïque (RAR).

Ainsi, la présente invention concerne des composés de formule (I) 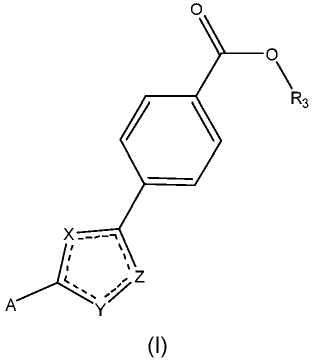 dans laquelle:
A représente 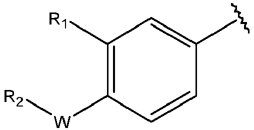
R1 représente un radical alkyle ramifié, ou un radical NR₄R₅;
R2 est un atome d'hydrogène, un radical alkyle linéaire ou ramifié, un radical monohydroxyalkyle ;
R3 est un hydrogène ;
R4 et R5, identiques ou différents, représentent un radical alkyle linéaire ou ramifié ;
R4 et R5, pris ensemble, pouvant également être liés et former un hétérocycle de type pyrrolidine avec l'atome d'azote auquel ils sont liés, cet hétérocycle pouvant éventuellement être substitué ;
W représente O, S, NH ou CH₂ ;
X, Y, Z, identiques ou différents représentent O, ou N ;
l'hétérocycle central 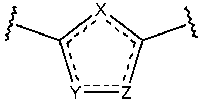 correspond à l'une des structures présentées ci-dessous, les liaisons en pointillée pouvant correspondre à une simple ou double liaison en fonction de la nature des atomes X, Y et Z et de leur covalence 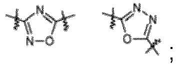
ainsi que les sels d'addition des composés de formule générale (I) avec un acide pharmaceutiquement acceptable, les sels d'addition des composés de formule générale (I) avec une base pharmaceutiquement acceptable et les énantiomères des composés de formule générale (I).

Parmi les sels d'addition des composés de formule générale (I) avec un acide pharmaceutiquement acceptable, on peut citer de préférence les sels avec un acide organique ou avec un acide inorganique.

Les acides inorganiques appropriés sont par exemple les acides halohydriques (par exemple l'acide chlorhydrique et l'acide bromhydrique), l'acide sulfurique, l'acide nitrique et l'acide phosphorique.

Les acides organiques appropriés sont par exemple l'acide acétique, l'acide trifluoroacétique, l'acide trichloroacétique, l'acide propionique, l'acide glycolique, l'acide pyruvique, l'acide succinique, l'acide benzoïque, l'acide cinnamique, l'acide mandélique, l'acide méthanesulfonique, l'acide para-toluène sulfonique, l'acide salicylique, l'acide picrique, l'acide citrique, l'acide oxalique, l'acide tartrique, l'acide malonique, l'acide maléique, l'acide camphorsulfonique et l'acide fumarique.

Parmi les sels d'addition des composés de formule générale (I) avec une base pharmaceutiquement acceptable, on peut citer de préférence les sels avec une base organique ou avec une base inorganique.

Les bases inorganiques appropriées comprennent les hydroxydes, les carbonates et les bicarbonates de métaux alcalins ou de métaux alcalino-terreux. Parmi ces bases, on peut citer par exemple l'hydroxyde de potassium, l'hydroxyde de sodium, l'hydroxyde de lithium, l'hydroxyde de calcium, le carbonate de potassium, le carbonate de sodium, le carbonate de lithium, le carbonate de calcium, le bicarbonate de potassium, le bicarbonate de sodium, le bicarbonate de lithium ou encore le bicarbonate de calcium.

Les bases organiques appropriés comprennent les amines et les amino-acides. Parmi les amines, on peut citer par exemple les amines primaires, secondaires ou tertiaires aliphatiques ou aromatiques comme la methylamine, l'ethylamine, l'éthanolamine, la propylamine, l'isopropylamine, les 4 isomères de la butylamine, la diméthylamine, diethylamine, la diethanolamine, la dipropylamine, la diisopropylamine, la di n-butylamine, la pyrrolidine, la piperidine, la morpholine, la diethanol phénylamine, la trimethylamine, la triethylamine, la tripropylamine, la quinuclidine, la pyridine, la quinoline ou l'isoquinoline.

Parmi les amino-acides, on peut citer par exemple la lysine, l'arginine et l'ornithine.

Selon la présente invention, on désigne par radical alkyle une chaîne hydrocarbonée saturée, linéaire ou ramifiée, comprenant de 1 à 10 atomes de carbone.

Selon la présente invention, on désigne par radical alkyle fluoré, un radical alkyle pour lequel un ou plusieurs atomes d'hydrogène sont remplacés par des atomes de fluor.

Selon la présente invention, on désigne par radical alkényle, une chaîne hydrocarbonée insaturée, linéaire ou ramifiée comprenant de 2 à 10 atomes de carbone et comprenant une ou plusieurs doubles liaisons.

Selon la présente invention, on désigne par radical alkynyle, une chaîne hydrocarbonée insaturée, linéaire ou ramifiée comprenant de 2 à 10 atomes de carbone et comprenant une ou plusieurs triples liaisons.

Selon la présente invention, on désigne par radical alkyle substitué une chaîne hydrocarbonée saturée, linéaire ou ramifiée, comprenant de 1 à 10 atomes de carbone et substitué par un ou plusieurs radicaux choisis parmi un atome d'halogène, un radical amino, un radical dialkylamino, un radical amide, un radical alkoxyle, un radical hétérocycloalkyle, un radical hydroxyle, un radical silylé, un radical alkylcarbamoyl.

Selon la présente invention, on désigne par radical alkényle substitué une chaîne hydrocarbonée insaturée, linéaire ou ramifiée, comprenant de 2 à 10 atomes de carbone, comprenant une ou plusieurs doubles liaisons et substituée par un ou plusieurs radicaux choisis parmi un atome d'halogène, un radical amino, un radical alkoxyle et un radical hydroxyle.

Selon la présente invention, on désigne par radical alkynyle substitué une chaîne hydrocarbonée insaturée, linéaire ou ramifiée, comprenant de 2 à 10 atomes de carbone, comprenant une ou plusieurs triples liaisons et substituée par un ou plusieurs radicaux choisis parmi un atome d'halogène, un radical alkoxyle, un radical amino et un radical hydroxyle.

Selon la présente invention, on désigne par cycloalkyle une chaine hydrocarbonée, saturée, cyclique comprenant de 3 à 7 atomes de carbones.

Selon la présente invention, on désigne par cycloalkyle substitué une chaine hydrocarbonée, saturée, cyclique comprenant de 3 à 7 atomes de carbones et substituée par un ou plusieurs radicaux choisis parmi un atome d'halogène, un radical alkyle, un radical alkoxyle, un radical hydroxyle ou un radical amino.

Selon la présente invention, on désigne par cycloalkyle-alkyle, un alkyle substitué par un cycloalkyle.

Selon la présente invention, on désigne par radical aryle, un cycle hydrocarboné aromatique ou deux cycles fusionnés hydrocarbonés aromatiques.

Selon la présente invention, on désigne par radical aryle substitué, un cycle ou deux cycles fusionnés hydrocarboné(s) aromatique(s) substitué(s) par un ou plusieurs groupes d'atomes choisis parmi un alkyle, un alkoxyle, un aryle, un halogène, un hydroxyle, un cyano, un trifluorométhyle, un groupement amine substituée ou non substituée et un nitro.

Selon la présente invention, on désigne par radical aralkyle, un alkyle substitué par un aryle. Selon la présente invention, on désigne par radical aralkyle substitué, un alkyle substitué par un aryle substitué.

Selon la présente invention, on désigne par radical hétérocyclique une chaîne hydrocarbonée cyclique ou polycyclique, saturée ou insaturée, comprenant un ou plusieurs hétéroatomes choisis parmi O, S et N.

Selon la présente invention, on désigne par radical hétérocyclique substitué, un radical hétérocyclique substitué par un ou plusieurs groupes d'atomes choisis parmi un alkyle, un alkoxyle, un halogène, un hydroxyle, un cyano, un trifluorométhyle et un nitro.

Selon la présente invention, on désigne par radical hétéroaryle, un radical hétérocyclique aromatique c'est-à-dire une chaîne hydrocarbonée cyclique ou polycyclique, aromatique, comprenant un ou plusieurs hétéroatomes choisis parmi O, S et N.

Selon la présente invention, on désigne par radical hétéroaryle substitué, un radical hétéroaryle substitué par un ou plusieurs groupes d'atomes choisis par exemple parmi un alkyle, un alkoxy, un aryle, un aryle substitué, un halogène, un hydroxy, un cyano, un trifluorométhyle et un nitro.

Selon la présente invention, on désigne par radical hétéroaralkyle, un radical alkyle substitué par un radical hétéroaryle.

Selon la présente invention, on désigne par radical hétéroaralkyle substitué, un radical hétéroaralkyle substitué par un ou plusieurs groupes d'atomes choisis parmi un alkyle, un alkoxyle, un halogène, un hydroxyle, un cyano, un trifluorométhyle et un nitro.

Selon la présente invention, on désigne par radical alkoxyle un atome d'oxygène substitué par un radical alkyle.

Selon la présente invention, on désigne par atome d'halogène un atome de fluor, de chlore, de brome ou d'iode.

Parmi les composés de formule générale (I), on peut notamment citer les composés suivants :
acide 4-[3-(3-adamantan-1-yl-4-éthoxyméthoxy-phényl)-[1,2,4]oxadiazol-5-yl]-benzoique
acide 4-[5-(5,5,8,8-tétramethyl-5,6,7,8-tetrahydro-naphthalen-2-yl)-thiazol-2-yl]-benzoique
acide 4-[5-(3-adamantan-1-yl-4-éthoxyméthoxy-phényl)-thiazol-2-yl]-benzoique
acide 4-{5-[3-adamantan-1-yl-4-(2-méthoxy-éthoxyméthoxy)-phényl]-[1,3,4]oxadiazol-2-yl}-benzoique
acide 4-[5-(3-adamantan-1-yl-4-hydroxy-phényl)-thiazol-2-yl]-benzoique
acide 4-[3-(3-adamantan-1-yl-4-hydroxy-phényl)-[1,2,4]oxadiazol-5-yl]-benzoique
acide 4-{5-[3-adamantan-1-yl-4-(2-methoxy-ethoxy)-phényl]-thiazol-2-yl}-benzoique
acide 4-[5-(3-tert-butyl-4-hydroxy-phényl)-thiazol-2-yl]-benzoique
acide 4-[5-(3-adamantan-1-yl-4-méthoxy-phényl)-thiazol-2-yl]-benzoique
acide 4-[5-(3-adamantan-1-yl-4-isobutoxy-phényl)-thiazol-2-yl]-benzoique
acide 4-[5-(3-adamantan-1-yl-4-propoxy-phényl)-thiazol-2-yl]-benzoique
acide 4-[5-(3-adamantan-1-yl-4-éthoxy-phényl)-thiazol-2-yl]-benzoique
acide 4-[5-(3-adamantan-1-yl-4-hydroxy-phényl)-[1,3,4]oxadiazol-2-yl]-benzoique
acide 4-[5-(3-adamantan-1-yl-4-isobutoxy-phényl)-[1,3,4]oxadiazol-2-yl]-benzoique
acide 4-{3-[3-adamantan-1-yl-4-(2-méthoxy-éthoxy)-phényl]-[1,2,4]oxadiazol-5-yl}-benzoique
acide 4-{3-[3-adamantan-1-yl-4-(2-diméthylamino-ethoxy)-phényl]-[1,2,4]oxadiazol-5-yl}-benzoique
acide 4-[3-(3-adamantan-1-yl-4-méthoxy-phényl)-[1,2,4]oxadiazol-5-yl]-benzoique
acide 4-[3-(3-adamantan-1-yl-4-éthoxy-phényl)-[1,2,4]oxadiazol-5-yl]-benzoique
acide 4-[3-(3-adamantan-1-yl-4-propoxy-phényl)-[1,2,4]oxadiazol-5-yl]-benzoique
acide 4-[3-(3-adamantan-1-yl-4-isobutoxy-phényl)-[1,2,4]oxadiazol-5-yl]-benzoique
acide 4-{3-[3-adamantan-1-yl-4-(2,2-diméthoxy-éthoxy)-phényl]-[1,2,4]oxadiazol-5-yl}-benzoique
acide 4-[3-(3-tert-butyl-4-éthoxyméthoxy-phényl)-[1,2,4]oxadiazol-5-yl]-benzoique
acide 4-{5-[4-(2-méthoxy-éthoxyméthoxy)-3-(1-méthyl-cyclohexyl)-phényl]-thiazol-2-yl}-benzoique
acide 4-[5-(3-adamantan-1-yl-4-méthoxy-phényl)-[1,3,4]oxadiazol-2-yl]-benzoique
acide 4-[5-(3-adamantan-1-yl-4-éthoxy-phényl)-[1,3,4]oxadiazol-2-yl]-benzoique
acide 4-[5-(3-adamantan-1-yl-4-cyclopropylméthoxy-phényl)-[1,3,4]oxadiazol-2-yl]-benzoique
acide 4-{5-[3-adamantan-1-yl-4-(2,2,2-trifluoro-ethoxy)-phényl]-[1,3,4]oxadiazol-2-yl}-benzoique
acide 4-[5-(3-adamantan-1-yl-4-propoxy-phényl)-[1,3,4]oxadiazol-2-yl]-benzoique
acide 4-[3-(3-tert-butyl-4-hydroxy-phényl)-[1,2,4]oxadiazol-5-yl]-benzoique
acide 4-{3-[4-éthoxyméthoxy-3-(1-méthyl-cyclohexyl)-phényl]-[1,2,4]oxadiazol-5-yl}-benzoique
acide 4-{3-[4-hydroxy-3-(1-méthyl-cyclohexyl)-phényl]-[1,2,4]oxadiazol-5-yl}-benzoique
acide 4-{3-[3-tert-butyl-4-(2-méthoxy-éthoxy)-phényl]-[1,2,4]oxadiazol-5-yl}-benzoique
acide 4-{3-[3-tert-butyl-4-(2-diméthylamino-éthoxy)-phényl]-[1,2,4]oxadiazol-5-yl}-benzoique
acide 4-[3-(3-tert-butyl-4-méthoxy-phényl)-[1,2,4]oxadiazol-5-yl]-benzoique
acide 4-[3-(3-tert-butyl-4-éthoxy-phényl)-[1,2,4]oxadiazol-5-yl]-benzoique
acide 4-[3-(3-tert-butyl-4-propoxy-phényl)-[1,2,4]oxadiazol-5-yl]-benzoique
acide 4-[3-(3-tert-butyl-4-isobutoxy-phényl)-[1,2,4]oxadiazol-5-yl]-benzoique
acide 4-{3-[3-tert-butyl-4-(2,2,2-trifluoro-ethoxy)-phényl]-[1,2,4]oxadiazol-5-yl}-benzoique
acide 4-[3-(3-tert-butyl-4-cyclopropylmethoxy-phényl)-[1,2,4]oxadiazol-5-yl]-benzoique
acide 4-{3-[3-tert-Butyl-4-(2,2-dimethoxy-éthoxy)-phényl]-[1,2,4]oxadiazol-5-yl}-benzoique
acide 4-{3-[3-tert-butyl-4-([1,3]dioxolan-2-ylméthoxy)-phényl]-[1,2,4]oxadiazol-5-yl}-benzoique
acide 4-{5-[4-hydroxy-3-(1-méthyl-cyclohexyl)-phényl]-thiazol-2-yl}-benzoique
acide 4-{5-[4-hydroxy-3-(1-méthyl-cyclohexyl)-phényl]-thiazol-2-yl}-benzoique
acide 4-{5-[4-hydroxy-3-(1-méthyl-cyclohexyl)-phényl]-thiazol-2-yl}-benzoique
acide 4-{5-[4-isobutoxy-3-(1-méthyl-cyclohexyl)-phényl]-thiazol-2-yl}-benzoique
acide 4-{5-[4-méthoxy-3-(1-méthyl-cyclohexyl)-phényl]-thiazol-2-yl}-benzoique
acide 4-{5-[3-(1-méthyl-cyclohexyl)-4-propoxy-phényl]-thiazol-2-yl}-benzoique
acide 4-{5-[4-cyclopropylméthoxy-3-(1-méthyl-cyclohexyl)-phényl]-thiazol-2-yl}-benzoique
acide 4-{5-[4-éthylcarbamoylméthoxy-3-(1-méthyl-cyclohexyl)-phényl]-thiazol-2-yl}-benzoique
acide 4-[3-(3-adamantan-1-yl-4-triméthylsilanylméthoxy-phényl)-[1,2,4]oxadiazol-5-yl]-benzoique
acide 4-{5-[3-adamantan-1-yl-4-(2,2-diméthoxy-éthoxy)-phényl]-[1,3,4]oxadiazol-2-yl}-benzoique
acide 4-{5-[3-adamantan-1-yl-4-(2-méthoxy-éthoxyméthoxy)-phényl]-4H-pyrazol-3-yl}-benzoique
acide 4-[5-(3-adamantan-1-yl-4-propylamino-phényl)-thiazol-2-yl]-benzoique
acide 4-[5-(3-tert-butyl-4-méthoxy-phényl)-thiazol-2-yl]-benzoique
acide 4-[5-(3-tert-butyl-4-éthoxy-phényl)-thiazol-2-yl]-benzoique
acide 4-[5-(3-tert-butyl-4-propoxy-phényl)-thiazol-2-yl]-benzoique
acide 4-{5-[3-tert-butyl-4-(2,2,2-trifluoro-éthoxy)-phényl]-thiazol-2-yl}-benzoique
acide 4-[5-(3-tert-butyl-4-cyclopropylméthoxy-phényl)-thiazol-2-yl]-benzoique
acide 4-{5-[3-tert-butyl-4-(2-methoxy-ethoxy)-phényl]-thiazol-2-yl}-benzoique
acide 4-{5-[3-tert-butyl-4-(2,2-diméthoxy-éthoxy)-phényl]-thiazol-2-yl}-benzoique
acide 4-{5-[3-(1-méthyl-cyclohexyl)-4-(2,2,2-trifluoro-éthoxy)-phényl]-thiazol-2-yl}-benzoique
acide 4-{3-[4-méthoxy-3-(1-methyl-cyclohexyl)-phényl]-[1,2,4]oxadiazol-5-yl}-benzoique
acide 4-{3-[3-(1-méthyl-cyclohexyl)-4-propoxy-phényl]-[1,2,4]oxadiazol-5-yl}-benzoique
acide 4-{3-[4-isobutoxy-3-(1-methyl-cyclohexyl)-phényl]-[1,2,4]oxadiazol-5-yl}-benzoique
acide 4-{3-[4-cyclopropylmethoxy-3-(1-methyl-cyclohexyl)-phényl]-[1,2,4]oxadiazol-5-yl}-benzoique
acide 4-{3-[4-(2,2-diméthoxy-éthoxy)-3-(1-methyl-cyclohexyl)-phényl]-[1,2,4]oxadiazol-5-yl}-benzoique
acide 4-[3-(3-adamantan-1-yl-4-hydroxy-phényl)-isoxazol-5-yl]-benzoique
acide 4-[5-(3-adamantan-1-yl-4-hydroxy-phényl)-4H-pyrazol-3-yl]-benzoique
acide 4-[3-(3-adamantan-1-yl-4-cyclopropylméthoxy-phényl)-isoxazol-5-yl]-benzoique
acide 4-[3-(3-adamantan-1-yl-4-méthoxy-phényl)-isoxazol-5-yl]-benzoique
acide 4-[3-(3-adamantan-1-yl-4-propoxy-phényl)-isoxazol-5-yl]-benzoique
acide 4-[3-(3-adamantan-1-yl-4-isobutoxy-phényl)-isoxazol-5-yl]-benzoique
acide 4-{3-[3-adamantan-1-yl-4-(2,2-diméthoxy-éthoxy)-phényl]-isoxazol-5-yl}-benzoique
acide 4-[5-(3-tert-butyl-4-éthylcarbamoylméthoxy-phényl)-thiazol-2-yl]-benzoique
acide 4-[5-(3-tert-butyl-4-isobutoxy-phényl)-thiazol-2-yl]-benzoique
acide 4-[3-(3-adamantan-1-yl-4-éthoxy-phényl)-isoxazol-5-yl]-benzoique
acide 4-{5-[3-adamantan-1-yl-4-([1,3]dioxolan-2-ylméthoxy)-phényl]-[1,3,4]oxadiazol-2-yl}-benzoique
acide 4-{3-[3-(1-méthyl-cyclohexyl)-4-(2,2,2-trifluoro-ethoxy)-phényl]-[1,2,4]oxadiazol-5-yl}-benzoique
acide 4-{3-[4-([1,3]dioxolan-2-ylmethoxy)-3-(1-methyl-cyclohexyl)-phényl]-[1,2,4]oxadiazol-5-yl}-benzoique
acide 4-[5-(3-adamantan-1-yl-4-propylamino-phényl)-thiazol-2-yl]-benzoique
acide 4-{3-[3-tert-butyl-4-(2-hydroxy-éthoxy)-phényl]-[1,2,4]oxadiazol-5-yl}-benzoique
acide 4-{3-[3-tert-butyl-4-(3-hydroxy-propoxy)-phényl]-[1,2,4]oxadiazol-5-yl}-benzoique
acide 4-[5-(3-tert-butyl-4-propoxy-phényl)-1-méthyl-1H-imidazol-2-yl]-benzoique
acide 4-[5-(4-allyloxy-3-tert-butyl-phényl)-[1,3,4]thiadiazol-2-yl]-benzoique
acide 4-{4-[3-(1-méthyl-cyclohexyl)-4-(pyridin-4-ylméthoxy)-phényl]-oxazol-2-yl}-benzoique
acide 4-[5-(4-éthoxy-3-pyrrolidin-1-yl-phényl)-4H-[1,2,4]triazol-3-yl]-benzoique
acide 4-{5-[3-diéthylamino-4-(4-fluoro-benzyloxy)-phényl]-[1,3,4]thiadiazol-2-yl}-benzoique
acide 4-[5-(3-tert-butyl-4-isobutoxy-phényl)-[1,3,4]thiadiazol-2-yl]-benzoique 4-[3-(3-tert-butyl-4-hydroxy-phényl)-[1,2,4]oxadiazol-5-yl]-benzoate de méthyle
acide 4-[5-(4-butoxy-3-tert-butyl-phényl)-1-methyl-1H-pyrrol-2-yl]-benzoique
acide 4-{5-[4-(2-amino-éthoxy)-3-tert-butyl-phényl]-thiophen-2-yl}-benzoique
acide 4-{3-[3-tert-butyl-4-((E)-propenyl)-phényl]-[1,2,4]oxadiazol-5-yl}-benzoique
acide 4-[3-(3-tert-butyl-4-propyl-phényl)-[1,2,4]oxadiazol-5-yl]-benzoique
acide 4-[5-(3-tert-butyl-4-éthylsulfanyl-phényl)-thiazol-2-yl]-benzoique
acide 4-[5-(3-tert-butyl-4-cyclopropylméthylsulfanyl-phényl)-oxazol-2-yl]-benzoique
acide 4-[3-(4-cyclopropylméthoxy-3-pyrrolidin-1-yl-phényl)-[1,2,4]oxadiazol-5-yl]-benzoique
acide 4-[3-(4-hydroxy-3-pyrrolidin-1-yl-phényl)-[1,2,4]oxadiazol-5-yl]-benzoique
acide 4-{3-[4-(2-hydroxy-éthoxy)-3-pyrrolidin-1-yl-phényl]-[1,2,4]oxadiazol-5-yl}-benzoique
acide 3-[5-(3-adamantan-1-yl-4-hydroxy-phényl)-2-éthyl-2H-pyrazol-3-yl]-benzoique
acide 4-{3-[4-diéthylamino-3-(3-hydroxy-propoxy)-phényl]-[1,2,4]oxadiazol-5-yl}-benzoique
acide 4-[3-(4-diéthylamino-3-propoxy-phényl)-isoxazol-5-yl]-benzoique
acide 4-[3-(4-tert-butyl-3-éthylamino-phényl)-[1,2,4]oxadiazol-5-yl]-benzoique
acide 4-{2-[3-(1-méthyl-cyclohexyl)-4-(pyridin-4-ylmethoxy)-phényl]-oxazol-4-yl}-benzoique
acide 4-[2-(3-tert-butyl-4-cyclopropylmethylsulfanyl-phényl)-oxazol-5-yl]-benzoique
acide 4-[5-(3-tert-butyl-4-hydroxy-phenyl)-[1,3,4]oxadiazol-2-yl]-benzoique
acide 4-[5-(3-tert-butyl-4-éthoxy-phenyl)-[1,3,4]oxadiazol-2-yl]-benzoique 4-[5-(4-hydroxy-3-pyrrolidin-1-yl-phényl)-[1,3,4]oxadiazol-2-yl]-benzoique 4-[5-(4-hydroxy-3-pyrrolidin-1-yl-phenyl)-[1,3,4]oxadiazol-2-yl]-benzoate de méthyle 4-[3-(4-tert-butyl-3-éthylamino-phenyl)-[1,2,4]oxadiazol-5-yl]-benzoate de méthyle.

Les composés de formule générale (I) sont préparés selon les schémas réactionnels des figures 1 à 8 présentées ci-après. Le choix du schéma de synthèse utilisé est lié à l'hétérocycle central du composé que l'on souhaite synthétiser.

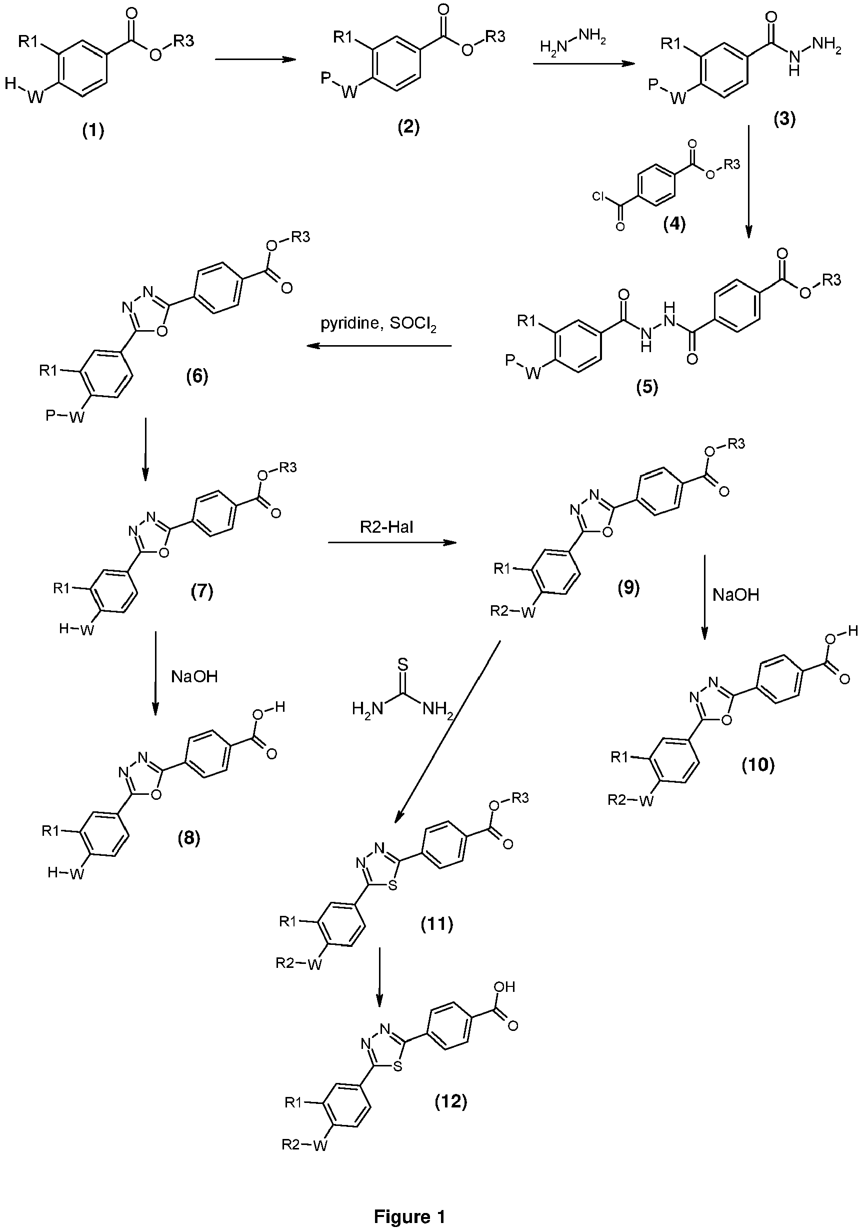

Selon la figure 1, des dérivés 1,2,4-oxadiazoles ou 1,2,4-thiadiazoles peuvent être obtenus. Ainsi, le dérivé hydrazide **(3)** peut être obtenu par réaction de l'ester **(2)** avec de l'hydrazine après une étape de protection dans les cas où W= O ou S. Le choix du groupement protecteur P est réalisé en fonction de la séquence réactionnelle qui suit. Nombreux groupements protecteurs classiquement utilisés comme décrits dans le livre « Protective groups in organic synthesis » (Third edition, authors : Theodora W. Greene & Peter G.M. Wuts, Editor : Wiley InterScience) peuvent être envisagés. Un groupement de type polyéther (ethyl méthyl éther par exemple) peut être envisagé. Par réaction du dérivé hydrazide **(3)** avec le chlorure d'acyle **(4)** (disponible commercialement dans le cas de R3 étant un méthyle), le composé **(5)** est ainsi obtenu. En présence de chlorure de thionyle et pyridine, ce dernier composé se cyclise pour conduire au dérivé oxadiazole **(6)**. Dans le cas de composés avec W=O, S ou N, après une étape de déprotection du groupement P selon les conditions classiques décrites dans le livre « Protective groups in organic chemistry », le composé **(7)** obtenu peut être saponifié pour conduire au dérivé **(8)** ou alkylé en présence d'un halogénure d'alkyle par exemple et d'une base telle que du carbonate de potassium ou par réaction d'amination réductrice lorsque W=N, pour conduire au composé **(9).** Après saponification du composé **(9)** selon des conditions classiques (hydroxyde de sodium aqueux par exemple), le dérivé **(10)** est obtenu. Il est également possible d'obtenir le dérivé thiadiazole **(11)** correspondant par traitement du composé **(9)** en présence de thiourée et de pyridine par exemple. Par saponification du composé **(11),** le dérivé **(12)** peut ainsi être obtenu.

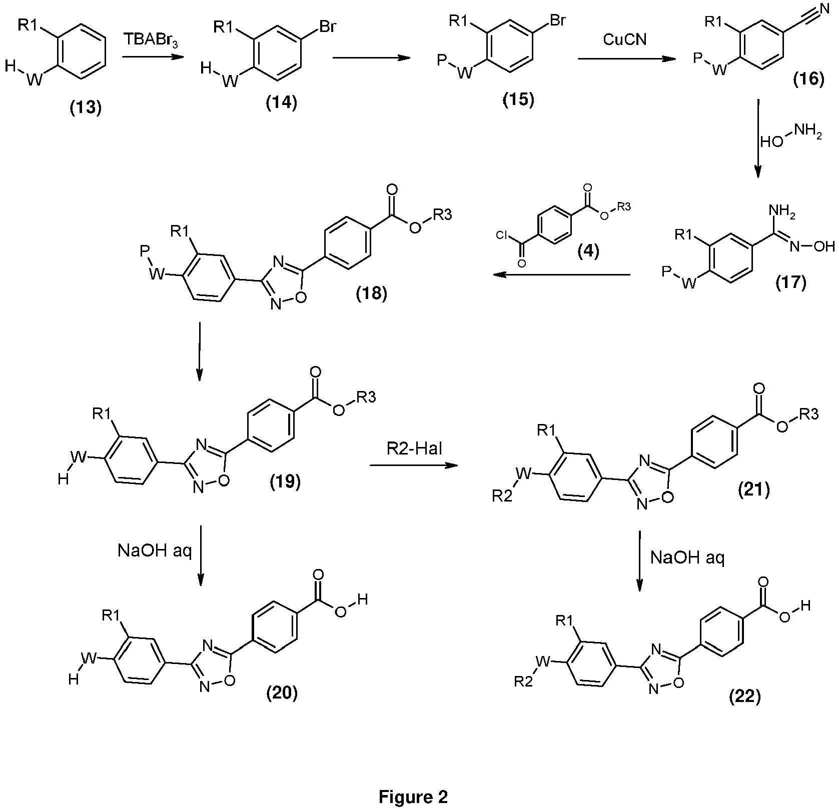

Selon la figure 2, d'autres dérivés oxadiazoles (1,3,4-oxadiazoles) peuvent être préparés. Après bromation des composés **(13)** disponibles commercialement ou préalablement préparés, par du bromure de tétrabutylammonium par exemple, les composés **(14)** obtenus sont protégés par un groupement protecteur de type éthyl-méthyl éther par exemple dans le cas de W= O ou S. Par réaction de substitution par du cyanure de cuivre (I), les composés **(15)** sont obtenus. En présence d'hydroxylamine, ces derniers composés réagissent pour former les dérivés **(17).** La réaction entre le composé **(17)** et le chlorure d'acide **(4)** disponible commercialement permet l'obtention de l'oxadiazole **(18).** Après déprotection de ce dernier lorsque W=O, S, N et obtention du composé **(19),** celui-ci pourra être saponifié pour conduire au composé **(20).** Par alkylation du composé **(19)** en présence d'un halogénure d'alkyle par exemple, et d'une base telle que du carbonate de potassium ou par réaction d'amination réductrice lorsque W=N, le composé **(21)** peut également être obtenu. La saponification de ce dernier conduit au composé acide **(22).**

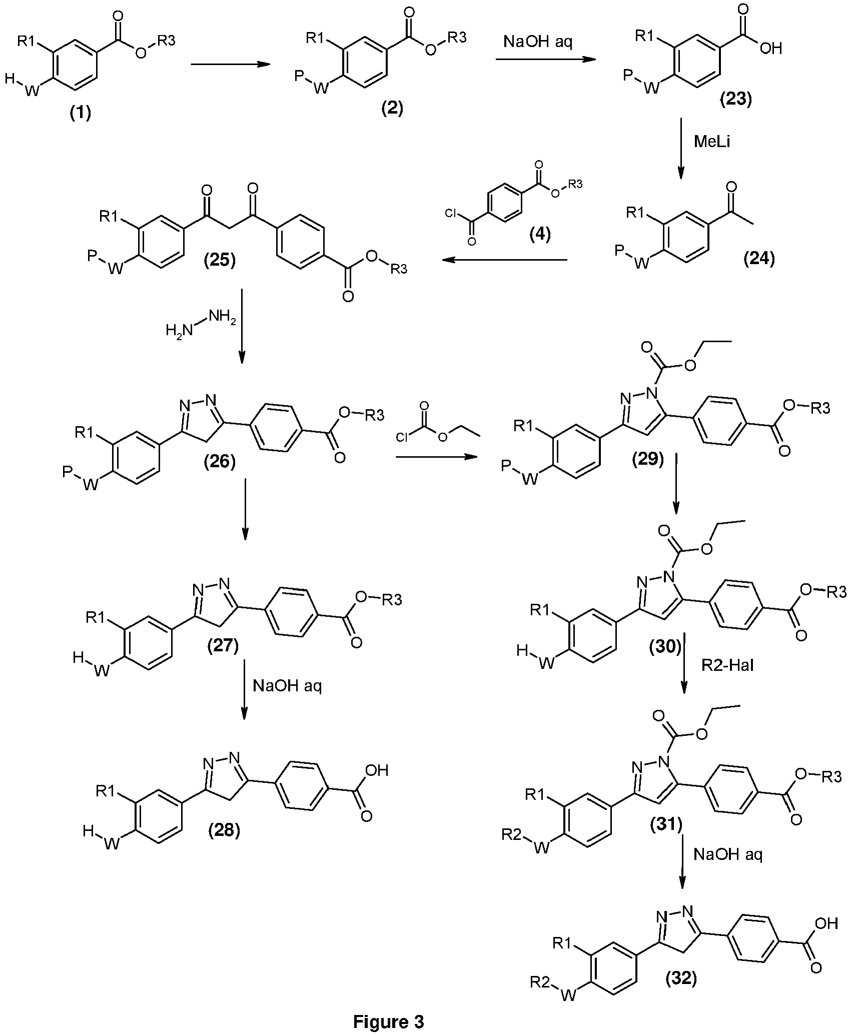

Les dérivés hétérocycliques de type pyrazole peuvent être obtenus selon la figure 3. Après protection des composés pour lesquels W=O ou S et saponification de leur fonction ester, les composés **(23)** sont obtenus. Par réaction du méthyllithium, par exemple, sur les dérivés acides **(23),** la méthylcétone correspondante **(24)** est obtenue. Cette dernière peut réagir avec le chlorure d'acide **(4)** afin de former le dérivé de type 1,3-diphényl-propane-1,3-dione **(25),** qui a son tour, après condensation avec l'hydrazine forme le dérivé diazole **(26).** Une étape de déprotection de ce dernier composé conduit au composé **(27)** qui, après saponification, permet l'obtention du composé **(28).** Une protection au niveau du noyau diazole peut également être envisagée pour obtenir le dérivé **(29).** Ainsi, après déprotection sélective de la fonction phénol, thiophénol ou amine de ce dernier lorsque W=O, S, N suivie d'une alkylation en présence d'un halogénure d'alkyle par exemple, et d'une base telle que du carbonate de potassium ou suivie par une réaction d'amination réductrice, le composé **(31)** peut-être préparé. Par réaction du composé **(31)** avec de l'hydroxyde de sodium aqueux par exemple, le composé **(32)** peut être préparé.

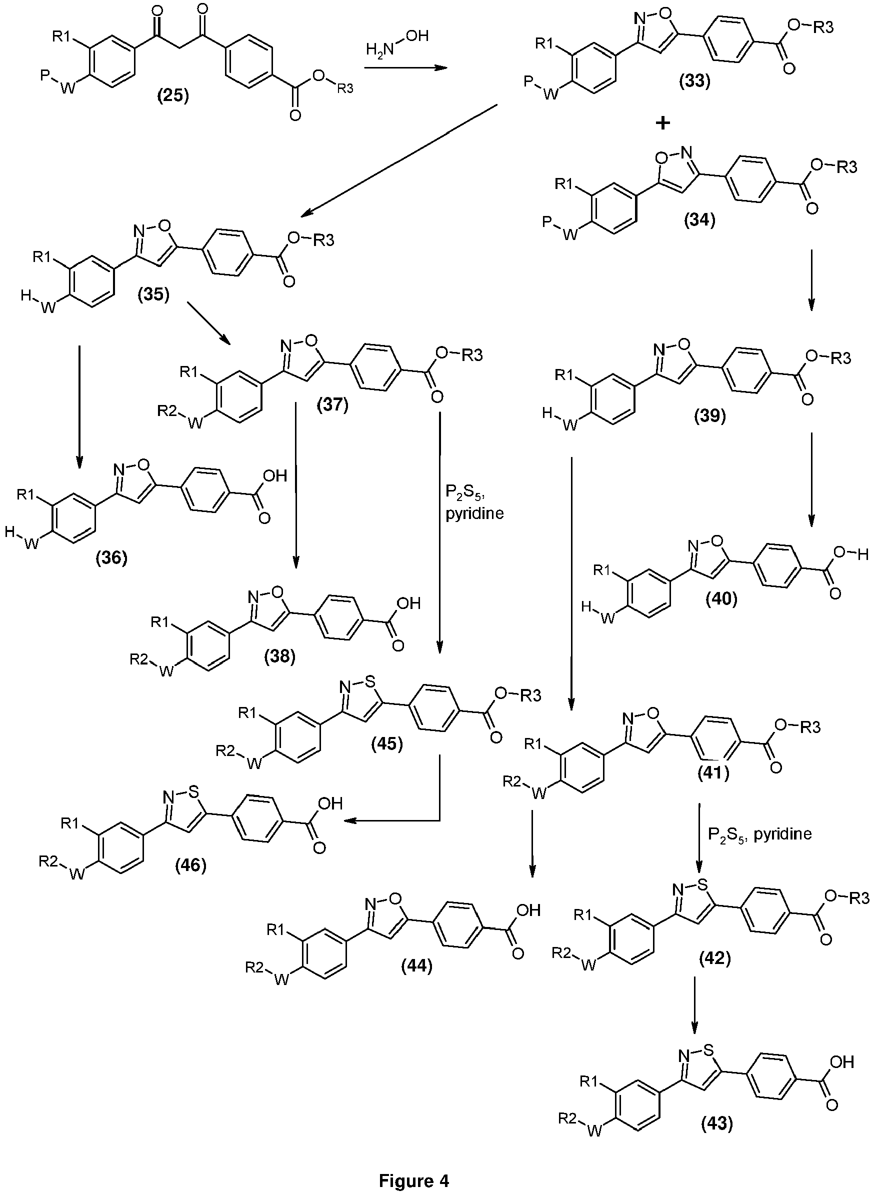

La figure 4 décrit une méthode permettant de préparer des composés isoxazoles. Ainsi, par condensation de l'hydroxylamine sur le dérivé de type 1,3-diphényl-propane-1,3-dione **(25),** préparé comme décrit ci-dessus, deux dérivés oxazoles **(33)** et **(34)** peuvent être obtenus en fonction du sens de la condensation et des conditions opératoires suivies. Une fois ces composés obtenus et séparés, ils pourront respectivement conduire aux dérivés **(35)** et **(39),** après déprotection de leur fonction amino, phénol ou thiophénol. Par saponification des composés **(35)** et **(39)** en présence d'hydroxyde de sodium aqueux par exemple, les composés **(36)** et **(40)** peuvent être préparés. Une alkylation des composés **(35)** et **(39)** en présence d'un halogénure d'alkyle par exemple, et d'une base telle que du carbonate de potassium, suivie d'une étape de saponification permet d'obtenir respectivement les composés **(38)** et **(44).** Par réaction des composés **(37)** et **(41)** (obtenus par alkylation de leurs fonctions phénol ou thiophénol ou par amination réductrice de l'aniline) avec du pentasulfure de diphosphore en présence de pyridine, les dérivés thiazoles correspondants **(45)** et **(42)** peuvent être préparés.

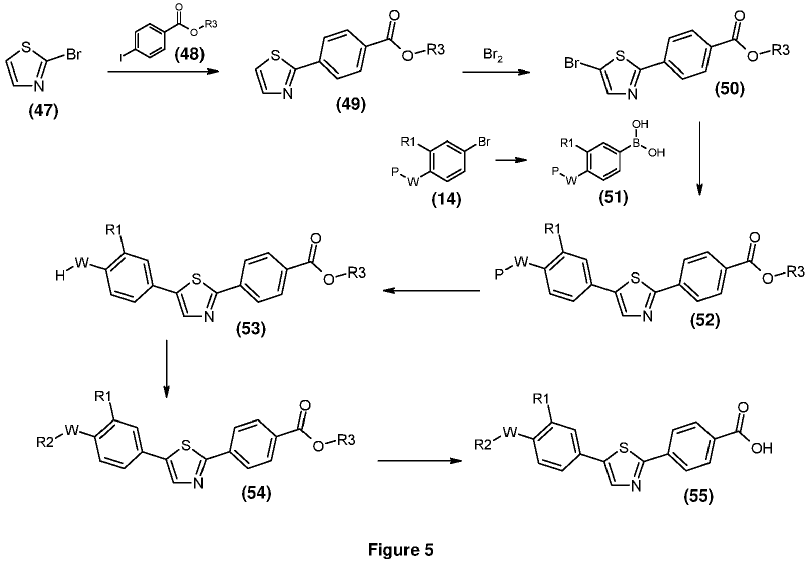

Selon le schéma de synthèse décrit sur la figure 5, des dérivés thiazoles peuvent être obtenus. Par réaction de couplage de type Negishi entre le 2-bromothiazole **(47)** et le dérivé organozincique préalablement préparé à partir du dérivé iodé **(48),** le composé **(49)** est obtenu. Une bromation de ce dernier composé par du brome, par exemple, conduit au composé **(50).** Par couplage de type Suzuki, en présence d'un catalyseur de type tétra(triphénylphosphine)palladium et d'une base telle que du carbonate de potassium aqueux par exemple, entre le dérivé bromé **(50)** et l'acide boronique **(51)** préalablement préparé à partir du composé bromé correspondant **(14),** le composé **(52)** est obtenu. Après déprotection de l'éventuelle fonction amino, phénol ou thiophénol puis alkylation en présence d'un halogénure d'alkyle par exemple, et d'une base telle que du carbonate de potassium ou par amination réductrice de la fonction aniline, le composé **(54)** est obtenu. Une saponification de ce dernier en présence d'hydroxyde de sodium aqueux par exemple conduit au composé **(55).**

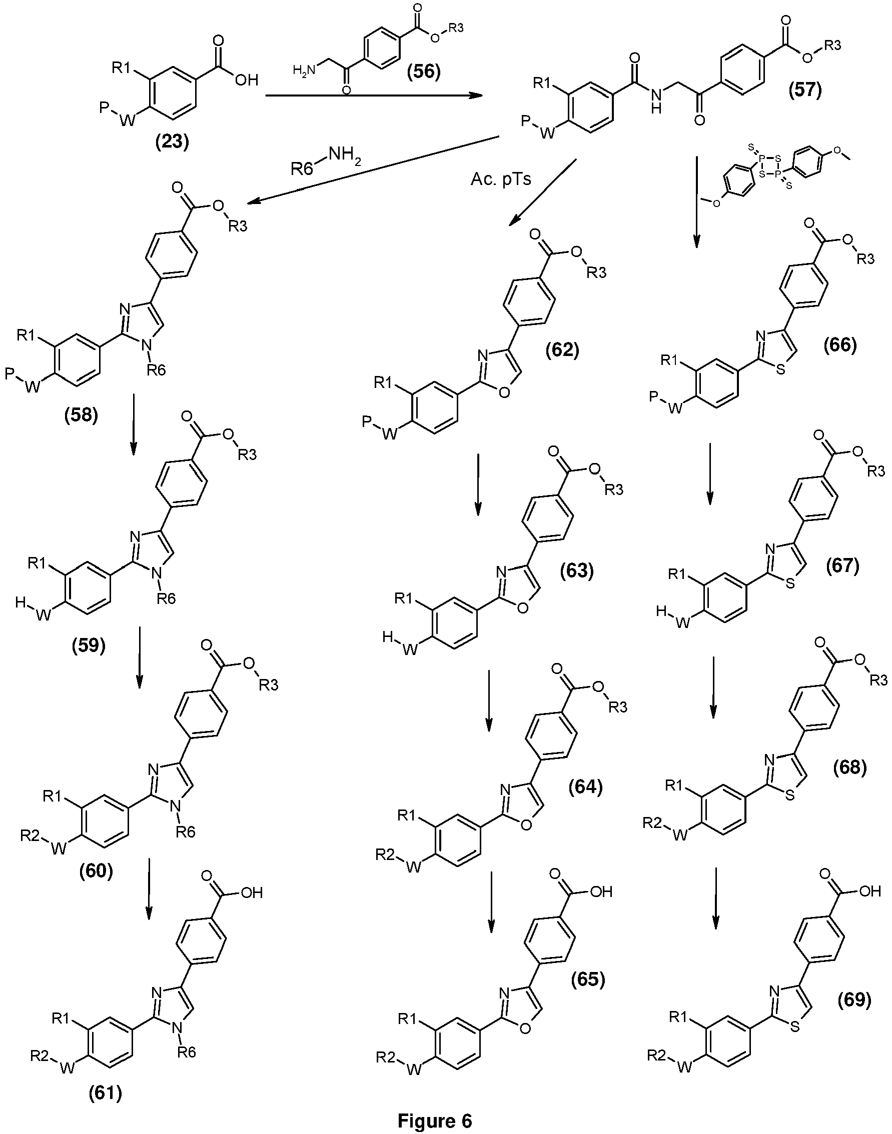

Le schéma de la figure 6 décrit l'obtention de dérivés imidazoles, oxazoles et thiazoles. Ainsi, par réaction de couplage de type peptidique, en présence de 1-hydroxybenzotriazole et du chlorhydrate de 1-(3-diméthylaminopropyl)-3-éthylcarbodiimide par exemple, entre l'acide **(23)** préalablement protégé et l'ester méthylique **(56)** disponible commercialement, le composé **(57)** est obtenu. Une cyclisation de ce dernier en présence d'acide para toluènesulfonique par exemple conduit au dérivé oxazole correspondant **(62),** une cyclisation en présence d'ammoniac ou d'une amine primaire conduit à l'imidazole éventuellement N-alkylé **(58)** et enfin, une cyclisation en présence de 2,4-disulfure de 2,4-bis(4-méthoxyphényl)-1,3-dithia-2,4-diphosphétane (réactif de Lawesson) permet d'obtenir le thiazole correspondant **(66).** Après déprotection de l'éventuelle fonction amino, phénol ou thiophénol (composés **(59), (63)** et **(67),** puis éventuellement une alkylation en présence d'un halogénure d'alkyle par exemple, et d'une base telle que du carbonate de potassium ou par amination réductrice sur la fonction aniline, les composé **(60) (64)** et **(68)** sont obtenus. Une saponification de ces derniers en présence d'hydroxyde de sodium aqueux par exemple conduit aux composés **(61) (65)** et **(69).**

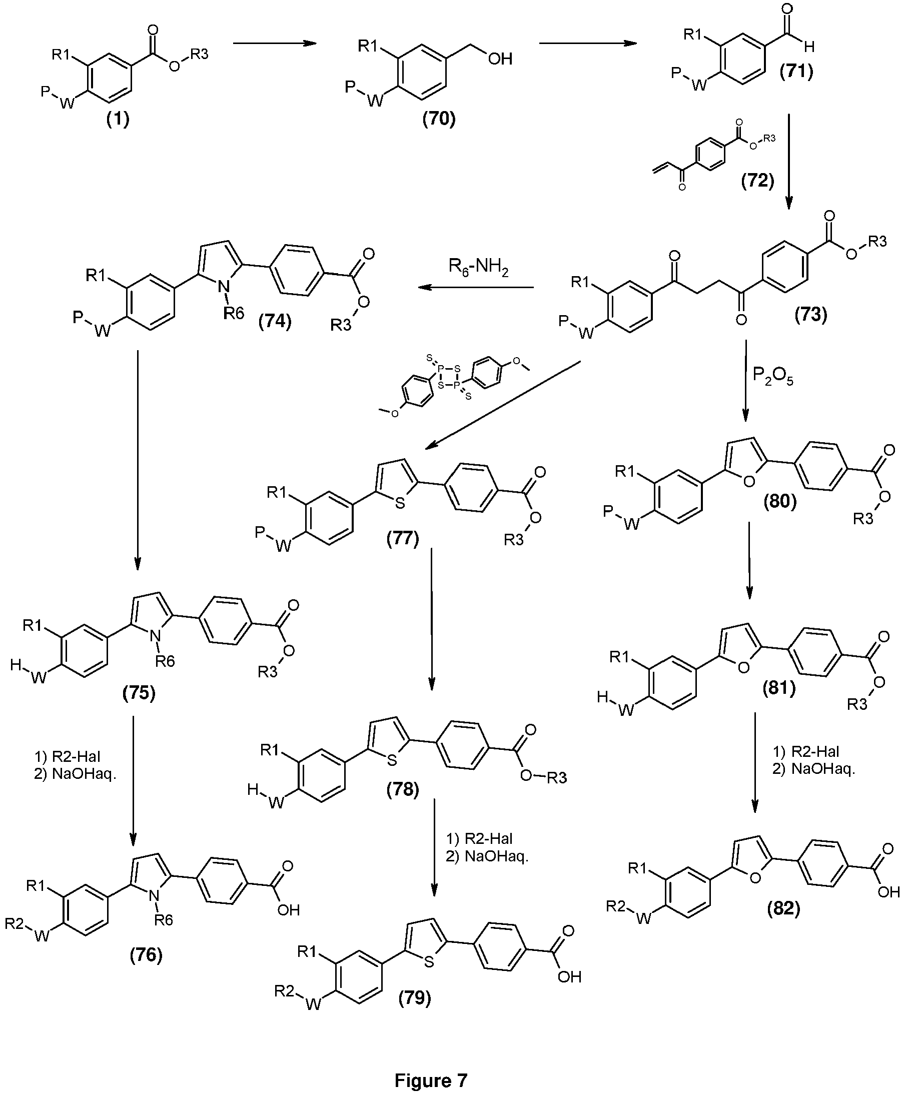

Selon les schémas décrits sur la figure 7, l'accès aux pyrroles, furanes et thiophènes est possible. Par réduction de la fonction ester des dérivés **(1)** (éventuellement préalablement protégés au niveau de la fonction phénol ou thiophénol) en présence d'hydrure d'aluminium lithium par exemple, le dérivé alcool benzylique **(70)** est obtenu. Une oxydation de ce dernier, en présence de dioxyde de manganèse par exemple, conduit au composé **(71).** Par réaction de Stetter entre cet aldéhyde **(71)** et le 4-acrylate de benzoate de méthyle par exemple préalablement préparé **(72)** selon les conditions classiques décrites dans la publication Angew. Chem. Int. Ed. 1976, 15 (11), p. 639-647, le dérivé **(73)** est obtenu. Par réaction de ce dernier en présence d'ammoniac ou d'une amine primaire, le pyrrole correspondant **(74)** est obtenu. Par réaction du composé **(73)** avec le 2,4-disulfure de 2,4-bis(4-méthoxyphényl)-1,3-dithia-2,4-diphosphétane (réactif de Lawesson), le dérivé thiophène correspondant **(77)** est obtenu. Par réaction du composé **(73)** avec du pentoxyde de phosphore, le dérivé furane **(80)** peut être préparé. Après déprotection de l'éventuelle fonction amino, phénol ou thiophénol (composés **(75), (78)** et **(81)**), puis éventuellement une alkylation en présence d'un halogénure d'alkyle par exemple, et d'une base telle que du carbonate de potassium ou amination réductrice sur la fonction aniline et enfin saponification en présence d'hydroxyde de sodium aqueux par exemple, les composés **(76) (79)** et **(82)** sont obtenus.

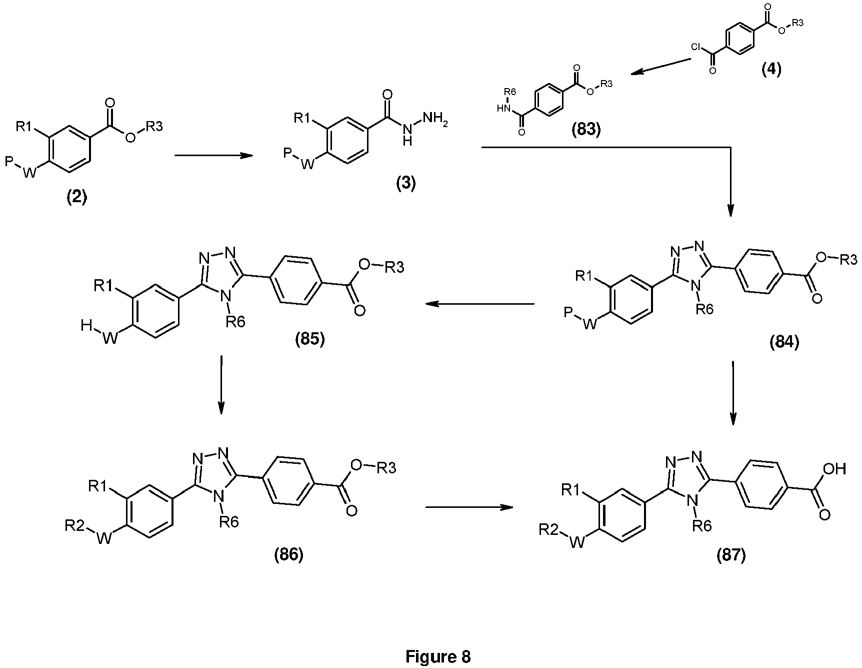

La figure 8 décrit une méthode permettant l'obtention des dérivés triazoles. Par réaction du dérivé ester **(2)** avec de l'hydrazine, le composé **(3)** est obtenu. Une réaction de condensation entre cet hydrazide **(3)** et le dérivé amide **(83)** (préalablement préparé par réaction du chlorure d'acide **(4)** disponible commercialement lorsque R3 est un méthyle, avec une amine de type R6NH₂, conduit au dérivé triazole **(84).** Dans le cas ou W=O, S ou N, une étape de déprotection permet de libérer les fonctions phénol ou thiophénol et conduit au composé **(85).** Une alkylation de ce composé en présence d'un halogénure d'alkyle par exemple, et d'une base telle que du carbonate de potassium, ou une amination réductrice sur la fonction aniline suivie d'une étape de saponification permet d'obtenir le composé **(87).**

Selon la présente description, les composés de formule générale (I) particulièrement préférés sont ceux pour lesquels :
A représente un groupement choisi parmi : 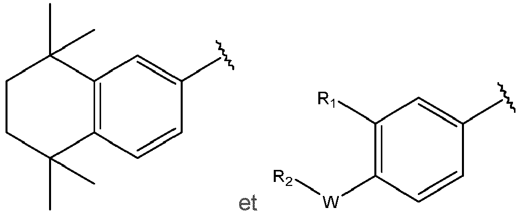
R1 est un radical alkyle linéaire ou ramifié, un radical cycloalkyle substitué, un radical adamantyle, ou un radical NR4R5 ;
R2 est un atome d'hydrogène, un radical alkyle linéaire ou ramifié, un radical polyéther, un radical mono ou polyhydroxyalkyle,
R3 est un atome d'hydrogène,
R4 et R5, identiques ou différents, représentent un radical alkyle linéaire ou ramifié, un radical alkyle substitué, un radical acyle. R4 et R5, pris ensemble, peuvent également être liés et former un hétérocycle avec l'atome d'azote de type azétidine, pyrrolidine, pipéridine, hétérocycle pouvant éventuellement être substitué.
X est O, S ou CH₂,
L'hétérocycle central est préférentiellement

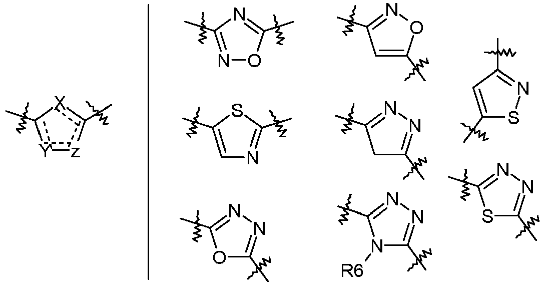

Selon la présente description, les composés de formule générale (I) particulièrement préférés sont ceux pour lesquels :
A représente un groupement choisi parmi : 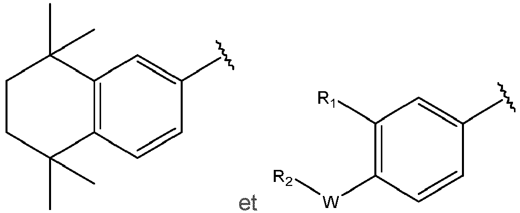
R1 est un radical alkyle ramifié, un radical cycloalkyle substitué ou un radical NR4R5
R2 est un atome d'hydrogène, un radical alkyle linéaire ou ramifié, un radical polyéther, un radical mono ou polyhydroxyalkyle,
R3 est un atome d'hydrogène,
R4 et R5, identiques ou différents, représentent un radical alkyle linéaire ou ramifié, un radical alkyle substitué, un radical acyle. R4 et R5, pris ensemble, peuvent également être liés et former un hétérocycle avec l'atome d'azote de type azétidine, pyrrolidine, pipéridine, hétérocycle pouvant éventuellement être substitué.
X est O, S
L'hétérocycle central est préférentiellement

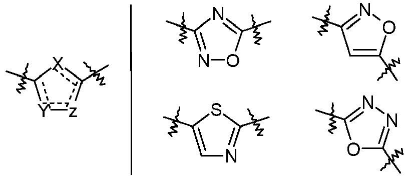

Selon la présente invention, les composés de formule générale (I) particulièrement préférés sont ceux pour lesquels :
A représente un groupement 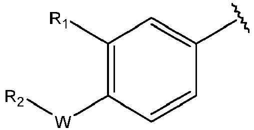
R1 est un radical alkyle ramifié, un radical NR4R5,
R2 est un atome d'hydrogène, un radical alkyle linéaire ou ramifié, un radical monohydroxyalkyle,
R3 est un atome d'hydrogène,
R4 et R5, identiques ou différents, représentent un radical alkyle linéaire ou ramifié, R4 et
R5, pris ensemble, peuvent également être liés et former un hétérocycle avec l'atome d'azote de type pyrrolidine, hétérocycle pouvant éventuellement être substitué.
X est O,
l'hétérocycle central est préférentiellement un oxadiazole

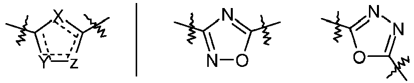

Il est également décrit un composé de formule (I) dans laquelle
A représente un groupement choisi parmi :

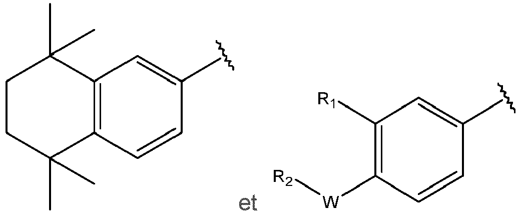

R1 est un alkyle ramifié ou un radical cycloalkyle substitué;
R2 est un atome d'hydrogène, un radical alkyle linéaire, ou un radical alkyle substitué par un radical alkoxyle ;
R3 est un hydrogène ; l'hétérocycle central 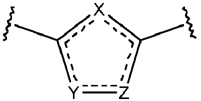 correspond à un cycle thiazolyle ou oxadiazolyle.

Selon un autre mode divulgué, le composé est un composé de formula (I) dans laquelle A représente un groupement choisi parmi : 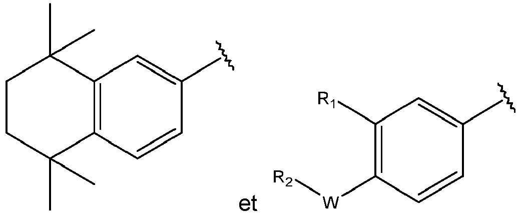
R1 est un radical tert-butyle ou un radical 1-méthyl-cyclohexyle ;
R2 est un radical ethoxymethyle ;
W est O ;
R3 est un hydrogène ;
X représente S, Y représente CH et Z représente N; ou X représente N, Y représente N et Z représente O.

La présente invention a également pour objet les composés de formule (I) tels que décrits ci-dessus à titre de médicament.

Les composés selon l'invention conviennent particulièrement bien dans les domaines de traitement suivants :
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose ;
5) les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanée telle que les kératoacanthomes ;
6) les désordres dermatologiques tels que les dermatoses immunes telles le lupus érythémateux, les maladies immunes bulleuses et les maladies du collagène, telle la sclérodermie ;
7) les affections dermatologiques ou générales à composante immunologique ;
8) les troubles ophtalmologiques, notamment les cornéopathies,
9) les stigmates de l'atrophie épidermique et/ou dermique induite par les corticostéroïdes locaux ou systémiques, ou toute autre forme d'atrophie cutanée,
10) le traitement de toute affection d'origine virale au niveau cutané ou général,
11) pour lutter contre les troubles de la fonction sébacée tels que l'hyperséborrhée de l'acné ou la séborrhée simple ;
12) les troubles de la cicatrisation, ou pour prévenir ou pour réparer les vergetures, ou encore pour favoriser la cicatrisation,
13) les désordres de la pigmentation, tel l'hyperpigmentation, le mélasma, l'hypopigmentation ou le vitiligo ;
16) les états cancéreux ou précancéreux ;
17) l'alopécie de différentes origines, notamment l'alopécie due à la chimiothérapie ou aux rayonnements.

La présente invention a également pour objet une composition pharmaceutique comprenant, dans un milieu physiologiquement acceptable, au moins un composé de formule (I) tel que défini ci-dessus.

La présente invention a aussi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou un de ses sels.

L'administration de la composition selon l'invention peut être effectuée par voie orale, entérale, parentérale, topique ou oculaire. De préférence, la composition pharmaceutique est conditionnée sous une forme convenant à une application par voie topique.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée. Par voie parentérale, la composition peut se présenter sous forme de solutions ou suspensions pour perfusion ou pour injection.

Les composés selon l'invention sont généralement administrés à une dose journalière d'environ 0,01 mg/kg à 100 mg/kg de poids corporel, en 1 à plusieurs prises.

Les composés sont utilisés par voie systémique à une concentration généralement comprise entre 0,001% et 10% en poids, de préférence entre 0,01% et 1% en poids, par rapport au poids de la composition.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

Les composés sont utilisés par voie topique à une concentration généralement comprise entre 0,001% et 10% en poids, de préférence entre 0,01% et 1% en poids, par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

### Partie expérimentale :

### A : préparation des composés oxadiazoles décrits dans la figure 1

### Exemple 1 : acide 4-{5-[3-adamantan-1-yl-4-(2-methoxy-ethoxymethoxy)-phényl]-[1,3,4]oxadiazol-2-yl}-benzoique

### 1.1 : 3-adamantan-1-yl-4-hydroxy-benzoate de méthyle

325ml (5 mmoles) d'acide méthanesulfonique sont additionnés goutte à goutte à une solution de 31g (200 mmoles) d'adamantanol et 31g (200 mmoles) de 4-hydroxybenzoate de méthyle dans 3l de dichlorométhane puis le mélange est chauffé à reflux pendant 24 heures. Après refroidissement à température ambiante, le milieu réactionnel est lavé à l'eau puis avec une solution saturée de bicarbonate de sodium. La phase organique est séchée sur sulfate de magnésium, filtrée, évaporée. Le résidu brut obtenu est purifié par chromatographie sur gel de silice élué avec un mélange heptane / acétate d'éthyle 97/3. 25g (44%) de 3-adamantan-1-yl-4-hydroxy-benzoate de méthyle sont obtenus.

### 1.2 : 3-adamantan-1-yl-4-(2-méthoxy-éthoxyméthoxy)-benzoate de méthyle

4,2g (105 mmoles) d'hydrure de sodium 60% dans l'hexane sont ajoutés par portions à une solution de 25g (88 mmoles) de 3-adamantan-1-yl-4-hydroxy-benzoate de méthyle dans 100ml de tétrahydrofurane et 100ml de diméthylformamide. Le milieu réactionnel est agité pendant 30 minutes puis 11ml (96 mmoles) de 1-chlorométhoxy-2-méthoxy-éthane sont additionnés goutte à goutte et le milieu réactionnel est à nouveau agité à température ambiante pendant 1 heure et demie. 150ml d'eau sont ensuite ajoutés et le milieu réactionnel est extrait à l'acétate d'éthyle. La phase organique est lavée à l'eau, séchée sur sulfate de magnésium, filtrée, évaporée. 39g de résidu brut sont obtenus et purifiés par chromatographie sur gel de silice élués avec un mélange heptane / acétate d'éthyle 8/2. 31,5g (96%) de 3-adamantan-1-yl-4-(2-méthoxy-éthoxyméthoxy)-benzoate de méthyle sont obtenus sous la forme d'un solide blanc.

### 1.3 : 3-adamantan-1-yl-4-(2-méthoxy-éthoxyméthoxy)-benzhydrazide

11ml (226 mmoles) d'hydrazine hydrate sont ajoutés à une solution de 10g (27 mmoles) de 3-adamantan-1-yl-4-(2-méthoxy-éthoxyméthoxy)-benzoate de méthyle dans 100ml de méthanol. Le milieu réactionnel est chauffé à reflux pendant 3 jours. Après refroidissement et addition d'eau, le milieu réactionnel est extrait à l'acétate d'éthyle. La phase organique est lavée à l'eau, séchée sur sulfate de magnésium, filtrée, évaporée. 10g de résidu brut sont obtenus et recristallisés dans l'éther diéthylique. 9,5g (95%) de 3-adamantan-1-yl-4-(2-méthoxy-éthoxyméthoxy)-benzhydrazide sont ainsi obtenus sous la forme d'un solide beige.

### 1.4 : 4-{N'-[3-adamantan-1-yl-4-(2-méthoxy-éthoxyméthoxy)-benzoyl]-hydrazinocarbonyl}-benzoate de méthyle

Une solution de 5,8g (29 mmoles) de 4-chlorocarbonyl-benzoate de méthyle dans 40ml de tétrahydrofurane est additionnée goutte à goutte à une solution de 11g (29 mmoles) de 3-adamantan-1-yl-4-(2-méthoxy-éthoxyméthoxy)-benzhydrazide dans 80ml de tétrahydrofurane. 1,1g (10% massique) de 4-diméthylamino-pyridine est ajouté et le milieu réactionnel est agité pendant 24 heures à température ambiante. De l'eau est ajoutée et le milieu réactionnel est extrait à l'acétate d'éthyle. La phase organique est lavée à l'eau, séchée sur sulfate de magnésium, filtrée, évaporée. 15,4g de résidu brut sont obtenus et purifiés par recristallisations successives dans du dichlorométhane puis de l'éther diéthylique. 9,6g (60%) de 4-{N'-[3-adamantan-1-yl-4-(2-méthoxy-éthoxyméthoxy)-benzoyl]-hydrazinocarbonyl}-benzoate de méthyle sont obtenus.

### 1.5 : 4-{5-[3-adamantan-1-yl-4-(2-méthoxy-éthoxyméthoxy)-phényl]-[1,3,4]oxadiazol-2-yl}-benzoate de méthyle

3,7ml (46 mmoles) de pyridine puis 1,7ml (23 mmoles) de chlorure de thionyle sont ajoutés à une solution de 9,6g (18 mmoles) de 4-{N'-[3-adamantan-1-yl-4-(2-méthoxy-éthoxyméthoxy)-benzoyl]-hydrazinocarbonyl}-benzoate de méthyle dans 100ml d'éther diéthylique, préalablement refroidie à 0°C. Le milieu réactionnel est agité de 0°C à température ambiante pendant 4 heures puis 100ml de toluène sont additionnés et le milieu est chauffé à reflux pendant 2 heures et demie. Après refroidissement, le milieu réactionnel est filtré, le précipité est rincé au tétrahydrofurane puis le filtrat concentré. 15g de résidu brut sont obtenus et purifié par chromatographie sur gel de silice élué avec un mélange heptane/ acétate d'éthyle 7/3. 8g (87%) de 4-{5-[3-adamantan-1-yl-4-(2-méthoxy-éthoxyméthoxy)-phényl]-[1,3,4]oxadiazol-2-yl}-benzoate de méthyle sont obtenus.

### 1.6 : acide 4-{5-[3-adamantan-1-yl-4-(2-méthoxy-éthoxyméthoxy)-phényl]-[1,3,4]oxadiazol-2-yl}-benzoique

0,8ml (0,8 mmole) d'une solution aqueuse d'hydroxyde de lithium de concentration 1M sont ajoutés à une solution de 0,2g (0,4 mmole) de 4-{5-[3-adamantan-1-yl-4-(2-méthoxy-éthoxyméthoxy)-phényl]-[1,3,4]oxadiazol-2-yl}-benzoate de méthyle dans 9ml de tétrahydrofurane et 3ml de méthanol. Le milieu réactionnel est chauffé à reflux pendant 6 heures puis refroidi, acidifié à pH5 avec une solution aqueuse d'acide chlorhydrique de concentration 1M et extrait à l'acétate d'éthyle. La phase organique est ensuite séchée sur sulfate de magnésium, filtrée et évaporée. 350mg de résidu brut sont obtenus et purifiés par chromatographie sur gel de silice élués avec un mélange heptane/ acétate d'éthyle 7/3. 150mg (77%) d'acide 4-{5-[3-adamantan-1-yl-4-(2-méthoxy-éthoxyméthoxy)-phényl]-[1,3,4]oxadiazol-2-yl}-benzoique sont obtenus.

### Exemple 2: acide 4-[5-(3-adamantan-1-yl-4-méthoxy-phényl)-[1,3,4]oxadiazol-2-yl]-benzoique

2.1 : 4-[5-(3-adamantan-1-yl-4-hydroxy-phényl)-[1,3,4]oxadiazol-2-yl]-benzoate de méthyle 3ml d'une solution commerciale d'acide sulfurique concentré sont additionnés à une solution de 7,8g (15 mmoles) de 4-{5-[3-adamantan-1-yl-4-(2-méthoxy-éthoxyméthoxy)-phényl]-[1,3,4]oxadiazol-2-yl}-benzoate de méthyle (préparé comme décrit dans l'exemple 1.5) dans 60ml de méthanol et 70ml de tétrahydrofurane. Le milieu réactionnel est agité à température ambiante pendant 18 heures puis filtré. Le filtrat est ramené à pH7 par addition d'une solution aqueuse d'hydroxyde de sodium, et extrait à l'acétate d'éthyle. La phase organique est lavée à l'eau, séchée sur sulfate de magnésium, filtrée, évaporée. 5,8g (90%) de 4-[5-(3-adamantan-1-yl-4-hydroxy-phényl)-[1,3,4]oxadiazol-2-yl]-benzoate de méthyle sont ainsi obtenus.

### 2.2 : 4-[5-(3-adamantan-1-yl-4-méthoxy-phényl)-[1,3,4]oxadiazol-2-yl]-benzoate de méthyle

Une solution de 250mg (0,6 mmole) de 4-[5-(3-Adamantan-1-yl-4-hydroxy-phényl)-[1,3,4]oxadiazol-2-yl]-benzoate de méthyle, 36mg (0,6 mmole) d'hydroxyde de potassium, 40ml (0,7 mmole) d'iodure de méthyle dans 20ml de diglyme est chauffée à reflux pendant 5 heures dans un tube fermé. Après refroidissement, de l'eau est additionnée et le milieu est extrait à l'acétate d'éthyle. La phase organique est séchée sur sulfate de magnésium, filtrée, évaporée. 260mg (100%) de 4-[5-(3-adamantan-1-yl-4-methoxy-phényl)-[1,3,4]oxadiazol-2-yl]-benzoate de méthyle sont obtenus.

### 2.3 : acide 4-[5-(3-adamantan-1-yl-4-méthoxy-phényl)-[1,3,4]oxadiazol-2-yl]-benzoique

0,7ml (0,7 mmole) d'une solution aqueuse d'hydroxyde de sodium de concentration 1M sont ajoutés à une solution de 250mg (0,6 mmole) de 4-[5-(3-adamantan-1-yl-4-methoxy-phényl)-[1,3,4]oxadiazol-2-yl]-benzoate de méthyle dans 10ml de tétrahydrofurane et 4ml de méthanol. Après agitation à température ambiante pendant 6 heures, le milieu réactionnel est acidifié à pH 3-4 par addition d'une solution aqueuse d'acide chlorhydrique de concentration 1M. Le produit précipite et est filtré, rincé à l'eau, séché sous vide. 212mg (85%) d'acide 4-[5-(3-adamantan-1-yl-4-méthoxy-phényl)-[1,3,4]oxadiazol-2-yl]-benzoique sont obtenus.

| n° exemple dans partie expérimentale | Structure chimique | nom du composé | voie de synthèse | RMN 1H | Aspect |
|---|---|---|---|---|---|
| exemple 1 | 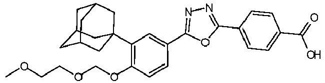 | acide 4-{5-[3-adamantan-1-yl-4-(2-méthoxy-éthoxyméthoxy)-phényl]-[1,3,4]oxadiazol-2-yl}-benzoique | A (figure 1) | ¹H NMR (400 MHz, CDCl₃): δ (ppm) 8.13 (m, 4 H); 7.95 (d, J = 2.2 Hz, 1 H); 7.85 (dd, J = 8.6, 2.1 Hz, 1 H); 7.21 (d, J = 8.6 Hz, 1 H); 5.34 (s, 2 H); 3.81 (dd, J = 5.6, 3.5 Hz, 2 H); 3.53 (m, J = 4.4 Hz, 2 H); 3.32 (s, 3 H); 2.09 (s, 6 H); 2.04 (s, 3 H); 1.73 (s, 6 H). | solide blanc |
| exemple 2 | 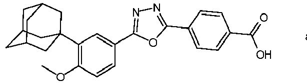 | acide 4-[5-(3-adama ntan-1-yl-4-méthoxy-phényl)-[1,3,4]oxadiazol-2-yl]-benzoique | A (figure 1) | ¹H NMR (400 MHz, CDCl₃): δ (ppm) 8.10 (m, 4 H); 7.90 (d, J = 2.2 Hz, 1 H); 7.86 (dd, J = 8.5, 2.1 Hz, 1 H); 6.91 (d, J = 8.5 Hz, 1 H); 3.83 (s, 3 H); 2.05 (s, 6 H); 2.01 (s, 3 H); 1.70 (s, 6 H). | solide blanc |
| | 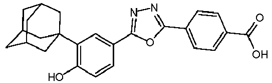 | acide 4-[5-(3-adamantan-1-yl-4-hydroxy-phényl)-[1,3,4]oxadiazol-2-yl]-benzoique | A (figure 1) | ¹H NMR (400 MHz, DMSO): δ (ppm) 10.37 (s, 1 H); 8.16 (s, 4 H); 7.79-7.83 (m, 2 H); 7.00 (d, J = 8.3 Hz, 1 H); 2.14 (s, 6 H); 2.08 (s, 3 H); 1.76 (s, 6 H). | solide beige |
| | 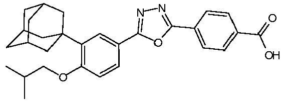 | acide 4-[5-(3-adamantan-1-yl-4-isobutoxy-phényl)-[1,3,4]oxadiazol-2-yl]-benzoique | A (figure 1) | ¹H NMR (400 MHz, CDCl₃): δ (ppm) 8.09-8.11 (m, 4 H); 7.90 (d, J = 2.2 Hz, 1 H); 7.83 (dd, J = 8.6 Hz et 2.2 Hz, 1 H); 6.89 (d, J = 8.6 Hz, 1 H); 3.76 (d, J = 6.3 Hz, 2 H); 2.51 (m, 1 H); 2.09 (s, 6 H); 2.02 (s, 3 H); 1.71 (s, 6 H); 1.05 (d, J = 6.7 Hz, 6 H). | solide beige |
| | 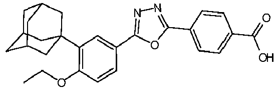 | acide 4-[5-(3-adamantan-1-yl-4-éthoxy-phényl)-[1,3,4]oxadiazol-2-yl]-benzoique | A (figure 1) | ¹H NMR (400 MHz, CDCl₃): δ (ppm) 8.02 (m, 4 H); 7.81 (d, J = 2.3 Hz, 1 H); 7.76 (dd, J = 8.5, 2.2 Hz, 1 H); 6.81 (d, J = 8.5 Hz, 1 H); 3.99 (q, J = 6.9 Hz, 2 H); 2.01 (s, 6 H); 1.94 (s, 3 H); 1.64 (s, 6 H); 1.37 (t, J = 6.9 Hz, 3 H). | solide blanc |
| | 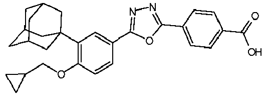 | acide 4-[5-(3-adamantan-1-yl-4-cyclopropylméthoxy-phényl)-[1,3,4]oxadiazol-2-yl]-benzoique | A (figure 1) | ¹H NMR (400 MHz, CDCl₃): δ (ppm) 8.05 (m, 4 H); 7.84 (s, 1 H); 7.77 (d, J = 8.6 Hz, 1 H); 6.79 (d, J = 8.5 Hz, 1 H); 3.79 (d, J = 6.9 Hz, 2 H); 2.07 (s, 6 H); 1.98 (s, 3 H); 1.66 (s, 6 H); 0.73 (s, 1 H); 0.56 (d, J = 7.8 Hz, 2 H); 0.28 (d, J = 5.1 Hz, 2 H). | solide blanc |
| | 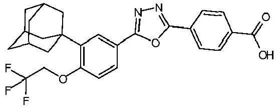 | acide [1,3,4]oxadiazol-2-yl}-benzoique | A (figure 1) | ¹H NMR (400 MHz, CDCl₃): δ (ppm) 8.00 (m, 4 H); 7.85 (d, J = 2.2 Hz, 1 H); 7.78 (dd, J = 8.5, 2.2 Hz, 1 H); 6.76 (d, J = 8.5 Hz, 1 H); 4.32 (q, J = 8.0 Hz, 2 H); 1.94 (m, 9 H); 1.60 (s, 6 H). | solide blanc |
| | 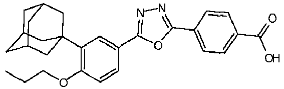 | acide 4-[5-(3-adama ntan-1-yl-4-propoxy-phényl)-[1,3,4]oxadiazol-2-yl]-benzoique | A (figure 1) | ¹H NMR (400 MHz, CDCl₃): δ (ppm) 7.98 (m, 4 H); 7.76 (d, J = 2.3 Hz, 1 H); 7.71 (dd, J = 8.5, 2.3 Hz, 1 H); 6.78 (d, J = 8.5 Hz, 1 H); 3.84 (t, J = 6.3 Hz, 2 H); 1.97 (s, 6 H); 1.90 (s, 3 H); 1.58 (s, 6 H); 0.94 (t, J = 7.4 Hz, 3 H). | solide blanc |
| | 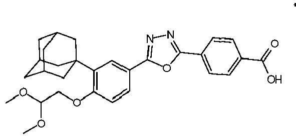 | acide 4-{5-[3-adamantan-1-yl-4-(2,2-diméthoxy-éthoxy)-phényl]-[1,3,4]oxadiazol-2-yl}-benzoique | A (figure 1) | ¹H NMR (400 MHz, CDCl₃): δ(ppm) 8.14 (m, 4 H); 7.94 (d, J = 2.2 Hz, 1 H); 7.87 (dd, J = 8.5, 2.1 Hz, 1 H); 6.89 (d, J = 8.5 Hz, 1 H); 4.80 (t, J = 5.3 Hz, 1 H); 4.03 (d, J = 5.3 Hz, 2 H); 3.43 (s, 6 H); 2.12 (s, 6 H); 2.06 (s, 3 H); 1.74 (s, 6 H). | solide blanc |
| | 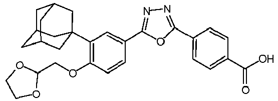 | acide 4-{5-[3-adamantan-1-yl-4-([1,3]dioxolan-2-ylméthoxy)-phényl]-[1,3,4]oxadiazol-2-yl}-benzoique | A (figure 1) | ¹H NMR (400 MHz, CDCl₃): δ (ppm) 8.10 (m, 4 H); 7.91 (d, J = 2.3 Hz, 1 H); 7.64 (dd, J = B.5, 2.3 Hz, 1 H); 6.89 (d, J = 8.5 Hz, 1 H); 5.29 (t, J = 3.8 Hz, 1 H); 4.04 (d, J = 3.9 Hz, 2 H); 3.98 (m, 2 H); 3.89-3.91 (m, 2 H); 2.07 (s, 6 H); 2.01 (s, 3 H); 1.69 (s, 6 H). | solide blanc |
| | 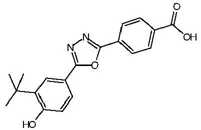 | acide 4-[5-(3-tert-butyl-4-hydroxy-phenyl)-[1,3,4]oxadiazol-2-yl]-benzoique | A (figure 1) | | |
| | 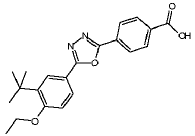 | acide 4-[5-(3-tert-butyl-4-éthoxy-phenyl)-[1,3,4]oxadiazol-2-yl]-benzoique | A (figure 1) | | |
| | 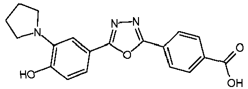 | 4-[5-(4-hydroxy-3-pyrrolidin-1-yl-phényl)-[1,3,4]oxadiazol-2-yl]-benzoique | A (figure 1) | | |
| | 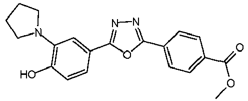 | 4-[5-(4-hydroxy-3-pyrrolidin-1-yl-phenyl)-[1,3,4]oxadiazol-2-yl]-benzoate de méthyle | A (figure 1) | | |

### B : préparation des composés oxadiazoles décrits dans la figure 2

### Exemple 3: acide 4-[3-(3-tert-butyl-4-hydroxy-phényl)-[1,2,4]oxadiazol-5-yl]-benzoique

### 3.1: 4-bromo-2-tert-butyl-phénol

47g (98 mmoles) de bromure de tétrabutylammonium sont additionnés par portions à une solution de 15ml (98 mmoles) de 2-tert-butyl-phénol dans 300ml de chloroforme. Le milieu réactionnel est agité à température ambiante pendant 1 heure puis hydrolysé par addition de 300ml d'une solution aqueuse saturée de thiosulfate de sodium et extrait au dichlorométhane. Les phases organiques sont rassemblées, lavées à l'eau, séchées sur sulfate de magnésium, filtrées puis évaporées. Le résidu brut obtenu est repris dans de l'éther diéthylique, les sels inorganiques restants précipitent. Le milieu est filtré, la phase éthérée est lavée avec une solution saturée de chlorure de sodium, séchée sur sulfate de magnésium, filtrée et évaporée. 22g (100%) de 4-bromo-2-tert-butyl-phenol sont obtenus sous la forme d'une huile jaune clair.

### 3.2 : 4-bromo-2-tert-butyl-1-éthoxyméthoxy-benzène

4,7g (117 mmoles) d'hydrure de sodium sont ajoutés par portions, à une solution de 22,4g (98 mmoles) de 4-bromo-2-tert-butyl-phénol dans un mélange de 100ml de tétrahydrofurane et 100ml de diméthylformamide. Après agitation à température ambiante pendant 20 minutes, 10ml (107 mmoles) de chlorométhoxy-éthane sont additionnés goutte à goutte puis le milieu réactionnel est agité pendant 2 heures à température ambiante. Après hydrolyse par addition d'eau, le milieu réactionnel est extrait à l'acétate d'éthyle. La phase organique est lavée par une solution saturée de chlorure de sodium, séchée sur sulfate de magnésium, filtrée et évaporée. 31,5g de résidu brut sont obtenus et purifiés par chromatographie sur gel de silice élués par un mélange heptane / acétate d'éthyle en augmentant régulièrement la polarité jusqu'à obtenir une proportion 9/1. 20g (71%) de 4-bromo-2-tert-butyl-1-éthoxyméthoxy-benzène sont obtenus sous la forme d'une huile jaune.

### 3.3 : 3-tert-butyl-4-éthoxyméthoxy-benzonitrile

6,8g (77 mmoles) de cyanure de cuivre sont additionnés par portions à une solution de 20g (70 mmoles) de 4-bromo-2-tert-butyl-1-éthoxyméthoxy-benzène dans 200ml de diméthyformamide et 1ml de pyridine. Le milieu réactionnel est agité à reflux pendant 5 heures puis filtré. Après addition d'eau et dilution avec de l'acétate d'éthyle, le milieu réactionnel est extrait à l'acétate d'éthyle. De nombreux lavages à l'eau de la phase organique sont réalisés puis la phase organique est séchée sur sulfate de magnésium, filtrée et évaporée. 17g de résidu brut sont obtenus et purifiés par chromatographie sur gel de silice élués par un mélange heptane / acétate d'éthyle en augmentant régulièrement la polarité jusqu'à obtenir une proportion 9/1. 9,6g (59%) de 3-tert-butyl-4-éthoxyméthoxy-benzonitrile sont obtenus sous la forme d'une huile marron.

### 3.4 : 3-tert-butyl-4-éthoxyméthoxy-N-hydroxy-benzamidine

4,3g (62 mmoles) de chlorhydrate d'hydroxylamine sont additionnés par portions à une solution de 9,6g (41 mmoles) de 3-tert-butyl-4-éthoxyméthoxy-benzonitrile dans 100ml d'éthanol. Une solution de 2,5g (62 mmoles) d'hydroxyde de sodium dans 20ml d'eau est ensuite additionnée goutte à goutte au mélange précédent. Le milieu réactionnel est alors chauffé à reflux pendant 24 heures puis hydrolysé par addition d'eau et extrait à l'acétate d'éthyle. La phase organique est lavée par une solution saturée de chlorure de sodium, séchée sur sulfate de magnésium, filtrée et évaporée. 11g de résidu brut sont obtenus et purifiés par chromatographie sur gel de silice élués avec un mélange heptane / acétate d'éthyle en augmentant régulièrement la polarité jusqu'à obtenir une proportion 5/5 dans chacun des solvants. 5,2g (48%) de 3-tert-butyl-4-éthoxyméthoxy-N-hydroxy-benzamidine sont obtenus sous la forme d'un solide blanc.

### 3.5 : 4-[3-(3-tert-butyl-4-éthoxyméthoxy-phényl)-[1,2,4]oxadiazol-5-yl]-benzoate de méthyle

Une suspension de 5g (25 mmoles) de 4-chlorocarbonyl-benzoate de méthyle dans 25ml de pyridine est additionnée à une solution de 5,2g (20 mmoles) de 3-tert-butyl-4-éthoxyméthoxy-N-hydroxy-benzamidine dans 50ml de pyridine. Le milieu réactionnel est ensuite chauffé à reflux pendant 30 minutes. Après refroidissement, de l'eau est additionnée et le milieu est extrait au dichlorométhane. La phase organique est lavée à l'eau, séchée sur sulfate de magnésium, filtrée et évaporée. 9g de résidu brut sont obtenus et purifiés par chromatographie sur gel de silice élué avec de l'heptane. 7g (87%) de 4-[3-(3-tert-butyl-4-éthoxyméthoxy-phényl)-[1,2,4]oxadiazol-5-yl]-benzoate de méthyle sont obtenus sous la forme d'un solide blanc.

### 3.6: acide 4-[3-(3-tert-butyl-4-éthoxyméthoxy-phényl)-[1,2,4]oxadiazol-5-yl]-benzoique

19ml (19 mmoles) d'une solution aqueuse d'hydroxyde de lithium de concentration 1M sont additionnés à une solution de 6,5g (16 mmoles) de 4-[3-(3-tert-butyl-4-éthoxyméthoxy-phényl)-[1,2,4]oxadiazol-5-yl]-benzoate de méthyle dans 70ml de tétrahydrofurane. Le milieu réactionnel est agité à température ambiante pendant 5 heures puis acidifié par addition d'une solution aqueuse d'acide chlorhydrique de concentration 1M jusqu'à pH 3-4. Le produit précipite. Après filtration et rinçage du solide obtenu à l'eau puis à l'heptane, 6g de résidu brut sont obtenus et purifiés par chromatographie sur gel de silice élué par un mélange heptane / acétate d'éthyle 8/2. 4,8g (76%) d'acide 4-[3-(3-tert-butyl-4-éthoxyméthoxy-phényl)-[1,2,4]oxadiazol-5-yl]-benzoique sont obtenus sous la forme d'un solide blanc.

### 3.7 : 4-[3-(3-tert-butyl-4-hydroxy-phényl)-[1,2,4]oxadiazol-5-yl]-benzoate de méthyle

1ml d'acide sulfurique concentré est additionné à une solution de 0,9g (2,3 mmoles) de 4-[3-(3-tert-butyl-4-éthoxyméthoxy-phényl)-[1,2,4]oxadiazol-5-yl]-benzoate de méthyle dans un mélange de 15ml de méthanol et 15ml de tétrahydrofurane. Le milieu réactionnel est agité à température ambiante pendant 20 heures puis chauffé à 50°C pendant 7 heures. Après addition d'eau, le produit précipite. Le précipité est filtré, rincé à l'eau puis à l'heptane. 0,75g (94%) de 4-[3-(3-tert-butyl-4-hydroxy-phényl)-[1,2,4]oxadiazol-5-yl]-benzoate de méthyle sont obtenus.

### 3.8 : acide 4-[3-(3-tert-butyl-4-hydroxy-phényl)-[1,2,4]oxadiazol-5-yl]-benzoique

1,8ml (1,8 mmole) d'une solution aqueuse d'hydroxyde de lithium de concentration 1M sont ajoutés à une solution de 0,3g (0,9 mmole) de 4-[3-(3-tert-butyl-4-hydroxy-phényl)-[1,2,4]oxadiazol-5-yl]-benzoate de méthyle dans 8ml de tétrahydrofurane. Le milieu réactionnel est agité à température ambiante pendant 24 heures puis acidifié par addition d'une solution aqueuse d'acide chlorhydrique de concentration 1M jusqu'à pH 3-4. Le produit précipite. Après filtration et rinçage du solide obtenu à l'eau puis à l'heptane, 0,25g (86%) d'acide 4-[3-(3-tert-butyl-4-hydroxy-phényl)-[1,2,4]oxadiazol-5-yl]-benzoique sont obtenus.

### Exemple 4 : acide 4-[3-(3-tert-butyl-4-éthoxy-phényl)-[1,2,4]oxadiazol-5-yl]-benzoique

### 4.1 : 4-[3-(3-tert-butyl-4-éthoxy-phényl)-[1,2,4]oxadiazol-5-yl]-benzoate de méthyle

60µl (0,7 mmole) d'iodure d'éthyle sont additionnés à un mélange de 200mg (0,6 mmole) de 4-[3-(3-tert-butyl-4-hydroxy-phényl)-[1,2,4]oxadiazol-5-yl]-benzoate de méthyle (préparé comme décrit dans l'exemple 1.7) et 300mg (0,9 mmole) de carbonate de césium dans 10ml de diméthylformamide. Le milieu réactionnel est chauffé à 80°C pendant 18 heures. Après refroidissement, le milieu réactionnel est filtré puis évaporée. Le résidu brut obtenu est purifié par chromatographie sur gel de silice, élué avec un mélange heptane / acétate d'éthyle 1/1. 188mg (87%) de 4-[3-(3-tert-butyl-4-éthoxy-phényl)-[1,2,4]oxadiazol-5-yl]-benzoate de méthyle sont obtenus.

### 4.2 : acide 4-[3-(3-tert-butyl-4-éthoxy-phényl)-[1,2,4]oxadiazol-5-yl]-benzoique

750µl (0,75 mmole) d'une solution aqueuse d'hydroxyde de lithium de concentration 1M sont ajoutés à une solution de 188mg (0,50 mmole) de 4-[3-(3-tert-butyl-4-éthoxy-phényl)-[1,2,4]oxadiazol-5-yl]-benzoate de méthyle dans 6ml de tétrahydrofurane et 0,5ml d'eau. Le milieu réactionnel est agité à température ambiante pendant 20 heures puis le tétrahydrofurane est évaporé. Après dilution avec de l'eau et acidification par une solution aqueuse d'acide chlorhydrique de concentration 1M, le produit précipite. Après filtration, rinçage du précipité à l'eau et à l'heptane, 125mg (68%) d'acide 4-[3-(3-tert-butyl-4-éthoxy-phényl)-[1,2,4]oxadiazol-5-yl]-benzoique sont obtenus.

RMN 1H (δ, DMSO) : 1.41 (s, 9H), 7 (d, J = 8.4 Hz, 1H), 7.8 (dd, J = 8.4-2.1 Hz, 1H), 7.9 (d, J = 2.1 Hz, 1H), 8.18 (dd, J = 6.8-1.8 Hz, 2H), 8.3 (dd, J = 6.7-1.7 Hz, 2H).

### Exemple 5 : acide 4-{3-[3-(1-méthyl-cyclohexyl)-4-propoxy-phényl]-[1,2,4]oxadiazol-5-yl}-benzoique

### 5.1 : 4-bromo-2-(1-méthyl-cyclohexyl)-phénol

2ml (31 mmoles) d'acide méthanesulfonique sont additionnés à une solution de 4,7g (41 mmoles) de 1-méthylcyclohexanol et 7g (40 mmoles) de 4-bromophénol dans 25ml de dichlorométhane puis le mélange est chauffé à reflux pendant 24 heures. Après refroidissement à température ambiante, le milieu réactionnel est lavé à l'eau puis avec une solution saturée de bicarbonate de sodium. La phase organique est séchée sur sulfate de magnésium, filtrée, évaporée. Le résidu brut obtenu est purifié par chromatographie sur gel de silice élué avec un mélange heptane / acétate d'éthyle 97/3. 4,7g (44%) de 4-bromo-2-(1-méthyl-cyclohexyl)-phénol sont obtenus.

### 5.2 : 4-bromo-1-éthoxyméthoxy-2-(1-méthyl-cyclohexyl)-benzène

0,8g (20 mmoles) d'hydrure de sodium à 60% dans l'heptane sont additionnés par portions à une solution de 4,5g (17 mmoles) de 4-bromo-2-(1-méthyl-cyclohexyl)-phénol dans 20ml de tétrahydrofurane et 20ml de diméthylformamide. Le milieu réactionnel est agité pendant 20 minutes à température ambiante puis 1,7ml (19 mmoles) de chlorométhoxy-éthane sont additionnés. Après 2 heures d'agitation à température ambiante, le milieu est hydrolysé par addition d'eau puis extrait à l'acétate d'éthyle. La phase organique est lavée par une solution saturée de chlorure de sodium, séchée sur sulfate de magnésium, filtrée et évaporée. 5,1g de résidu brut obtenus sont purifiés par chromatographie sur gel de silice élués avec un mélange heptane / acétate d'éthyle en augmentant régulièrement la polarité jusqu'à obtenir une proportion 9/1. 4,9g (89%) de 4-bromo-1-éthoxyméthoxy-2-(1-méthyl-cyclohexyl)-benzène sont obtenus sous la forme d'une huile marron.

### 5.3 : 4-éthoxyméthoxy-3-(1-méthyl-cyclohexyl)-benzonitrile

1,4g (16 mmoles) de cyanure de cuivre sont ajoutés à une solution de 4,8g (15 mmoles) de 4-éthoxyméthoxy-3-(1-méthyl-cyclohexyl)-benzonitrile dans 50ml de diméthylformamide et 1ml de pyridine. Le milieu réactionnel est agité à reflux pendant 10 heures puis refroidi et dilué à l'acétate d'éthyle. Après filtration, la phase organique est lavée à l'eau, séchée sur sulfate de magnésium, filtrée, évaporée. 10g de résidu brut sont obtenus et purifiés par chromatographie sur gel de silice élué avec un mélange heptane / acétate d'éthyle en augmentant régulièrement la polarité jusqu'à obtenir une proportion 9/1. 1,8g (46%) de 4-éthoxyméthoxy-3-(1-méthyl-cyclohexyl)-benzonitrile sont obtenus sous forme d'une huile jaune.

### 5.4 : 4-éthoxyméthoxy-N-hydroxy-3-(1-méthyl-cyclohexyl)-benzamidine

0,7g (10 mmoles) de chlorhydrate d'hydroxylamine suivi de 0,4g (10 mmoles) d'hydroxyde de sodium préalablement dilués dans 5ml d'eau sont ajoutés à une solution de 1,8g (7 mmoles) de 4-éthoxyméthoxy-3-(1-méthyl-cyclohexyl)-benzonitrile dans 25ml d'éthanol. Le milieu réactionnel est chauffé à reflux pendant 24 heures. Après refroidissement et addition d'eau, le milieu est extrait à l'acétate d'éthyle. La phase organique est lavée avec une solution saturée de chlorure de sodium, séchée sur sulfate de magnésium, filtrée, évaporée. 2,2g de résidu brut sont obtenus et purifiés par chromatographie sur gel de silice élué avec un mélange heptane / acétate d'éthyle 7/3. 1,5g (76%) de 4-éthoxyméthoxy-N-hydroxy-3-(1-méthyl-cyclohexyl)-benzamidine sont obtenus.

### 5.5 : 4-{3-[4-éthoxyméthoxy-3-(1-méthyl-cyclohexyl)-phényl]-[1,2,4]oxadiazol-5-yl}-benzoate de méthyle

Une suspension de 1g (5 mmoles) de 4-chlorocarbonyl-benzoate de méthyle dans 15ml de pyridine est additionnée à une solution de 1,5g (5 mmoles) de 4-éthoxyméthoxy-N-hydroxy-3-(1-méthyl-cyclohexyl)-benzamidine dans 15ml de pyridine. Le milieu réactionnel est chauffé à reflux pendant 30 minutes puis refroidi, dilué au dichlorométhane et lavé à l'eau. La phase organique est séchée sur sulfate de magnésium, filtrée, évaporée. 1,7g (75%) de 4-{3-[4-éthoxyméthoxy-3-(1-méthyl-cyclohexyl)-phényl]-[1,2,4]oxadiazol-5-yl}-benzoate de méthyle sont obtenus.

### 5.6 : acide 4-{3-[4-éthoxyméthoxy-3-(1-méthyl-cyclohexyl)-phényl]-[1,2,4]oxadiazol-5-yl}-benzoique

De manière analogue à l'exemple 3.6, à partir de 1,7g (4 mmoles) de 4-{3-[4-éthoxyméthoxy-3-(1-méthyl-cyclohexyl)-phényl]-[1,2,4]oxadiazol-5-yl}-benzoate de méthyle, 1,3g (76%) d'acide 4-{3-[4-éthoxyméthoxy-3-(1-méthyl-cyclohexyl)-phényl]-[1,2,4]oxadiazol-5-yl}-benzoique sont obtenus sous la forme d'un solide blanc.

### 5.7: 4-{3-[4-hydroxy-3-(1-méthyl-cyclohexyl)-phényl]-[1,2,4]oxadiazol-5-yl}-benzoate de méthyle

De manière analogue à l'exemple 3.7, à partir de 1,1g (3 mmoles) de 4-{3-[4-éthoxyméthoxy-3-(1-méthyl-cyclohexyl)-phényl]-[1,2,4]oxadiazol-5-yl}-benzoate de méthyle, 0,9g (93%) de 4-{3-[4-hydroxy-3-(1-méthyl-cyclohexyl)-phényl]-[1,2,4]oxadiazol-5-yl}-benzoate de méthyle sont obtenus.

### 5.8: 4-{3-[3-(1-Methyl-cyclohexyl)-4-propoxy-phényl]-[1,2,4]oxadiazol-5-yl}-benzoate de méthyle

De manière analogue à l'exemple 4.1, à partir de 150mg (0,4 mmole) de 4-{3-[4-Hydroxy-3-(1-methyl-cyclohexyl)-phényl]-[1,2,4]oxadiazol-5-yl}-benzoate de méthyle et de 55µl (1,6 mmoles) d'iodure den-propyle, 135mg (82%) de 4-{3-[3-(1-Methyl-cyclohexyl)-4-propoxy-phényl]-[1,2,4]oxadiazol-5-yl}-benzoate de méthyle sont obtenus.

### 5.9 : acide 4-{3-[3-(1-Methyl-cyclohexyl)-4-propoxy-phényl]-[1,2,4]oxadiazol-5-yl}-benzoique

De manière analogue à l'exemple 4.2, à partir de 135mg (0,3 mmole) de 4-{3-[3-(1-Methyl-cyclohexyl)-4-propoxy-phényl]-[1,2,4]oxadiazol-5-yl}-benzoate de méthyle, 85mg (65%) d' acide 4-{3-[3-(1-Methyl-cyclohexyl)-4-propoxy-phényl]-[1,2,4]oxadiazol-5-yl}-benzoique sont obtenus sous la forme d'un solide blanc.

| n° exemple dans partie expérimentale | Structure chimique | nom du composé | voie de synthèse | RMN 1H | Aspect |
|---|---|---|---|---|---|
| exemple 3 | 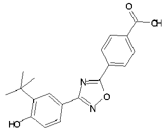 | acide 4-[3-(3-tert-butyl-4-hydroxy-phenyl)-[1,2,4]oxadiazol-5-yl]-benzoique | B (figure 2) | ¹H NMR (400 MHz, DMSO): δ (ppm) 13.45 (br s, 1 H); 10.20 (s, 1 H); 8.29 (d, J = 8.3 Hz, 2 H); 8.18 (d, J = 8.3 Hz, 2 H); 7.93 (d, J = 2.1 Hz, 1 H); 7.80 (dd, J = 8.3, 2.1 Hz, 1 H); 6.98 (d, J = 8.3 Hz, 1 H); 1.42 (s, 9 H). | solide blanc |
| exemple 4 | 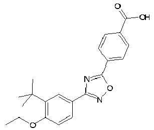 | acide 4-[3-(3-tert-butyl-4-ethoxy-phenyl)-[1,2,4]oxadiazol-5-yl]-benzoique | B (figure 2) | ¹H NMR (400 MHz, DMSO): δ (ppm) 8.20 (d, J = 8.1 Hz, 2 H); 8.13 (d, J = 8.1 Hz, 2 H); 7.95 (s, 1 H); 7.88 (d, J = 8.5 Hz, 1 H); 6.94 (d, J = 8.5 Hz, 1 H); 4.09 (q, J = 7.0 Hz, 2 H); 1.44 (t, 7.0 Hz, 3H); 1.38 (s, 9 H). | solide blanc |
| exemple 5 | 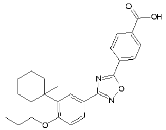 | acide 4-{3-[3-(1-méthyl-cyclohexyl)-4-propoxy-phenyl]-[1,2,4]oxadiazol-5-yl}-benzoique | B (figure 2) | ¹H NMR (400 MHz, DMSO): δ (ppm) 13.4 (brs, 1H); 8.28 (d, J = 8.1 Hz, 2 H); 8.18 (d, J = 8.1 Hz, 2 H); 8.00 (s, 1 H); 7.91 (d, J = 8.5 Hz, 1 H); 7.15 (d, J = 8.6 Hz, 1 H); 4.04 (m, 2 H); 2.15 (m, 2 H); 1.82 (m, 2 H); 1.73 (m, 2 H); 1.47-1.57 (br m, 4 H); 1.33 (s, 3 H); 1.05 (t, J = 7.4 Hz, 3 H). | solide blanc |
| | 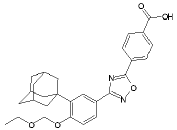 | acide 4-[3-(3-adamantan-1-yl-4-ethoxymethoxy-phenyl)-[1,2,4]oxadiazol-5-yl]-benzoique | B (figure 2) | ¹H NMR (400 MHz, CDCl₃): δ (ppm) 8.24 (d, J = 8.1 Hz, 2 H); 8.17 (d, J = 8.2 Hz, 2 H); 8.00 (d, J = 2.1 Hz, 1 H); 7.91 (dd, J = 8.5, 2.0 Hz, 1 H); 7.18 (d, J = 8.6 Hz, 1 H); 5.30 (s, 2 H); 3,74 (q, J = 7.1 Hz, 2 H); 2.13 (s, 6 H); 2.06 (s, 3 H); 1.75 (s, 6 H); 1.22 (t, J = 7.1 Hz, 3 H). | solide blanc |
| | 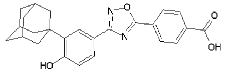 | acide 4-[3-(3-adamantan-1-yl-4-hydroxy-phenyl)-[1,2,4]oxadiazol-5-yl]-benzoique | B (figure 2) | ¹H NMR (400 MHz, CDCl₃): δ (ppm) 8.85 (s, 1 H); 8.20 (d, J = 8.2 Hz, 2 H); 8.14 (d, J = 8.2 Hz, 2 H); 7.91 (d, J = 2.1 Hz, 1 H); 7.74 (dd, J = 8.3, 2.1 Hz, 1 H); 6.84 (d, J = 8.3 Hz, 1 H); 2.13 (s, 6 H); 2.02 (s, 3 H); 1.72 (s, 6 H). | solide blanc |
| | 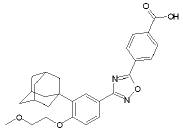 | acide 4-{3-[3-adamantan-1-yl-4-(2-methoxy-ethoxy)-phenyl]-[1,2,4]oxadiazol-5-yl}-benzoique | B (figure 2) | ¹H NMR (400 MHz, DMSO): δ (ppm) 8.22 (s, 4 H); 7.91 (s, 2 H); 7.16 (d, J = 7.8 Hz, 1 H); 4.19 (s, 2 H); 3.75 (s, 2 H); 3.34 (s, 3 H); 2.11 (s, 6 H); 2.04 (s, 3 H); 1.73 (s, 6 H). | solide marron |
| | 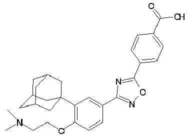 | acide 4-{3-[3-adamantan-1-yl-4-(2-dimethylamino-ethoxy)-phenyl]-[1,2,4]oxadiazol-5-yl}-benzoique | B (figure 2) | ¹H NMR (400 MHz, DMSO): δ (ppm) 8.10-8.12 (m, 4 H); 7,6 (M, 1H); 7.49 (s, 1 H); 6,73 (M, 1H); 4.36 (t, J = 5.8 Hz, 2 H); 3.11 (M, 2 H); 2.13 (M, 6 H); 1.99 (M, 3 H); 1.70 (t, J = 16.6 Hz, 6 H). | solide blanc |
| | 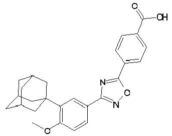 | acide 4-[3-(3-adamantan-1-yl-4-methoxy-phenyl)-[1,2,4]oxadiazol-5-yl]-benzoique | B (figure 2) | ¹H NMR (400 MHz, DMSO): δ (ppm) 8.31 (d, J = 8.1 Hz, 2 H); 8.21 (d, J = 8.1 Hz, 2 H); 7.95-7.98 (m, 2 H); 7.18 (d, J = 8.5 Hz, 1 H); 3.89 (s, 3 H); 2.08 (s, 6 H); 2.03 (s, 3 H); 1.72 (s, 6 H). | solide blanc |
| | 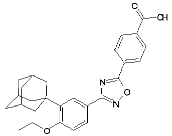 | acide 4-[3-(3-adamantan-1-yl-4-ethoxy-phenyl)-[1,2,4]oxadiazol-5-yl]-benzoique | B (figure 2) | 1H NMR (400 MHz, DMSO): δ (ppm) 8.13-8.18 (m, 4 H); 7.90 (m, 2 H); 7.15-7.16 (m, 1 H); 4.14 (m, 2 H); 2.13 (s, 6 H); 2.07 (s, 3 H); 1.75 (s, 6 H); 1.44 (m, 3 H). | solide blanc |
| | 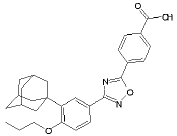 | acide 4-[3-(3-adamantan-1-yl-4-propoxy-phenyl)-[1,2,4]oxadiazol-5-yl]-benzoique | B (figure 2) | ¹H NMR (400 MHz, DMSO): δ (ppm) 8.31 (m, 2 H); 8.25 (m, 2 H); 7.99 (m, 2 H); 7.13 (m, 1 H); 4.00 (m, 2 H); 2.11 (s, 6 H); 2.01 (s, 3 H); 1.79 (m, 2 H); 1.70 (s, 6 H); 1.05 (m, 3 H). | solide blanc |
| | 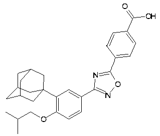 | acide 4-[3-(3-adamantan-1-yl-4-isobutoxy-phenyl)-[1,2,4]oxadiazol-5-yl]-benzoique | B (figure 2) | ¹H NMR (400 MHz, DMSO): δ (ppm) 8.30 (m, 2 H); 8.21 (m, J = 6.7 Hz, 2 H); 7.95 (m, 2 H); 7.15 (m, 1H); 3.88 (m, 2 H); (s, 6 H); 2.07 (s, 3 H); 1.75 (s, 6 H); 1.20 (m, 1H);1.08 (m, 6 H). | solide blanc |
| | 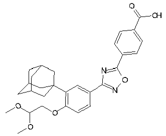 | acide 4-{3-[3-adamantan-1-yl-4-(2,2-dimethoxy-ethoxy)-phenyl]-[1,2,4]oxadiazol-5-yl}-benzoique | B (figure 2) | ¹H NMR (400 MHz, DMSO): δ (ppm) 8.30 (m, 4 H); 8.01-8.02 (m, 2 H); 7,20 (m, 2H); 4.86 (t, J = 5.3 Hz, 1 H); 4.13 (d, J = 5.3 Hz, 2 H); 2.13 (s, 6 H); 2.01 (s, 3 H); 1.71 (t, J = 15.8 Hz, 6 H). Un aromatique est masqué par un signal pyridine(?) | solide marron clair |
| | 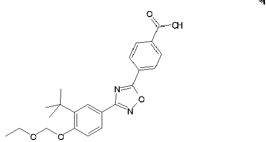 | acide 4-[3-(3-tert-butyl-4-ethoxymethoxy-phenyl)-[1,2,4]oxadiazol-5-yl]-benzoique | B (figure 2) | ¹H NMR (400 MHz, CDCl₃): δ(ppm) 8.29-8.32 (m, 4 H); 8.11 (d, J = 2.3 Hz, 1 H); 7.98 (dd, J = 8.5, 2.2 Hz, 1 H);7.28 (d, J = 8.5 Hz, 1 H); 5.36 (s, 2 H); 3.78 (q, J = 7.0 Hz, 2 H); 1.47 (s, 9 H); 1.27 (t, J = 7.0 Hz, 3 H). | solide blanc |
| | 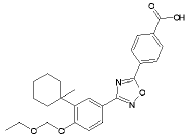 | acide 4-{3-[4-ethoxymethoxy-3-(1-methyl-cyclohexyl)-phenyl]-[1,2,4]oxadiazol-5-yl}-benzoique | B (figure 2) | ¹H NMR (400 MHz, CDCl₃): δ (ppm) 8.35 (d, J = 8.2 Hz, 2 H); 8.29-8.30 (d, 2 H); 8.14 (d, J = 2.3 Hz, 1 H); 7.98 (dd, J = 8.5, 2.3 Hz, 1 H); 7.28 (s, 1 H);5.34 (s, 2 H); 3.76 (q, J = 7.0 Hz, 2 H); 2.20 (m, 2 H); 1.82 (m, 2 H); 2.18 (m, 2 H);1.55-1.66 (m, 5 H); 1.42 (m, 1 H);1.27 (t, J = 7.0 Hz, 3 H). | solide blanc |
| | 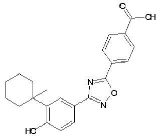 | acide 4-{3-[4-hydroxy-3-(1-methyl-cyclohexyl)-phenyl]-[1,2,4]oxadiazol-5-yl}-benzoique | B (figure 2) | ¹H NMR (400 MHz, DMSO): δ (ppm) 9.42 (s, 1 H); 8.19 (d, J = 8.1 Hz, 2 H); 8.12 (d, J = 8.1 Hz, 2 H); 7.93 (s, 1 H); 7.70 (d, J = 8.4 Hz, 1 H); 6.86 (d, J = 8.3 Hz, 1 H); 2.13 (m, 2 H); 1.69 (m, 2 H); 1.43 (m, 5 H); 1.33 (m, 1 H);1.28 (s, 3 H). | solide blanc |
| | 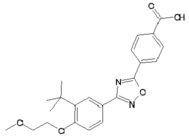 | acide 4-{3-[3-tert-butyl-4-(2-methoxy-ethoxy)-phenyl]-[1,2,4]oxadiazol-5-yl}-benzoique | B (figure 2) | ¹H NMR (400 MHz, DMSO): δ (ppm) 8.20 (d, J = 8.2 Hz, 2 H); 8.13 (d, J = 8.0 Hz, 2 H); 7.98 (s, 1 H); 7.88 (d, J = 8.5 Hz, 1 H); 6.92 (d, J = 8.5 Hz, 1 H); 4.14 (s, 2 H); 3.75 (s, 2 H); 3.37 (s, 3H); 1.37 (s, 9 H). | solide rose pale |
| | 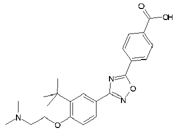 | acide 4-{3-[3-tert-butyl-4-(2-dimethylamino-ethoxy)-phenyl]-[1,2,4]oxadiazol-5-yl}-benzoique | B (figure 2) | ¹H NMR (400 MHz, DMSO): δ (ppm) 8.34 (s, 4 H); 8.16 (m, 2 H); 7.22 (m, 1 H); 4.16 (s, 2 H); 2.72 (s, 2 H); 2.23 (s, 6 H); 1.41 (s, 9 H). | solide blanc cassé |
| | 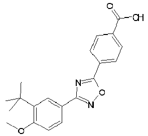 | acide 4-[3-(3-tert-butyl-4-methoxy-phenyl)-[1,2,4]oxadiazol-5-yl]-benzoique | B (figure 2) | ¹H NMR (400 MHz, DMSO): δ (ppm) 8.20 (d, J = 8.7 Hz, 2 H); 8.13 (d, J = 9.7 Hz, 2 H); 7.97 (s, 1 H); 7.90 (d, J = 9.8 Hz, 1 H); 6.93 (d, J = 9.0 Hz, 1 H); 3.85 (s, 3 H); 1.35 (s, 9 H). | solide blanc |
| | 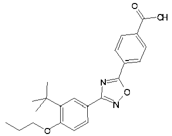 | acide 4-[3-(3-tert-butyl-4-propoxy-phenyl)-[1,2,4]oxadiazol-5-yl]-benzoique | B (figure 2) | ¹H NMR (400 MHz, DMSO): δ (ppm) 8.20 (d, J = 8.2 Hz, 2 H); 8.13 (d, J = 8.2 Hz, 2 H); 7.97 (s, 1 H); 7.88 (d, J = 8.5 Hz, 1 H); 6.91 (d, J = 8.5 Hz, 1 H); 3.92-4.00 (m, 2 H); 1.83 (m, 2 H); 1.38 (s, 9 H); 1.05 (t, J = 7.5 Hz, 3 H). | solide blanc |
| | 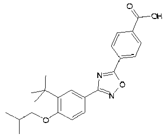 | acide 4-[3-(3-tert-butyl-4-isobutoxy-phenyl)-[1,2,4]oxadiazol-5-yl]-benzoique | B (figure 2) | ¹H NMR (400 MHz, DMSO): δ (ppm) 8.28 (d, J = 8.2 Hz, 2 H); 8.17 (d, J = 8.0 Hz, 2 H); 7.97 (s, 1 H); 7.92 (d, J = 8.6 Hz, 1 H); 7.12 (d, J = 8.6 Hz, 1 H); 3.88 (d, J = 6.2 Hz, 2 H); 2.15 (m, 1 H); 1.43 (s, 9 H); 1.08 (d, J = 6.7 Hz, 6 H). | solide beige |
| | 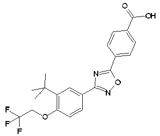 | acide 4-{3-[3-tert-butyl-4-(2,2,2-trifluoro-ethoxy)-phenyl]-[1,2,4]oxadiazol-5-yl}-benzoique | B (figure 2) | ¹H NMR (400 MHz, DMSO): δ (ppm) 8.31 (d, J = 8.1 Hz, 2 H); 8.19 (d, J = 8.1 Hz, 2 H); 8.03 (d, J = 2.3 Hz, 1 H); 7.98 (dd, J = 8.6 et 2.3 Hz, 1 H); 7.26 (d, J = 8.7 Hz, 1 H); 4.92 (q, J = 8.8 Hz, 2 H); 1.42 (s, 9 H). | solide blanc |
| | 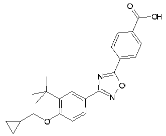 | acide 4-[3-(3-tert-butyl-4-cyclopropylmethoxy-phenyl)-[1,2,4]oxadiazol-5-yl]-benzoique | B (figure 2) | ¹H NMR (400 MHz, DMSO): δ (ppm) 8.29 (d, J = 8.1 Hz, 2 H); 8.18 (d, J = 8.1 Hz, 2 H); 7.98 (s, 1 H); 7.92 (d, J = 8.6 Hz, 1 H); 7.13 (d, J = 8.5 Hz, 1 H); 3.97 (d, J = 7.0 Hz, 2 H); 1.45 (s, 9 H); 1.32 (s, 1 H); 0.63 (d, J = 7.8 Hz, 2 H); 0.38 (d, J = 5.1 Hz, 2 H). | solide blanc |
| | 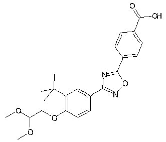 | acide 4-{3-[3-tert-butyl-4-(2,2-d imethoxy-ethoxy)-phenyl]-[1,2,4]oxadiazol-5-yl}-benzoique | B (figure 2) | ¹H NMR (400 MHz, DMSO): δ (ppm) 8.30 (s, 2 H); 8.19 (s, 2 H); 7.98 (m, 2 H); 7.21 (d, J = 8.5 Hz, 1 H); 4.82 (s, 1 H); 4.10 (s, 2 H); 3.39 (m, 6 H); 1.42 (s, 9 H). | solide rose pale |
| | 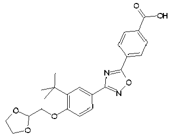 | acide 4-{3-[3-tert-butyl-4-([1,3]dioxolan-2-ylmethoxy)-phenyl]-[1,2,4]oxadiazol-5-yl]-benzoique | B (figure 2) | ¹H NMR (400 MHz, DMSO): δ (ppm) 8.30 (d, J = 8.2 Hz, 2 H); 8.18 (d, J = 8.2 Hz, 2 H); 7.98 (d, J = 2.2 Hz, 1 H); 7.93 (dd, J = 8.5, 2.1 Hz, 1 H); 7.19 (d, J = 8.6 Hz, 1 H); 5.32 (t, J = 3.8 Hz, 1 H); 4.14 (d, J = 3.8 Hz, 2 H); 3.95-3.96 (m, 4 H); 1.42 (s, 9 H). | solide blanc |
| | 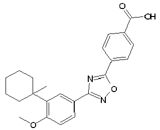 | acide 4-{3-[4-methoxy-3-(1-methyl-cyclohexyl)-phenyl]-[1,2,4]oxadiazol-5-yl}-benzoique | B (figure 2) | ¹H NMR (400 MHz, DMSO): δ (ppm) 8.27 (s, 2 H); 8.17 (s, 2 H); 7.98 (s, 1 H); 7.93 (br s, 1 H); 7.18 (s, 1 H); 3.87 (s, 3 H); 2.06 (br s, 2 H); 1.73 (br s, 2 H); 1.46-1.56 (m, 6 H); 1.29 (s, 3 H). | solide blanc |
| | 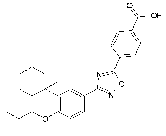 | acide 4-{3-[4-isobutoxy-3-(1-methyl-cyclohexyl)-phenyl]-[1,2,4]oxadiazol-5-yl}-benzoique | B (figure 2) | ¹H NMR (400 MHz, DMSO): ? (ppm) 8.27 (d, J = 8.0 Hz, 2 H); 8.17 (d, J = 8.0 Hz, 2 H); 8.00 (s, 1 H); 7.90 (d, J = 8.6 Hz, 1 H); 7.15 (d, J = 8.6 Hz, 1 H); 3.86 (d, J = 6.1 Hz, 2 H); 2.15 (br s, 2 H); 1.72 (br s, 2 H); 1.39-1.50 (m, 9 H); 1.05 (d, J = 6.7 Hz, 6 H). | solide blanc |
| | 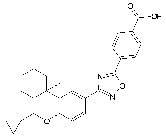 | acide 4-{3-[4-cyclopropylmethoxy-3-(1-methyl-cyclohexyl)-phenyl]-[1,2,4]oxadiazol-5-yl}-benzoique | B (figure 2) | ¹H NMR (400 MHz, DMSO): δ (ppm) 13.4 (br s, 1H); 8.28 (m, 2 H); 8.18 (m, 2 H); 7.99 (s, 1 H); 7.89 (d, J = 8.6 Hz, 1 H); 7.12 (d, J = 8.6 Hz, 1 H); 3.94 (d, J = 7.0 Hz, 2 H); 2.20 (m, 2 H); 1.57 (m, 2 H); 1.46-1.57 (m, 6 H); 1.35 (m, 4 H); 0.60 (s, 2 H); 0.35 (s, 2 H). | solide blanc |
| | 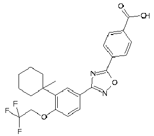 | acide 4-{3-[3-(1-methyl-cyclohexyl)-4-(2,2,2-trifluoro-ethoxy)-phenyl]-[1,2,4]oxadiazol-5-yl}-benzoique | B (figure 2) | ¹H NMR (400 MHz, DMSO): δ (ppm) 8.24 (d, J = 7.9 Hz, 2 H); 8.15 (d, J = 8.0 Hz, 2 H); 8.06 (d, 2.1 Hz, 1 H); 7.97 (dd, J = 8.8, 2.1 Hz, 1 H); 7.26 (d, J = 8.6 Hz, 1 H); 4.90 (q, J = 8.7 Hz, 2 H); (m, 2 H); 1.72 (m, 2 H); 1.52 (m, 6 H); 1.33 (s, 3 H). | solide blanc |
| | 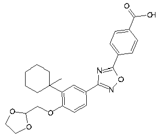 | acide 4-{3-[4-([1,3]dioxola n-2-y lmethoxy)-3-(1-methyl-cyclohexyl)-phenyl]-[1,2,4]oxadiazol-5-yl}-benzoique | B (figure 2) | | |
| | 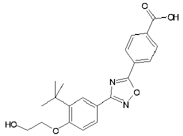 | acide 4-{3-[3-tert-Butyl-4-(2-hydroxy-ethoxy)-phenyl]-[1,2,4]oxadiazol-5-yl}-benzoique | B (figure 2) | | |
| | 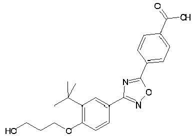 | acide 4-{3-[3-tert-Butyl-4-(3-hydroxy-propoxy)-phenyl]-[1,2,4]oxadiazol-5-yl}-benzoique | B (figure 2) | | |
| | 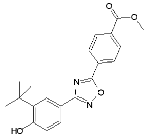 | 4-[3-(3-tert-butyl-4-hydroxy-phenyl)-[1,2,4]oxadiazol-5-yl]-benzoate de méthyle | B (figure 2) | | |
| | 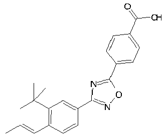 | acide 4-{3-[3-tert-butyl-4-((E)-propenyl)-phenyl]-[1,2,4]oxadiazol-5-yl}-benzoique | B (figure 2) | | |
| | 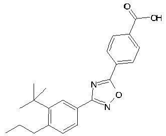 | acide 4-[3-(3-tert-butyl-4-propyl-phenyl)-[1,2,4]oxadiazol-5-yl]-benzoique | B (figure 2) | | |
| | 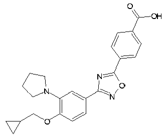 | acide 4-[3-(4-cyclopropylmethoxy-3-pyrrolidin-1-yl-phenyl)-[1,2,4]oxadiazol-5-yl]-benzoique | B (figure 2) | | |
| | 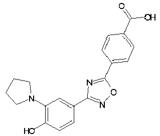 | acide 4-[3-(4-hydroxy-3-pyrrolidin-1-yl-phenyl)-[1,2,4]oxadiazol-5-yl]-benzoique | B (figure 2) | | |
| | 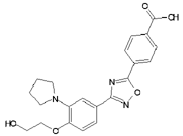 | acide 4-{3-[4-(2-hydroxy-ethoxy)-3-pyrrolidin-1-yl-phenyl]-[1,2,4]oxadiazol-5-yl}-benzoique | B (figure 2) | | |
| | 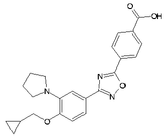 | acide 4-[3-(4-Cyclopropylmethoxy-3-pyrrolidin-1-yl-phenyl)-[1,2,4]oxadiazol-5-yl]-benzoique | B (figure 2) | | |
| | 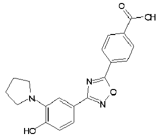 | acide 4-[3-(4-Hydroxy-3-pyrrolidin-1-yl-phenyl)-[1,2,4]oxadiazol-5-yl]-benzoique | B (figure 2) | | |
| | 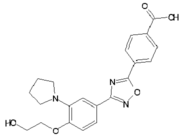 | 4-{3-[4-(2-hydroxy-ethoxy)-3-pynolidin-1-yl-phenyl]-[1,2,4]oxadiazol-5-yl}-benzoic acid | B (figure 2) | | |
| | 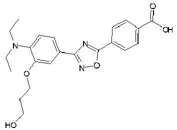 | acide 4-{3-[4-diéthylamino-3-(3-hydroxy-propoxy)-phenyl]-[1,2,4]oxadiazol-5-yl}-benzoique | B (figure 2) | | |
| | 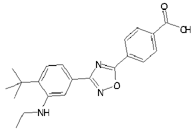 | acide 4-[3-(4-tert-butyl-3-éthylamino-phényl)-[1,2,4]oxadiazol-5-yl] benzoique | B (figure 2) | | |
| | 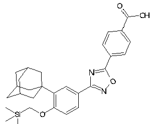 | acide 4-[3-(3-adamantan-1-yl-4-trimethylsilanylmethoxy-phenyl)-[1,2,4]oxadiazol-5-yl]-benzoique | B (figure 2) | | |
| | 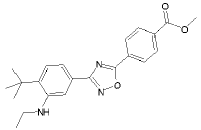 | 4-[3-(4-tert-butyl-3-éthylamino-phenyl)-[1,2,4]oxadiazol-5-yl]-benzoate de méthyle | B (figure 2) | | |

### C : préparation des composés pyrazoles décrits dans la figure 3

### Exemple 6 : acide 4-[5-(3-adamantan-1-yl-4-hydroxy-phényl)-4H-pyrazol-3-yl]-benzoique

### 6.1 : 3-adamantan-1-yl-4-(2-méthoxy-éthoxyméthoxy)-benzoate de méthyle

1,7g (42 mmoles) d'hydrure de sodium à 60% dans l'hexane sont additionnés à une solution de 10g (35 mmoles) de 3-adamantan-1-yl-4-hydroxy-benzoate de méthyle (préparé comme décrit dans l'exemple 1.1) dans 50ml de tétrahydrofurane et 50ml de diméthylformamide, préalablement refroidie à 0°C. Le milieu réactionnel est agité pendant 20 minutes puis 4,4ml (38 mmoles) de 1-chlorométhoxy-2-méthoxy-éthane sont additionnés goutte à goutte. Le milieu réactionnel est agité de 0°C à température ambiante pendant 2 heures. De l'eau est additionnée et le milieu réactionnel est extrait à l'acétate d'éthyle. La phase organique est séchée sur sulfate de magnésium, filtrée, évaporée. 14,8g de résidu brut sont obtenus et purifiés par chromatographie sur gel de silice élué avec un mélange heptane / acétate d'éthyle 7/3. 12,8g (99%) de 3-adamantan-1-yl-4-(2-méthoxy-éthoxyméthoxy)-benzoate de méthyle sont obtenus.

### 6.2 : acide 3-adamantan-1-yl-4-(2-méthoxy-éthoxyméthoxy)-benzoique

2,8g (69 mmoles) d'hydroxyde de sodium sont ajoutés à une solution de 12,8g (34 mmoles) de 3-adamantan-1-yl-4-(2-méthoxy-éthoxyméthoxy)-benzoate de méthyle dans 90ml de tétrahydrofurane, 30ml de méthanol et 1ml d'eau. Le milieu réactionnel est chauffé à reflux pendant 8 heures. Après addition d'eau, le milieu réactionnel est acidifié à pH6 puis extrait à l'acétate d'éthyle. La phase organique est lavée à l'eau, séchée sur sulfate de magnésium, filtrée, évaporée. 12,4g (97%) d'acide 3-adamantan-1-yl-4-(2-méthoxy-éthoxyméthoxy)-benzoique sont obtenus.

### 6.3 : 1-[3-adamantan-1-yl-4-(2-méthoxy-éthoxyméthoxy)-phényl]-éthanone

101ml (101 mmoles) d'une solution de méthyllithium dans le tétrahydrofurane de concentration 1M sont additionnés goutte à goutte à une solution, préalablement refroidie à - 78°C, de 12,1g (34 mmoles) d'acide 3-adamantan-1-yl-4-(2-méthoxy-éthoxyméthoxy)-benzoique dans 90ml de tétrahydrofurane et 90ml d'éther diéthylique. Le milieu réactionnel est agité de -78°C à température ambiante pendant 18 heures. 9ml (75 mmoles) de chlorure de triméthylsilyle sont ajoutés et le milieu réactionnel est à nouveau agité pendant 45 minutes. 100ml d'eau suivis de 100ml d'une solution aqueuse d'acide chlorhydrique de concentration 1M sont additionnés puis le milieu réactionnel est extrait à l'éther diéthylique. La phase organique est lavée à l'eau, séchée sur sulfate de magnésium, filtrée, évaporée. 19g de résidu brut sont obtenus et purifiés par chromatographie sur gel de silice élués avec un mélange heptane / acétate d'éthyle 6/4. 12g (98%) de 1-[3-adamantan-1-yl-4-(2-méthoxy-éthoxyméthoxy)-phényl]-éthanone sont obtenus.

### 6.4 : 4-{3-[3-adamantan-1-yl-4-(2-méthoxy-éthoxyméthoxy)-phényl]-3-oxo-propionyl}-benzoate de méthyle

65ml (130 mmoles) d'une solution commerciale de lithium diisopropylamide dans du tétrahydrofurane de concentration 2M sont additionnés goutte à goutte à une solution, préalablement refroidie à -78°C, de 12g (33 mmoles) de 1-[3-adamantan-1-yl-4-(2-méthoxy-éthoxyméthoxy)-phényl]-éthanone dans 90ml de térahydrofuranne. Après agitation à -78°C pendant 1 heure, une solution de 7,2g (36 mmoles) de 4-chlorocarbonyl-benzoate de méthyle dans 70ml de dioxanne est additionnée goutte à goutte. Le milieu réactionnel est agité de -78°C à température ambiante pendant 18 heures. Après addition d'eau et neutralisation à pH5-6 par addition d'une solution aqueuse d'acide chlorhydrique de concentration 1M, le milieu réactionnel est extrait à l'acétate d'éthyle. La phase organique est lavée à l'eau, séchée sur sulfate de magnésium, filtrée, évaporée. 20,5g de résidu brut sont obtenus et purifiés par chromatographie sur gel de silice, élués avec un mélange dichlorométhane / acétate d'éthyle 99/1. 5g (29%) de 4-{3-[3-adamantan-1-yl-4-(2-méthoxy-éthoxyméthoxy)-phényl]-3-oxo-propionyl}-benzoate de méthyle sont obtenus.

### 6.5 : 4-{5-[3-adamantan-1-yl-4-(2-méthoxy-éthoxyméthoxy)-phényl]-4H-pyrazol-3-yl}-benzoate de méthyle

0,5ml (10,4 mmoles) d'hydrazine hydrate sont ajoutés à une solution de 2,7g (5,2 mmoles) de 4-{3-[3-adamantan-1-yl-4-(2-méthoxy-éthoxyméthoxy)-phényl]-3-oxo-propionyl}-benzoate de méthyle dans 35ml de méthanol. Le milieu réactionnel est agité à température ambiante pendant 18 heures. Après addition d'eau, le milieu réactionnel est extrait à l'acétate d'éthyle. La phase organique est lavée à l'eau, séchée sur sulfate de magnésium, filtrée, évaporée. Le résidu obtenu est recristallisé dans un mélange heptane/ éther diéthylique (1/1). 2,7g (75%) de 4-{5-[3-adamantan-1-yl-4-(2-méthoxy-éthoxyméthoxy)-phényl]-4H-pyrazol-3-yl}-benzoate de méthyle sont obtenus sous forme d'un solide jaune pâle.

### 6.6 : 3-[3-adamantan-1-yl-4-(2-méthoxy-éthoxyméthoxy)-phényl]-5-(4-méthoxycarbonyl-phényl)-pyrazole-1-carboxylate d'éthyle

0,2ml (1,4 mmoles) de triéthylamine puis 0,2ml (1,8 mmoles) de chloroformate d'éthyle sont ajoutés à une solution de 0,3g (0,6 mmole) de 4-{5-[3-adamantan-1-yl-4-(2-méthoxy-éthoxyméthoxy)-phényl]-4H-pyrazol-3-yl}-benzoate de méthyle. Le milieu réactionnel est agité à température ambiante pendant 18 heures. Après addition d'eau, le milieu réactionnel est extrait au dichlorométhane. La phase organique est séchée sur sulfate de magnésium, filtrée, évaporée. 0,5g de résidu brut sont obtenus et purifiés par chromatographie sur gel de silice élué avec un mélange heptane / acétate d'éthyle 8/2. 0,3g (88%) de 3-[3-adamantan-1-yl-4- (2- méthoxy-éthoxyméthoxy)-phényl]-5-( 4-méthoxycarbonyl-phényl)-pyrazole-1-carboxylate d'éthyle sont obtenus.

### 6.7 : 3-(3-adamantan-1-yl-4-hydroxy-phényl)-5-(4-méthoxycarbonyl-phényl)-pyrazole-1-carboxylate d'éthyle

0,3g (0,5 mmole) de 3-[3-adamantan-1-yl-4-(2-méthoxy-éthoxyméthoxy)-phényl]-5-(4-méthoxycarbonyl-phényl)-pyrazole-1-carboxylate d'éthyle sont placés en solution dans 3ml de méthanol, 10ml de tétrahydrofurane et 0,3ml d'acide sulfurique concentré puis agité à température ambiante pendant 48 heures. De l'eau est additionnée et le milieu réactionnel est extrait à l'acétate d'éthyle. La phase organique est séchée sur sulfate de magnésium, filtrée, évaporée. 264mg (100%) de 3-(3-adamantan-1-yl-4-hydroxy-phényl)-5-(4-méthoxycarbonyl-phényl)-pyrazole-1-carboxylate d'éthyle sont obtenus.

### 6.8 : acide 4-[5-(3-adamantan-1-yl-4-hydroxy-phényl)-2H-pyrazol-3-yl]-benzoique

0,8ml (0,8 mmole) d'une solution aqueuse d'hydroxyde de sodium de concentration 1M sont ajoutés à une solution de 130mg (0,3 mmole) de 3-(3-adamantan-1-yl-4-hydroxy-phényl)-5-(4-méthoxycarbonyl-phényl)-pyrazole-1-carboxylate d'éthyle dans 5ml de tétrahydrofurane et 0,5ml d'eau. Le milieu réactionnel est agité à température ambiante pendant 4 heures. Après addition d'eau et acidification avec une solution aqueuse d'acide chlorhydrique de concentration 1M jusqu'à pH4-5, le précipité formé est filtré, lavé à l'eau et séché. 130mg de résidu brut sont obtenus et purifiés par chromatographie sur gel de silice élué avec un mélange heptane / acétate d'éthyle 1/1. 80mg (74%) d'acide 4-[5-(3-adamantan-1-yl-4-hydroxy-phényl)-2H-pyrazol-3-yl]-benzoique sont obtenus.

### Exemple 7 : acide 4-{5-[3-adamantan-1-yl-4-(2-méthoxy-éthoxyméthoxy)-phényl]-4H-pyrazol-3-yl }-benzoique

0,7ml (0,7 mmole) d'une solution aqueuse d'hydroxyde de sodium de concentration 1M sont ajoutés à une solution de 130mg (0,2 mmole) de 3-[3-adamantan-1-yl-4-(2-méthoxy-éthoxyméthoxy)-phényl]-5-(4-méthoxycarbonyl-phényl)-pyrazole-1-carboxylate d'éthyle (préparé comme décrit en 6.6) dans 7ml de tétrahydrofurane et 0,5ml d'eau. Le milieu réactionnel est chauffé à 60°C pendant 17 heures. Après refroidissement et acidification à pH3 avec une solution aqueuse d'acide chlorhydrique de concentration 1M, le milieu réactionnel est filtré. Le précipité est lavé à l'eau, séché sous vide. 93mg (84%) d'acide 4-{5-[3-adamantan-1-yl-4-(2-méthoxy-éthoxyméthoxy)-phényl]-4H-pyrazol-3-yl}-benzoique sont obtenus sous forme d'un solide blanc.

| n° exemple dans partie expérimentale | Structure chimique | Nom chimique | Voie de synthèse | RMN 1H | aspect |
|---|---|---|---|---|---|
| exemple 6 | 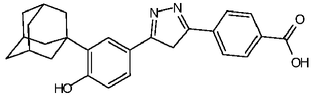 | acide 4-[5-(3-adamantan-1-yl-4-hydroxy-phényl)-4H-pyrazol-3-yl]-benzoique | C (figure 3) | ¹H NMR (400 MHz, CDCl₃): δ (ppm) 7.78 (d, J = 7.9 Hz, 2 H); 7.62 (d, J = 8.0 Hz, 2 H); 7.13 (d, J = 8.1 Hz, 1 H); 6.58 (d, J = 8.1 Hz, 1 H); 6.50 (s, 1 H); 1.91 (s, 6 H); 1.81 (s, 3 H); 1.51 (s, 6 H). | solide jaune |
| exemple 7 | 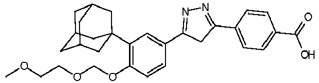 | acide 4-{5-[3-adamantan-1-yl-4-(2-méthoxy-éthoxyméthoxy)-phényl]-4H-pyrazol-3-yl}-benzoique | C( figure 3) | ¹H NMR (400 MHz, CDCl₃): δ (ppm) 7.91 (d, J = 8.0 Hz, 2 H); 7.74 (d, J = 8.1 Hz, 2 H); 7.46 (s, 1 H); 7.39 (d, J = 8.6 Hz, 1 H); 7.03 (d, J = 8.5 Hz, 1 H); 6.67 (s, 1 H); 5.18 (s, 2 H); 3.70 (t, J = 4.4 Hz, 2 H); 3.44 (t, J = 4.4 Hz, 2 H); 3.23 (s, 3 H); 1.99 (s, 6 H); 1.93 (s, 4 H); 1.62 (s, 6 H). | solide jaune |
| | 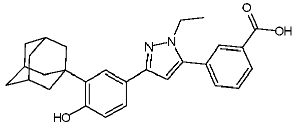 | acide 3-[5-(3-adamantan-1-yl-4-hydroxy-phényl)-2-éthyl-2H-pyrazol-3-yl]-benzoique | C( figure 3) | | |

### D: préparation des composés isoxazoles décrits dans la figure 4

### Exemple 8 : acide 4-[3-(3-adamantan-1-yl-4-hydroxy-phényl)-isoxazol-5-yl]-benzoique

### 8.1 : 4-[3-(3-adamantan-1-yl-4-hydroxy-phényl)-isoxazol-5-yl]-benzoate de méthyle

Une solution de 2,1g (4 mmoles) de 3-[3-adamantan-1-yl-4-(2-méthoxy-éthoxyméthoxy)-phényl]-5-(4-méthoxycarbonyl-phényl)-pyrazole-1-carboxylate d'éthyle (préparé comme décrit en 6.6) dans 50ml d'éthanol est ajoutée à une solution de 0,7g (10 mmoles) de chlorhydrate d'hydroxylamine dans 20ml d'eau. Le milieu réactionnel est chauffé à reflux pendant 18 heures. Après refroidissement, le précipité formé est filtré puis séché sous vide. 1,5g (48%) de 4-[3-(3-adamantan-1-yl-4-hydroxy-phényl)-isoxazol-5-yl]-benzoate de méthyle sont obtenus.

### 8.2 : acide 4-[3-(3-adamantan-1-yl-4-hydroxy-phényl)-isoxazol-5-yl]-benzoique

500µl (0,5 mmole) d'une solution aqueuse d'hydroxyde de sodium de concentration 1M sont ajoutés à une solution de 140mg (0,3 mmole) de 4-[3-(3-adamantan-1-yl-4-hydroxy-phényl)-isoxazol-5-yl]-benzoate de méthyle dans 10ml de tétrahydrofurane et 1ml d'eau. Après agitation à température ambiante pendant 18 heures, de l'eau est additionnée puis le milieu est acidifié avec une solution aqueuse d'acide chlorhydrique de concentration 1M. Le précipité formé est filtré puis séché sous vide. Le résidu obtenu est purifié sur gel de silice élué avec un mélange heptane / acétate d'éthyle 50/50. 80mg (60%) d'acide 4-[3-(3-adamantan-1-yl-4-hydroxy-phényl)-isoxazol-5-yl]-benzoique sont obtenus sous forme d'un solide blanc.

### Exemple 9 : acide 4-[3-(3-adamantan-1-yl-4-propoxy-phényl)-isoxazol-5-yl]-benzoique

### 9.1 : 4-[3-(3-adamantan-1-yl-4-propoxy-phényl)-isoxazol-5-yl]-benzoate de méthyle

170mg (0,9 mmole) de carbonate de césium sont ajoutés à une solution de 320mg (0,7 mmole) de 4-[3-(3-adamantan-1-yl-4-hydroxy-phényl)-isoxazol-5-yl]-benzoate de méthyle (préparé comme décrit en 8.1), 60µl (0,9 mmole) d'iodure de propyle dans 7ml de diméthylformamide. Le milieu réactionnel est chauffé à 80°C pendant 18 heures, refroidi puis filtré. Après évaporation à sec du filtrat, le résidu brut obtenu est purifié par chromatographie sur gel de silice élué avec un mélange heptane / acétate d'éthyle 50/50. 140mg (42%) de 4-[3-(3-adamantan-1-yl-4-propoxy-phényl)-isoxazol-5-yl]-benzoate de méthyle sont obtenus sous la forme d'un solide blanc.

### 9.2 : acide 4-[3-(3-adamantan-1-yl-4-propoxy-phényl)-isoxazol-5-yl]-benzoique

500µl (0,5 mmole) d'une solution aqueuse d'hydroxyde de sodium de concentration 1M sont ajoutés à une solution de 140mg (0,3 mmole) de 4-[3-(3-adamantan-1-yl-4-propoxy-phényl)-isoxazol-5-yl]-benzoate de méthyle dans un mélange de 7ml de tétrahydrofurane et 2ml d'eau. Après agitation à température ambiante pendant 18 heures, le tétrahydrofurane est évaporé à sec puis le milieu réactionnel est acidifié à pH2-3 avec une solution aqueuse d'acide chlorhydrique 1M. Le produit formé précipite, est filtré et rincé avec du méthanol. 130mg (94%) d'acide 4-[3-(3-adamantan-1-yl-4-propoxy-phényl)-isoxazol-5-yl]-benzoique sont obtenus.

| n° exemple dans partie expérimentale | Structure chimique | nom du composé | Voie de synthèse | RMN 1H | aspect |
|---|---|---|---|---|---|
| exemple 8 | 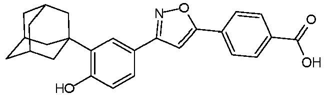 | acide 4-[3-(3-adamantan-1-yl-4-hydroxy-phényl)-isoxazol-5-yl]-benzoique | D (figure 4) | ¹H NMR (400 MHz, CDCl₃): δ (ppm) 9.01 (s, 1 H); 8.02 (d, J = 7.9 Hz, 2 H); 7.81 (d, J = 8.2 Hz, 2 H); 7.51 (s, 1 H); 7.39 (d, J = 8.3 Hz, 1 H); 6.79 (d, J = 8.4 Hz, 1 H); 6.61 (s, 1 H); 2.06 (s, 6 H); 1.97 (s, 3 H); 1.67 (s, 6 H). | solide blanc |
| exemple 9 | 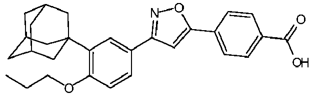 | 4-[3-(3-adamantan-1-yl-4-propoxy-phényl)-isoxazol-5-yl]-benzoique | D (figure 4) | ¹H NMR (400 MHz, DMSO): δ (ppm) 8.06-8.09 (m, 4 H); 7.70-7.74 (m, 2 H); 7.59 (s, 1 H); 7.14 (d, J = 8.8 Hz, 1 H); 4.05 (t, J = 6.3 Hz, 2 H); 2.15 (s, 6 H); 2.09 (s, 3 H); 1.85 (m, 2 H); 1.77 (s, 6 H); 1.11 (t, J = 7.3 Hz, 3 H). | solide blanc |
| | 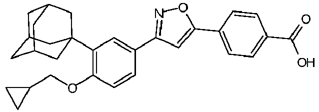 | acide 4-[3-(3-adamantan-1-yl-4-cyclopropylméthoxy-phényl)-is oxazol-5-yl]-benzoique | D (figure 4) | ¹H NMR (400 MHz, DMSO): δ (ppm) 8.07 (d, J = 8.0 Hz, 2 H); 8.00 (d, J = 8.2 Hz, 2 H); 7.68-7.72 (m, 2 H); 7.57 (s, 0.6 H); 7.09 (d, J = 8.8 Hz, 1 H); 6.87 (s, 0.4 H); 3.95 (d, J = 6.9 Hz, 2 H); 2.17 (s, 6 H); 2.09 (s, 3 H); 1.77 (s, 6 H); 1.17 (m, 1 H); 0.63 (d, J= 7.7 Hz, 2 H); 0.39 (d, J= 5.1 Hz, 2 H). | solide blanc |
| | 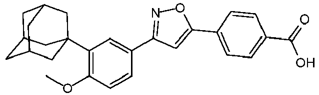 | acide 4-[3-(3-adamantan-1-yl-4-méthoxy-phényl)-isoxazol-5-yl]-benzoique | D (figure 4) | ¹H NMR (400 MHz, DMSO): δ (ppm) 8.08 (m, 4 H); 7.64-7.73 (m, 3 H); 7.17 (d, J = 8.9 Hz, 1 H); 3.89 (s, 3 H); 2.11 (br s, 9 H); 1.77 (s, 6 H). | solide blanc |
| | 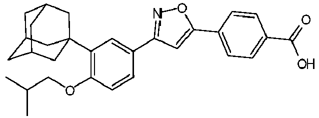 | acide 4-[3-(3-adamantan-1-yl-4-isobutoxy-phényl)-isoxazol-5-yl]-benzoique | D (figure 4) | ¹H NMR (400 MHz, DMSO): δ (ppm) 8.06 (d, J = 7.9 Hz, 2 H); 7.92 (d, J = 8.7 Hz, 2 H); 7.63 (s, 2 H); 7.15 (s, 1 H); 6.98 (d, J = 8.7 Hz, 1 H); 3.81 (s, 2 H); 2.18 (s, 1 H); 2.13 (s, 6 H); 2.07 (s, 3 H); 1.75 (s, 6 H); 1.09 (d, J = 6.6 Hz, 6 H) | solide jaune pale |
| | 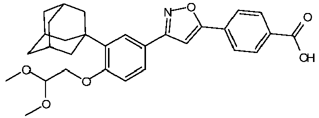 | 4-{3-[3-adamantan-1-yl-4-(2,2-diméthoxy-éthoxy)-phényl]-isoxazol 5-yl}-benzoique | D (figure 4) | ¹H NMR (400 MHz, DMSO): δ (ppm) 8.05-8.08 (m, 4 H); 7.70-7.74 (m, 3 H); 7.61 (s, 1 H); 7.17 (d, J = 8.9 Hz, 1 H); 4.83 (t, J = 5.2 Hz, 1 H); 4.08 (d, J = 5.3 Hz, 2 H); 3.40 (s, 6 H); 2.11 (m, 12 H); 1.77 (s, 6 H). | solide blanc |
| | 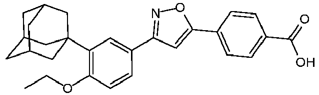 | 4-[3-(3-adamantan-1-yl-4-éthoxy-phényl)-isoxazol-5-yl]-benzoique | D (figure 4) | | |
| | 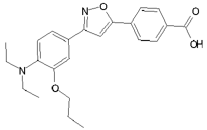 | acide 4-[3-(4-diéthylamino-3-propoxy-phényl)-isoxazol-5-yl]-benzoique | | | |

### E: préparation des composés thiazoles décrits dans la figure 5

### Exemple 10 : acide 4-[5-(3-adamantan-1-yl-4-éthoxyméthoxy-phényl)-thiazol-2-yl]-benzoique

### 10.1 : 1-(5-bromo-2-éthoxyméthoxy-phényl)-adamantane

4,7g (120 mmoles) d'hydrure de sodium à 60% dans l'hexane sont ajoutés par portions à une solution préalablement refroidie à 0°C, de 30g (100 mmoles) de 2-adamantan-1-yl-4-bromo-phénol dans un mélange de 300ml de diméthylformamide et 300ml de tétrahydrofurane. Après agitation à 0°C pendant 30 minutes, 10,6ml (120 mmoles) de chlorométhoxy-éthane sont ajoutés goutte à goutte. Le milieu réactionnel est agité de 0°C à température ambiante pendant 4 heures. Après addition d'eau, le milieu réactionnel est extrait à l'acétate d'éthyle. La phase organique est abondamment lavée à l'eau, séchée sur sulfate de magnésium, filtrée, évaporée. 35g (95%) de 1-(5-bromo-2-éthoxyméthoxy-phényl)-adamantane.

### 10.2 : acide 3-(adamantan-1-yl)-4-éthoxyméthoxy-phényl- boronique

28ml (70 mmoles) d'une solution de butyllithium 2,5M dans l'hexane sont additionnés goutte à goutte à une solution, préalablement refroidie à -78°C, de 21g (60 mmoles) de 1-(5-bromo-2-éthoxyméthoxy-phényl)-adamantane dans 210ml de tétrahydrofurane. Après agitation pendant 1 heure à -78°C, 15ml (60 mmoles) de tri-isopropyl-borate sont additionnés. Le milieu est agité 1 heure à -78°C puis à température ambiante pendant 5 heures. Après refroidissement à 0°C, 80ml (80 mmoles) d'une solution aqueuse d'acide chlorhydrique de concentration 1M sont additionnés, le milieu est agité pendant 30 minutes et extrait à l'acétate d'éthyle. La phase organique est lavée avec une solution saturée de chlorure de sodium, séchée sur sulfate de magnésium, filtrée puis évaporée. Le résidu obtenu est repris dans l'heptane puis le précipité est filtré. 13,4g (73%) d'acide 3-(adamantan-1-yl)-4-éthoxyméthoxy-phényl- boronique sont obtenus.

### 10.3 : 4-thiazol-2-yl-benzoate d'éthyle

31ml (20 mmoles) d'iodure de triméthylsilyle puis une solution de 30g (200 mmoles) de 2-bromothiazole dans 75ml de tétrahydrofurane sont ajoutés à une suspension de 36g (550 mmoles) de Zinc en poudre dans 90ml de tétrahydrofurane, préalablement activée par 4,5ml (150 mmoles) de 1,2-dibromoéthane. Le milieu réactionnel est agité pendant 15 minutes, puis une solution de 46ml (300 mmoles) de 4-iodobenzoate d'éthyle dans 375ml de tétrahydrofurane et enfin 2,7g de tétrakis-triphénylphosphine palladium sont ajoutés. Le milieu réactionnel est alors chauffé à 60°C pendant 18 heures. Après refroidissement et filtration sur célite, le filtrat est évaporé à sec. 156g de résidu brut sont obtenus et purifiés par chromatographie sur gel de silice élué avec un mélange heptane / acétate d'éthyle 9/1. 37g (93%) de 4-thiazol-2-yl-benzoate d'éthyle sont obtenus.

### 10.4 : 4-(5-bromo-thiazol-2-yl)-benzoate d'éthyle

Une solution de 11ml (200 mmoles) de brome dans 95ml de chloroforme est additionnée goutte à goutte à une solution de 37g (160 mmoles) de 4-thiazol-2-yl-benzoate d'éthyle dans 400ml de chloroforme, préalablement refroidie à 0°C. Le milieu réactionnel est ensuite agité de 0°C à température ambiante pendant 24 heures. Après addition d'eau et extraction au dichlorométhane, la phase organique est séchée sur sulfate de sodium, filtrée, évaporée. 59,6g de résidu brut sont obtenus et purifiés par chromatographie sur gel de silice élué avec un mélange heptane / acétate d'éthyle 9/1.16,3g (31%) de 4-(5-bromo-thiazol-2-yl)-benzoate d'éthyle sont obtenus sous forme d'un solide rose pâle.

### 10.5 : 4-[5-(3-adamantan-1-yl-4-éthoxyméthoxy-phényl)-thiazol-2-yl]-benzoate d'éthyle

20ml (40 mmoles) d'une solution aqueuse de carbonate de potassium de concentration 2M sont ajoutés goutte à goutte à un mélange de 5g (16 mmoles) de 4-(5-bromo-thiazol-2-yl)-benzoate d'éthyle et 7g (21 mmoles) d'acide 3-(adamantan-1-yl)-4-éthoxyméthoxy-phényl-boronique dans 50ml de diméthoxy-éthane. Le milieu réactionnel est ensuite dégazé sous azote puis 0,9g (0,8 mmole) de tétrakis-triphénylphosphine palladium sont ajoutés et le milieu réactionnel est chauffé à 100°C pendant 4 heures. Après refroidissement et addition d'eau, le milieu est extrait au dichlorométhane, puis séché sur sulfate de sodium, filtré, évaporé. 13,4g de résidu brut sont obtenus et purifiés par chromatographie sur gel de silice élué avec un mélange heptane / acétate d'éthyle 95/5. 7,5g (87%) de 4-[5-(3-adamantan-1-yl-4-éthoxyméthoxy-phényl)-thiazol-2-yl]-benzoate d'éthyle sont obtenus.

### 10.6 : acide 4-[5-(3-adamantan-1-yl-4-éthoxyméthoxy-phényl)-thiazol-2-yl]-benzoique

58mg (1,4 mmole) d'hydroxyde de sodium en poudre sont ajoutés à une solution de 500mg (1 mmole) de 4-[5-(3-adamantan-1-yl-4-éthoxyméthoxy-phényl)-thiazol-2-yl]-benzoate d'éthyle dans 25ml de tétrahydrofurane, 2,5ml de méthanol et 0,5ml d'eau. Le milieu réactionnel est agité à température ambiante pendant 4 heures puis de l'eau est additionnée, le milieu est acidifié à pH3 avec une solution aqueuse d'acide chlorhydrique de concentration 1M et extrait à l'acétate d'éthyle. La phase organique est séchée sur sulfate de magnésium, filtrée, évaporée. 430mg de résidu brut sont obtenus et recristallisés dans du toluène. 240mg (51%) d'acide 4-[5-(3-adamantan-1-yl-4-éthoxyméthoxy-phényl)-thiazol-2-yl]-benzoique sont obtenus.

### Exemple 11 : acide 4-[5-(3-adamantan-1-yl-4-isobutoxy-phényl)-thiazol-2-yl]-benzoique

### 11.1 : 4-[5-(3-adamantan-1-yl-4-hydroxy-phényl)-thiazol-2-yl]-benzoate d'éthyle

0,7ml d'acide sulfurique concentré sont ajoutés à une solution de 7g (13,5 mmoles) de 4-[5-(3-adamantan-1-yl-4-éthoxyméthoxy-phényl)-thiazol-2-yl]-benzoate d'éthyle (préparé comme décrit dans l'exemple 10.5) dans 35ml de tétrahydrofurane et 35ml d'éthanol. Le milieu réactionnel est agité pendant 4 heures à température ambiante puis pendant 8 heures à reflux. Après refroidissement, de l'eau est ajoutée et le milieu réactionnel est extrait à l'acétate d'éthyle. La phase organique est séchée sur sulfate de sodium, filtrée, évaporée. 7,8g (100%) de 4-[5-(3-adamantan-1-yl-4-hydroxy-phényl)-thiazol-2-yl]-benzoate d'éthyle sont obtenus.

### 11.2 : 4-[5-(3-adamantan-1-yl-4-isobutoxy-phényl)-thiazol-2-yl]-benzoate d'éthyle

Un mélange de 100mg (0,2 mmole) de 4-[5-(3-adamantan-1-yl-4-hydroxy-phényl)-thiazol-2-yl]-benzoate d'éthyle, 36mg (0,3 mmole) de 1-bromo-2-méthylpropane, 13,4mg (0,2 mmole) d'hydroxyde de potassium dans 2ml de diglyme est chauffé à reflux pendant 5 heures. Après refroidissement et dilution à l'eau, le milieu réactionnel est extrait à l'acétate d'éthyle. La phase organique est séchée sur sulfate de magnésium, filtrée, évaporée. Le résidu brut obtenu est purifié par chromatographie sur gel de silice élué avec un mélange heptane / acétate d'éthyle 9/1. 104mg (93%) de 4-[5-(3-adamantan-1-yl-4-isobutoxy-phényl)-thiazol-2-yl]-benzoate d'éthyle sont obtenus.

### 11.3 : acide 4-[5-(3-adamantan-1-yl-4-isobutoxy-phényl)-thiazol-2-yl]-benzoique

Selon un protocole identique à celui de l'exemple 10.6, à partir de 104mg (0,2 mmole), 95mg (97%) d' acide 4-[5-(3-Adamantan-1-yl-4-isobutoxy-phényl)-thiazol-2-yl]-benzoique sont obtenus sous forme d'un solide blanc.

### Exemple 12 : Acide 4-[5-(3-tert-butyl-4-méthoxy-phényl)-thiazol-2-yl]-benzoique

### 12.1 : 2-tert-butyl-4-iodo-phénol

16,5g (73 mmoles) de N-iodosuccinimide sont ajoutés par portions à une solution, préalablement refroidie à 0°C, de 10g (67 mmoles) de 2-tertbutylphénol et 1,5ml (20 mmoles) d'acide trifluoroacétique dans 250ml d'acétonitrile. Après agitation à température ambiante pendant 18 heures, de l'acétate d'éthyle est additionné et le milieu réactionnel est lavé avec une solution aqueuse saturée de chlorure de sodium, puis avec une solution de thiosulfate de sodium. La phase organique est séchée sur sulfate de magnésium, filtrée, évaporée. Le résidu brut est purifié par chromatographie sur colonne de silice élué avec un mélange heptane / acétate d'éthyle 95/5. 7,5g (37%) de 2-tert-butyl-4-iodo-phénol sont obtenus.

### 12.2 : 2-tert-butyl-4-iodo-1-(2-méthoxy-éthoxyméthoxy)-benzène

1,3g (33 mmoles) d'hydrure de sodium à 60% dans l'hexane sont ajoutés par portions à une solution, préalablement refroidie à 0°C, de 7,5g (27 mmoles) de 2-tert-butyl-4-iodo-phénol, 65ml de tétrahydrofurane et 65ml de diméthylformamide. Après agitation à 0°C pendant 30 minutes, 3,7ml (33 mmoles) de 1-chlorométhoxy-2-méthoxy-éthane sont additionnés goutte à goutte. Le milieu réactionnel est agité à température ambiante pendant 1 heure puis versé dans la glace et extrait à l'acétate d'éthyle. La phase organique est lavée avec une solution saturée de chlorure de sodium, séchée sur sulfate de magnésium, filtrée, évaporée.13,6g de résidu brut sont obtenus et purifiés par chromatographie sur gel de silice élué avec de l'heptane. 7,3g (74%) de 2-tert-butyl-4-iodo-1-(2-méthoxy-éthoxyméthoxy)-benzène sont obtenus.

### 12.3 : acide 3-tert-butyl-4-(2-méthoxy-éthoxyméthoxy)-benzène boronique

9,5ml (24 mmoles) d'une solution de butyllithium dans l'hexane de concentration 2,5M sont additionnés à une solution, préalablement refroidie à -78°C, de 7,2g (20 mmoles) de 2-tert-butyl-4-iodo-1-(2-méthoxy-éthoxyméthoxy)-benzène dans 72ml de tétrahydrofurane. Après agitation à -78°C pendant 1 heure, 5ml (22 mmoles) de tri-isopropylborate sont additionnés goutte à goutte. Le milieu réactionnel est agité de -78°C à température ambiante pendant 2 heures. Le milieu réactionnel est à nouveau refroidi à 0°C puis une solution aqueuse saturée de chlorure d'ammonium est ajoutée et le milieu est agité pendant 1 heure puis extrait à l'acétate d'éthyle. La phase organique est séchée sur sulfate de sodium, filtrée, évaporée. 5,5g (100%) d'acide 3-tert-butyl-4-(2-méthoxy-éthoxyméthoxy)-benzène boronique sont obtenus.

### 12.4 : 4-{5-[3-tert-butyl-4-(2-méthoxy-éthoxyméthoxy)-phényl]-thiazol-2-yl}-benzoate d'éthyle

De manière analogue à l'exemple 10.5, à partir de 4,7g (15 mmoles) de 4-(5-bromo-thiazol-2-yl)-benzoate d'éthyle (préparé comme décrit en 10.4) et de 5,5g (20 mmoles) d' acide 3-tert-butyl-4-(2-méthoxy-éthoxyméthoxy)-benzène boronique, 4,9g (69%) de 4-{5-[3-tert-butyl-4-(2-méthoxy-éthoxyméthoxy)-phényl]-thiazol-2-yl}-benzoate d'éthyle sont obtenus.

### 12.5 : 4-[5-(3-tert-butyl-4-hydroxy-phényl)-thiazol-2-yl]-benzoate d'éthyle

De manière analogue à l'exemple 11.1, à partir de 4,9g (10 mmoles) de 4-{5-[3-tert-butyl-4-(2-méthoxy-éthoxyméthoxy)-phényl]-thiazol-2-yl}-benzoate d'éthyle, 2,5g (63%) de 4-[5-(3-tert-butyl-4-hydroxy-phényl)-thiazol-2-yl]-benzoate d'éthyle sont obtenus.

### 12.6 : 4-[5-(3-tert-butyl-4-méthoxy-phényl)-thiazol-2-yl]-benzoate d'éthyle

De manière analogue à l'exemple 9.1, à partir de 200mg (0,5 mmole) de 4-[5-(3-tert-butyl-4-hydroxy-phényl)-thiazol-2-yl]-benzoate d'éthyle et de 100µl (1,6 mmole) d'iodure de méthyle, 191mg (92%) de 4-[5-(3-tert-butyl-4-méthoxy-phényl)-thiazol-2-yl]-benzoate d'éthyle sont obtenus.

### 12.7 : acide 4-[5-(3-tert-butyl-4-méthoxy-phényl)-thiazol-2-yl]-benzoique

De manière analogue à l'exemple 9.2, à partir de 187mg (0,45 mmoles) de 4-[5-(3-tert-butyl-4-méthoxy-phényl)-thiazol-2-yl]-benzoate d'éthyle, 130mg (74%) d' acide 4-[5-(3-tert-butyl-4-méthoxy-phényl)-thiazol-2-yl]-benzoique sont obtenus sous la forme d'une poudre jaune.

### Exemple 13: acide 4-{5-[4-hydroxy-3-(1-méthyl-cyclohexyl)-phényl]-thiazol-2-yl}-benzoique

### 13.1 : acide 4- éthoxyméthoxy-3-(1-méthyl-cyclohexyl)-benzène boronique

De manière analogue à l'exemple 12.3, à partir de 5,7g (16 mmoles) de 4-bromo-1-éthoxyméthoxy-2-(1-méthyl-cyclohexyl)-benzène (préparé comme décrit en 5.2), 5g (98%) d'acide 4- éthoxyméthoxy-3-(1-méthyl-cyclohexyl)-benzène boronique sont obtenus.

### 13.2 : 4-{5-[4-(2-méthoxy-éthoxyméthoxy)-3-(1-méthyl-cyclohexyl)-phényl]-thiazol-2-yl}-benzoate d'éthyle

De manière analogue à l'exemple 10.5, à partir de 0,8g (2,4 mmoles) de 4-(5-bromo-thiazol-2-yl)-benzoate d'éthyle (préparé comme décrit en 10.4) et de 1g (2,4 mmoles) d' acide 4-éthoxyméthoxy-3-(1-méthyl-cyclohexyl)-benzène boronique, 1g (66%) de 4-{5-[4-(2-méthoxy-éthoxyméthoxy)-3-(1-méthyl-cyclohexyl)-phényl]-thiazol-2-yl}-benzoate d'éthyle sont obtenus.

### 13.3 : 4-{5-[4-hydroxy-3-(1-méthyl-cyclohexyl)-phényl]-thiazol-2-yl}-benzoate d'éthyle

De manière analogue à l'exemple 11.1, à partir de 1g (2 mmoles) de 4-{5-[4-(2-méthoxy-éthoxyméthoxy)-3-(1-méthyl-cyclohexyl)-phényl]-thiazol-2-yl}-benzoate d'éthyle, 550mg (40%) de 4-{5-[4-hydroxy-3-(1-méthyl-cyclohexyl)-phényl]-thiazol-2-yl}-benzoate d'éthyle sont obtenus.

### 13.4 : acide 4-{5-[4-hydroxy-3-(1-méthyl-cyclohexyl)-phényl]-thiazol-2-yl}-benzoique

De manière analogue à l'exemple 9.2, à partir de 170mg (0,4 mmole) de 4-{5-[4-hydroxy-3-(1-méthyl-cyclohexyl)-phényl]-thiazol-2-yl}-benzoate d'éthyle, 140mg (87%) d'acide 4-{5-[4-hydroxy-3-(1-méthyl-cyclohexyl)-phényl]-thiazol-2-yl}-benzoique sont obtenus sous forme d'un solide jaune.

### Exemple 14 : acide 4-[5-(5,5,8,8-tétraméthyl-5,6,7,8-tétrahydro-naphthalen-2-yl)-thiazol-2-yl]-benzoique

### 14.1 : acide 1,1,4,4-tétraméthyl-1,2,3,4-tétrahydro-naphthalène 6-boronique

De manière analogue à l'exemple 12.3, à partir de 5g (19 mmoles) de 6-bromo-1,1,4,4-tétraméthyl-1,2,3,4-tétrahydro-naphthalène, 3,5g (79%) d'acide 1,1,4,4-tétraméthyl-1,2,3,4-tétrahydro-naphthalène 6-boronique sont obtenus.

### 14.2 : 4-[5-(5,5,8,8-tétraméthyl-5,6,7,8-tétrahydro-naphthalèn-2-yl)-thiazol-2-yl]-benzoate d'éthyle

De manière analogue à l'exemple 10.5, à partir de 0,7g (2,2 mmoles) de 4-(5-bromo-thiazol-2-yl)-benzoate d'éthyle (préparé comme décrit en 10.4) et de 0,7g (2,9 mmoles) d'acide 1,1,4,4-tétraméthyl-1,2,3,4-tétrahydro-naphthalène 6-boronique, 0,5g (54%) de 4-[5-(5,5,8,8-tétraméthyl-5,6,7,8-tétrahydro-naphthalèn-2-yl)-thiazol-2-yl]-benzoate d'éthyle sont obtenus.

### 14.3 : acide 4-[5-(5,5,8,8-tétraméthyl-5,6,7,8-tétrahydro-naphthalèn-2-yl)-thiazol-2-yl]-benzoique

De manière analogue à l'exemple 9.2, à partir de 0,5g (1,2 mmole) de 4-[5-(5,5,8,8-tétraméthyl-5,6,7,8-tétrahydro-naphthalèn-2-yl)-thiazol-2-yl]-benzoate d'éthyle, 0,42g (90%) d' acide 4-[5-(5,5,8,8-tétraméthyl-5,6,7,8-tétrahydro-naphthalèn-2-yl)-thiazol-2-yl]-benzoique sont obtenus.

| n° exemple dans partie expérimentale | Structure chimique | nom du composé | Voie de synthèse | RMN 1H | Aspect |
|---|---|---|---|---|---|
| exemple 10 | 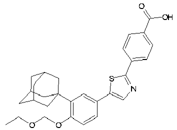 | acide 4-[5-(3-adamantan-1-yl-4-éthoxyméthoxy-phényl)-thiazol-2-yl]-benzoique | E (figure 5) | ¹H NMR (400 MHz, DMSO): δ (ppm) 8.27 (s, 1 H); 8.06-8.07 (m, 4 H); 7.56 (dd, J = 8.5, 2.2 Hz, 1 H); 7.45 (d, J = 2.3 Hz, 1 H); 7.15 (d, J = 8.6 Hz, 1 H); 5.35 (s, 2 H); 3.73 (q, J = 7.1 Hz, 2 H); 2.12 (s, 6 H); 2.08 (d, J = 4.9 Hz, 3 H); 1.76 (s, 6 H); 1.18 (t, J = 7.1 Hz, 3 H). | solide jaune |
| exemple 11 | 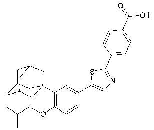 | acide 4-[5-(3-adamantan-1-yl-4-isobutoxy-phényl)-thiazol-2-yl]-benzoique | E (figure 5) | 1H NMR (400 MHz, CDCl₃): δ (ppm) 8.02 (d, J= 8.3 Hz, 2 H); 7.92 (d, J = 8.3 Hz, 2 H); 7.84 (s, 1 H); 7.35 (d, J = 2.4 Hz, 1 H); 7.31 (dd, J = 8.5, 2.3 Hz, 1 H); 6.81 (d, J = 8.4 Hz, 1 H); 3.71 (d, J = 6.2 Hz, 2 H); 2.07 (s, 6 H); 2.01 (s, 3 H); 1.70 (s, 6 H); 1.03 (d, J = 6.7 Hz, 6 H). | solide jaune |
| exemple 12 | 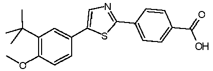 | acide 4-[5-(3-tert-butyl-4-méthoxy-phényl)-thiazol-2-yl]-benzoique | E (figure 5) | ¹H NMR (400 MHz, CDCl₃): δ (ppm) 8.01 (m, 2 H); 7.86 (m, 2 H); 7.80 (d, J = 3.9 Hz, 1 H); 7.35 (s, 1 H); 7.31 (s, 1 H); 6.79 (d, J = 8.3 Hz, 1 H); 3.75 (m, J = 3.7 Hz, 3 H); 1.28 (m, J= 4.0 Hz, 9 H). | solide jaune |
| exemple 13 | 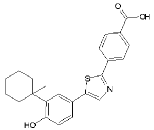 | acide 4-{5-[4-hydroxy-3-(1-méthyl-cyclohexyl)-phényl]-thiazol-2-yl}-benzoique | E (figure 5) | ¹H NMR (400 MHz, DMSO): δ (ppm) 9.82 (s, 1 H); 8.18 (s, 1 H); 8.06 (m, 4 H); 7.42-7.43 (m, 2 H); 6.89 (d, J = 8.0 Hz, 1 H); 2.16 (t, J = 10.0 Hz, 2 H); 1.54-1.66 (m, 8 H); 1.31 (s, 3 H). | solide jaune |
| exemple 14 | 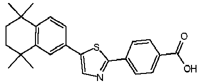 | acide 4-[5-(5,5,8,8-tétraméthyl-5,6,7,8-tétrahydro-naphthalen-2-yl) thiazol-2-yl]-benzoique | E (figure 5) | ¹H NMR (400 MHz, CDCl₃): δ (ppm) 8.19-8.21 (m, 2 H); 8.08-8.10 (m, 2 H); 8.04 (d, J = 1.9 Hz, 1 H); 7.53 (s, 1 H); 7.39 (d, 2 H); 1.73 (s, 4 H); 1.35 (s, 6 H); 1.32 (s, 6 H). | solide jaune |
| | 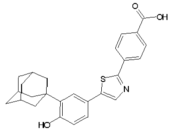 | acide 4-[5-(3-adamantan-1-yl-4-hydroxy-phényl)-thiazol-2-yl]-benzoique | E (figure 5) | ¹H NMR (400 MHz, DMSO): δ (ppm) 13.1 (br s, 1H); 9.79 (s, 1 H); 8.19 (s, 1 H); 8.05 (s, 4 H); 7.42 (dd, J = 8.3, 2.2 Hz, 1 H); 7.36 (d, J = 2.3 Hz, 1 H); 6.87 (d, J = 8.3 Hz, 1 H); 2.12 (s, 6 H); 2.06 (s, 3 H); 1.75 (s, 6 H). | solide jaune |
| | 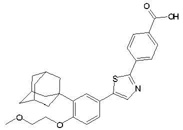 | acide 4-{5-[3-adamantan-1-yl-4-(2-méthoxy-éthoxy)-phényl]-thiazol-2-yl}-benzoique | E (figure 5) | ¹H NMR (400 MHz, CDCl₃): δ (ppm) 8.02 (d, J = 8.2 Hz, 2 H); 7.92 (d, J = 8.2 Hz, 2 H); 7.85 (s, 1H); 7.30-7.33 (m, 2 H); 6.81 (d, J = 8.3 Hz, 1 H); 4.07 (m, J = 4.9 Hz, 2 H); 3.74 (m, J = 4.8 Hz, 2 H); 3.37 (s, 2 H); 2.06 (s, 6 H); 2.01 (s, 3 H); 1.69 (s, 6 H). | solide jaune |
| | 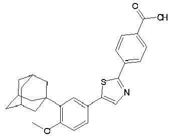 | acide 4-[5-(3-adamantan-1-yl-4-méthoxy-phényl)-thiazol-2-yl]-benzoique | E (figure 5) | ¹H NMR (400 MHz, CDCl₃): δ (ppm) 7.91 (d, J = 8.0 Hz, 2 H); 7.81 (d, J = 7.9 Hz, 2 H); 7.75 (s, 1 H); 7.23 (s, 2 H); 6.74 (d, J = 8.2 Hz, 1 H); 3.69 (s, 3 H); 1.91 (m, J= 12.4 Hz, 9 H); 1.59 (s, 6 H). | solide jaune |
| | 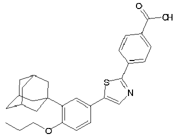 | acide 4-[5-(3-adamantan-1-yl-4-propoxy-phényl)-thiazol-2-yl]-benzoique | E (figure 5) | ¹H NMR (400 MHz, CDCl₃): δ (ppm) 8.00 (d, J = 8.1 Hz, 2 H); 7.89 (d, J = 8.0 Hz, 2 H); 7.82 (s, 1 H); 7.28-7.31 (m, 2 H); 6.79 (d, J = 8.3 Hz, 1 H); 3.87 (t, J = 6.3 Hz, 2 H); 2.04 (s, 6 H); 1.98 (s, 3 H); 1.78-1.81 (m, 2 H); 1.67 (s, 6 H); 1.02 (t, J = 7.4 Hz, 3 H). | solide jaune |
| | 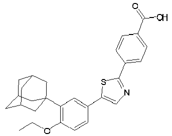 | acide 4-[5-(3-adamantan-1-yl-4-éthoxy-phényl)-thiazol-2-yl]-benzoique | E (figure 5) | ¹H NMR (400 MHz, CDCl₃): δ (ppm) 8.02 (d, J = 8.2 Hz, 2 H); 7.91 (d, J = 8.2 Hz, 2 H); 7.84 (s, 1 H); 7.29-7.32 (m, 2 H); 6.80 (d, J = 8.3 Hz, 1 H); 4.00 (q, J = 6.9 Hz, 2 H); 2.06 (s, 6 H); 2.00 (s, 3 H); 1.69 (s, 6 H); 1.41 (t, J= 6.9 Hz, 3 H). | solide jaune |
| | 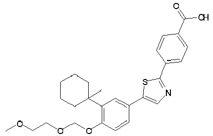 | acide 4-{5-[4-(2-méthoxy-éthoxyméthoxy)-3-(1-méthyl-cyclohexyl)-phényl]-thiazol-2-yl}-benzoique | E (figure 5) | ¹H NMR (400 MHz, CDCl₃): δ (ppm) 8.18 (d, J= 8.2 Hz, 2 H); 8.06 (d, J = 8.2 Hz, 2 H); 7.98 (s, 1 H); 7.54 (d, J = 2.4 Hz, 1 H); 7.40 (dd, J = 8.7, 2.1 Hz, 1 H); 7.24 (d, J = 8.5 Hz, 1 H); 5.35 (s, 2 H); 3.83-3.86 (m, 2 H); 3.60 (m, J = 4.9 Hz, 2 H); 3.39-3.40 (m, 3 H); 2.12 (m, J = 11.3 Hz, 2 H); 1.74 (m, 2 H); 1.40 (m, 6 H); 1.34 (s, 3 H). | solide jaune |
| | 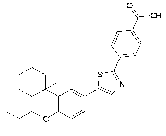 | acide 4-{5-[4-isobutoxy-3-(1-méthyl-cyclohexyl)-phényl]-thiazol-2-yl}-benzoique | E (figure 5) | ¹H NMR (400 MHz, DMSO): δ (ppm) 8.23 (s, 1 H); 8.05 (s, 4 H); 7.54 (d, J = 8.6 Hz, 1 H); 7.50 (d, J = 2.6 Hz, 1 H); 7.05 (d, J = 8.5 Hz, 1 H); 3.82 (d, J = 6.1 Hz, 2 H); 2.17 (s, 2 H); 2.08 (m, 1 H); 1.69 (d, J = 12.4 Hz, 2 H); 1.55 (m, 2 H); 1.44 (, 2 H); 1.32 (s, 3 H); 1.04 (d, J= 6.6 Hz, 6 H). | solide jaune |
| | 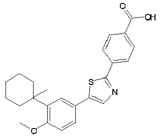 | acide 4-{5-[4-méthoxy-3-(1-méthyl-cyclohexyl)-phényl]-thiazol-2-yl}-benzoique | E (figure 5) | ¹H NMR (400 MHz, DMSO): δ (ppm) 13.2 (br s, 1H); 8.25 (s, 1 H); 8.05-8.07 (m, 4 H); 7.59 (dd, J = 8.4, 2.3 Hz, 1 H); 7.50 (s, 1 H); 7.10 (d, J = 8.5 Hz, 1 H); 3.84 (s, 3 H); 2.09 (t, J= 10.1 Hz, 2 H); 1.72 (d, J= 12.3 Hz, 2 H); 1.37-1.56 (br m, 6 H); 1.29 (s, 3 H). | solide jaune |
| | 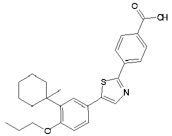 | acide 4-{5-[3-(1-méthyl-cyclohexyl)-4-propoxy-phényl]-thiazol-2-yl}-benzoique | E (figure 5) | 1H NMR (400 MHz, DMSO): δ (ppm) 8.24 (s, 1 H); 8.06 (t, J = 9.3 Hz, 4 H); 7.55 (d, J = 8.6 Hz, 1 H); 7.51 (s, 1 H); 7.06 (d, J = 8.5 Hz, 1 H); 2.17 (m, J = 9.9 Hz, 2 H); 1.77-1.80 (m, 2 H); 1.69 (m, J = 12.6 Hz, 2 H); 1.56 (m, 2 H); 1.45 (m, 2 H); 1.36 (m, J = 10.0 Hz, 2 H); 1.31 (s, 3 H); 1.23 (m, J = 6.9 Hz, 2 H); 1.04 (t, J = 7.4 Hz, 3 H). | solide jaune |
| | 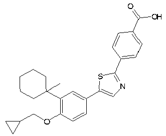 | acide 4-{5-[4-cyclopropylméthoxy-3-(1-méthyl-cyclohexyl)-phényl]-thiazol-2-yl}-benzoique | E (figure 5) | 1H NMR (400 MHz, DMSO): δ (ppm) 8.24 (s, 1 H); 8.06 (t, J = 9.3 Hz, 4 H); 7.55 (d, J = 8.6 Hz, 1 H); 7.51 (s, 1 H); 7.06 (d, J = 8.5 Hz, 1 H); 2.17 (t, J = 9.9 Hz, 2 H); 1.77-1.80 (m, 2 H); 1.69 (d, J = 12.6 Hz, 3 H); 1.56 (br s, 3 H); 1.45 (br s, 5 H); 1.36 (t, J = 10.0 Hz, 3 H); 1.31 (s, 3 H); 1.23 (d, J = 6.9 Hz, 3 H); 1.04 (t, J = 7.4 Hz, 3 H). | solide jaune |
| | 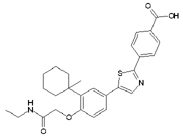 | acide 4-{5-[4-éthylcarbamoylméthoxy-3-(1 -méthyl-cyclohexyl)-phényl]-thiazol-2-yl}-benzoique | E (figure 5) | ¹H NMR (400 MHz, DMSO): δ (ppm) 13.12 (br s, 1 H); 8.26 (s, 1 H); 8.06 (s, 4 H); 7.93 (s, 1 H); 7.57 (d, J = 8.5 Hz, 1 H); 7.52 (s, 1 H); 6.98 (d, J = 8.5 Hz, 1 H); 4.55 (s, 2 H); 3.18 (t, J= 7.3 Hz, 2 H); 2.12 (m, 2 H); 1.74 (m, 2 H); 1.56 (m, 3 H); 1.46 (m, 5 H); 1.34 (s, 3 H); 1.17 (m, 1 H); 1.06 (t, J = 7.3 Hz, 3 H). | solide jaune |
| | 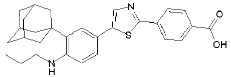 | acide 4-[5-(3-adamantan-1-yl-4-propylamino-phényl)-thiazol-2-yl]-benzoique | E (figure 5) | ¹H NMR (400 MHz, CDCl₃): δ (ppm) 8.06 (d, J = 8.2 Hz, 2 H); 7.95 (d, J = 8.2 Hz, 2 H); 7.85 (s, 1 H); 7.35 (s, 1 H); 7.33 (d, J = 8.7 Hz, 1 H); 6.63 (d, J = 8.4 Hz, 1 H); 3.69 (t, J = 6.0 Hz, 1 H); 3.13 (t, J = 6.9 Hz, 2 H); 2.12 (s, 3 H); 2.06 (s, 6 H); 1.70-1.75 (m, 8 H); 1.03 (t, J= 7.4 Hz, 3 H). | solide jaune |
| | 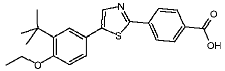 | acide 4-[5-(3-tert-butyl-4éthoxy-phényl)-thiazol-2-yl]-benzoique | E (figure 5) | ¹H NMR (400 MHz, CDCl₃): δ (ppm) 7.99 (d, J = 8.3 Hz, 2 H); 7.88 (d, J = 8.3 Hz, 2 H); 7.81 (s, 1 H); 7.36 (d, J = 2.3 Hz, 1 H); 7.29 (dd, J = 8.4, 2.3 Hz, 1 H); 6.78 (d, J = 8.5 Hz, 1 H); 3.98 (q, J = 7.0 Hz, 2 H); 1.37 (t, J = 6.9 Hz, 3 H); 1.30 (s, 9 H). | solide jaune |
| | 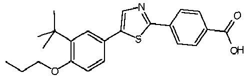 | acide 4-[5-(3-tert-butyl-4-propoxy-phényl)-thiazol-2-yl]-benzoique | E (figure 5) | ¹H NMR (400 MHz, CDCl₃): δ (ppm) 8.04 (d, J = 8.3 Hz, 2 H); 7.94 (d, J = 8.3 Hz, 2 H); 7.86 (s, 1 H); 7.42 (s, 1 H); 7.34 (d, J = 8.7 Hz, 1 H); 6.83 (d, J = 8.6 Hz, 1 H); 3.92 (m, 2 H); 1.82 (m, J = 9.7 Hz, 2 H); 1.36 (s, 9 H); 1.04 (m, 3 H). | solide jaune |
| | 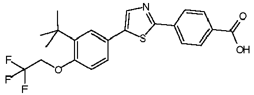 | acide 4-{5-[3-tert-butyl-4-(2,2,2-trifluoro-éthoxy)-phényl]-thiazol-2-yl}-benzoique | E (figure 5) | ¹H NMR (400 MHz, CDCl₃): δ (ppm) 8.04 (d, J = 8.0 Hz, 2 H); 7.94 (d, J = 8.2 Hz, 2 H); 7.88 (s, 1 H); 7.47 (s, 1 H); 7.37 (d, J = 8.3 Hz, 1 H); 6.76 (d, J = 8.4 Hz, 1 H); 4.35 (q, J = 8.0 Hz, 2 H); 1.35 (s, 9 H). | solide jaune |
| | 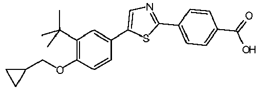 | acide 4-[5-(3-tert-butyl-4-cyclopropylméthoxy-phényl)-thiazol-2-yl] benzoique | E (figure 5) | ¹H NMR (400 MHz, CDCl₃): δ (ppm) 8.08 (d, J = 8.0 Hz, 2 H); 7.96 (d, J = 8.1 Hz, 2 H); 7.89 (s, 1 H); 7.45 (d, J = 2.5 Hz, 1 H); 7.35 (d, J = 8.4 Hz, 1 H); 6.80 (d, J = 8.4 Hz, 1 H); 3.83 (d, J= 6.9 Hz, 2 H); 1.41 (s, 9 H); 0.62 (d, J = 7.7 Hz, 2 H); 0.33 (d, J = 5.1 Hz, 2 H); -0.05 (s, 1 H). | solide jaune |
| | 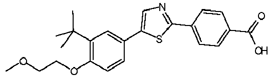 | acide 4-{5-[3-tert-butyl-4-(2-méthoxy-éthoxy)-phény l]-thiazol-2-yl]-benzoique | E (figure 5) | ¹H NMR (400 MHz, CDCl₃): δ (ppm) 8.21 (d, J = 8.1 Hz, 2 H); 8.09 (d, J = 8.1 Hz, 2 H); 8.00 (s, 1 H); 7.54 (s, 1 H); 7.45 (d, J = 8.4 Hz, 1 H); 6.94 (d, J = 8.4 Hz, 1 H); 4.21 (t, J = 4.8 Hz, 2 H); 3.86 (t, J = 4.8 Hz, 2 H); 3.49 (s, 3 H); 1.47 (s, 9 H). | solide jaune |
| | 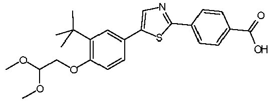 | acide 4-{5-[3-tert-butyl-4-(2,2-diméthoxy-éthoxy)-phényl]-thiazol-2 yl}-benzoique | E (figure 5) | ¹H NMR (400 MHz, CDCl₃): δ (ppm) 8.22 (d, J = 8.1 Hz, 2 H); 8.09 (d, J = 8.1 Hz, 2 H); 8.01 (s, 1 H); 7.55 (s, 1 H); 7.46 (d, J = 8.4 Hz, 1 H); 6.92 (d, J = 8.4 Hz, 1 H); 4.87 (t, J = 5.3 Hz, 1 H); 4.09 (d, J = 5.3 Hz, 2 H); 3.51 (s, 6 H); 1.47 (s, 9 H). | solide jaune |
| | 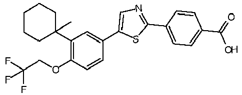 | acide 4-{5-[3-(1-méthyl-cyclohexyl)-4-(2,2,2-trifluoro-éthoxy)-phényl]-thiazol-2-yl}-benzoique | E (figure 5) | ¹H NMR (400 MHz, CDCl₃): δ (ppm) 8.08 (dd, J = 7.8, 2.6 Hz, 2 H); 7.94-7.96 (m, 3 H); 7.53 (s, 1 H); 7.39 (dd, J = 8.3, 2.4 Hz, 1 H); 6.79 (d, J = 8.5 Hz, 1 H); 4.36 (q, J = 8.1 Hz, 2 H); 2.10 (br s, 2 H); 1.68 (br s, 2 H); 1.56 (brs, 2 H); 1.37 (s, 3 H); 1.31 (s, 1 H);1.29 (d, J= 2.5 Hz, 3 H); 1.20 (s, 1 H). | solide jaune |
| | 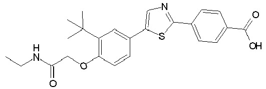 | acide 4-[5-(3-tert-butyl-4-éthylcarbamoylméthoxy-phényl)-thiazol-2-yl]-benzoique | E (figure 5) | ¹H NMR (400 MHz, CDCl₃): δ (ppm) 8.20 (d, J = 8.2 Hz, 2 H); 8.07 (d, J = 8.2 Hz, 2 H); 8.00 (s, 1 H); 7.57 (d, J = 2.3 Hz, 1 H); 7.46 (dd, J = 8.4, 2.2 Hz, 1 H); 6.89 (d, J = 8.5 Hz, 1 H); 6.48 (s, 1 H); 4.61 (s, 2 H); 3.44 (p, J = 6.8 Hz, 2 H); 1.49 (s, 9 H); 1.21 (t, J= 7.3 Hz, 3 H). | solide jaune |
| | 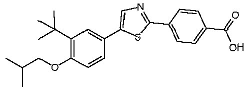 | acide 4-[5-(3-tert-butyl-4-isobutoxy-phényl)-thiazol-2-yl]-benzoique | E (figure 5) | ¹H NMR (400 MHz, CDCl₃): δ (ppm) 8.19 (d, J = 8.2 Hz, 2 H); 8.07 (d, J = 8.2 Hz, 2 H); 7.96 (s, 1 H); 7.52 (d, J = 2.3 Hz, 1 H); 7.43 (dd, J = 8.4, 2.3 Hz, 1 H); 6.91 (d, J = 8.5 Hz, 1 H); 3.82 (d, J = 6.3 Hz, 2 H); 2.18-2.20 (m, 1 H); 1.46 (s, 9 H); 1.11 (d, J = 6.7 Hz, 6 H). | solide jaune |
| | 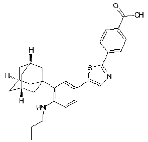 | acide 4-[5-(3-adamantan-1-yl-4-propylamino-phényl)-thiazol-2-yl]-benzoique | E (figure 5) | | |
| | 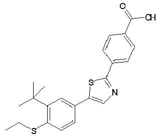 | acide 4-[5-(3-tert-butyl-4-éthylsulfanyl-phényl)-thiazol-2-yl]-benzoique | E (figure 5) | | |
| | 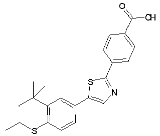 | acide 4-[5-(3-tert-butyl-4-éthylsulfanyl-phényl)-thiazol-2-yl]-benzoique | | | |
| | 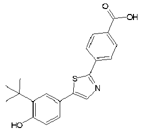 | acide 4-[5-(3-tert-butyl-4-hydroxy-phényl)-thiazol-2-yl]-benzoique | E (figure 5) | | |

**F : description des composés hétéroaryles décrits dans la figure 6,7 et 8**

| CHEMISTRY | nom du composé |
|---|---|
| 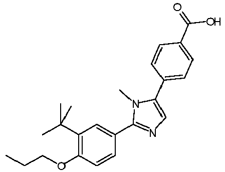 | acide 4-[2-(3-tert-Butyl-4-propoxy-phenyl)-3-methyl-3H-imidazol-4-yl]-benzoique |
| 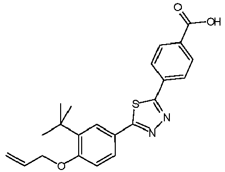 | 4-[5-(4-Allyloxy-3-tert-butyl-phenyl)-[1,3,4]thiadiazol-2-yl]-benzoic acid |
| 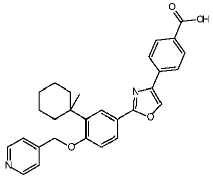 | 4-{2-[3-(1-Methyl-cyclohexyl)-4-(pyridin-4-ylmethoxy)-phenyl]-oxazol-4-yl}-benzoic acid |
| 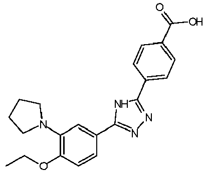 | 4-[5-(4-Ethoxy-3-pyrrolidin-1-yl-phenyl)-4H-[1 ,2,4 ]triazol-3-yl]-benzoic acid |
| 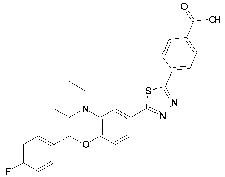 | 4-{5-[3-Diethylamino-4-(4-fluoro-benzyloxy)-phenyl]-[1,3,4]thiadiazol-2-yl}-benzoic acid |
| 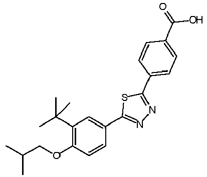 | 4-[5-(3-tert-Butyl-4-isobutoxy-phenyl)-[1,3,4]thiadiazol-2-yl]-benzoic acid |
| 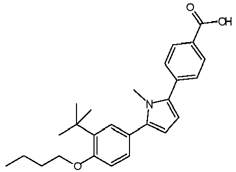 | 4-[5-(4-Butoxy-3-tert-butyl-phenyl)-1 -methyl-1H-pyrrol-2-yl]-benzoic acid |
| 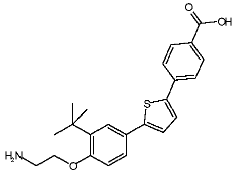 | 4-{5-[4-(2-Amino-ethoxy)-3-tert-butyl-phenyl]-thiophen-2-yl}-benzoic acid |
| 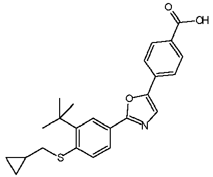 | acide 4-[2-(3-tert-Butyl-4-cyclopropylmethylsulfanyl-phenyl)-oxazol-5-yl]-benzoique |
| 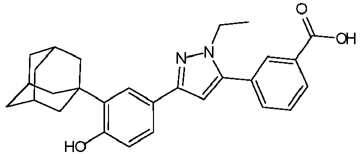 | 3-[5-(3-Adamantan-1-yl-4-hydroxy-phenyl)-2-ethyl-2H-pyrazol-3-yl]-benzoic acid |

### G : test de pharmacologie moléculaire

### Principe :

L'activité de nos molécules sur le récepteur RAR est mesurée à l'aide d'une lignée recombinante exprimant le "ligand-biding domain (LBD)" de RAR fusionné au "DNA binding domain (DBD)" du récepteur aux estrogènes. La transactivation spécifique du récepteur sur l'élément de réponse du récepteur aux estrogènes (ERE) entraine la transcription de la luciférase. L'expression de ce gène reporter est quantifiée par luminescence après ajout de son substrat, la luciférine.

Ces lignée ont été produites par le Pr Ballaguer (INSERM 439, Montpellier) après transfection stable de cellules HeLa par les plasmides ERE-βGlob-Luc-SV-Neo et RAR(α, β, γ)-ER-DBD-puro.

Les composes sont évalués en doses-réponses (10000nM-0.04nM) normalisées par rapport à un contrôle basal et un contrôle d'efficacité maximale (CD0193, agoniste de référence à concentration saturante 100nM) : Y=((X-0%)/(100%-0%))*100.

L'affinité (EC₅₀ en nM) et l'efficacité (en %) sont mesurées à l'aide du logiciel XLfit (IDBS) après une régression non-linéaire à quatre paramètres (modèle 205).

### Protocole :

Les cellules HeLa ERE Luc-hRAR sont ensemencées en microplaques 96 puits (10 000 cellules / 100µL). Après 4 heures d'incubation à 37°C / 5% CO2, elles sont traitées par 5µL de produits 20X (0.1% DMSO final). Après 18 heures d'incubation à 37°C / 5% CO2, 100µL de solution de révélation (luciférine en tampon de lyse) sont ajoutés et la luminescence est mesurée dans un lecteur de microplaques.

**Résultats :**

| **Nom du composé** | **RARα** | | | **RARβ** | | | **RARγ** | | |
|---|---|---|---|---|---|---|---|---|---|
| | **EC₅₀ (nM)** | **Efficacité (%)** | **Commentaire** | **EC₅₀ (nM)** | **Efficacité (%)** | **Commentaire** | **EC₅₀ (nM )** | **Efficacité (%)** | **Commentaire** |
| Acide 4-[5-(5,5,8,8-tétraméthyl -5,6,7,8-tetrahydro-naphthalen-2-yl)-thiazol-2-yl]-benzoique | 9793 | 64 | Agoniste total | 52 | 110 | Agoniste total | 162 | 78 | Agonis te total |
| Acide 4-[3-(3-tert-butyl-4-ethoxymethoxy-phenyl)-[1,2,4]oxadiazol-5-yl]-benzoique | 650 | 104 | Agoniste total | 6.2 | 95 | Agoniste total | 10 | 88 | Agonis te total |
| 4-[3-(3-tert-butyl-4-hydroxy-phenyl)-[1,2,4]oxadiazol-5-yl]-benzoique | 107 | 106 | Agoniste total | 3.0 | 110 | Agoniste total | 2.4 | 110 | Agonis te total |
| Acide 4-{3-[4-éthoxyméth oxy-3-(1-méthyl-cyclohexyl)-phenyl]-[1,2,4]oxadi azol-5-yl}-benzoique | 90 | 80 | Agoniste total | 0.56 | 107 | Agoniste total | 0.90 | 102 | Agonis te total |
| Acide 4-[3-(3-tert-butyl-4-éthoxy-phenyl)-[1,2,4]oxadiazol-5-yl]-benzoique | 255 | 105 | Agoniste total | 3.8 | 92 | Agoniste total | 9.3 | 96 | Agonis te total |
| Acide 4-{5-[3-(1-méthyl-cyclohexyl)-4-propoxy-phenyl]-thiazol-2-yl}-benzoique | 3175 | 68 | Agoniste total | 635 | 90 | Agoniste total | 1033 | 74 | Agonis te total |
| Acide 4-[5-(3-tert-butyl-4-méthoxy-phenyl)-thiazol-2-yl]-benzoique | 109 | 53 | Agoniste partiel | 164 | 46 | Agoniste partiel | 66 | 40 | Agonis te partiel |

| | | | | | | | | | |
|---|---|---|---|---|---|---|---|---|---|
| *(^{∗}) IA = Inactif (^{∗∗}) ND = EC₅₀ non déterminable* | | | | | | | | | |

L'acide 4-[5-(3-tert-butyl-4-méthoxy-phenyl)-thiazol-2-yl]-benzoique est agoniste partiel sur les trois isoformes RAR.

Les autres composés présentés sont agonistes totaux sur les trois isoformes RAR avec une gamme d'activité s'étendant de 10⁻⁹ à 10⁻⁶M. Ils sont préférentiellement actifs sur RAR beta et gamma.

## Revendications

1. Composé de formule (I) 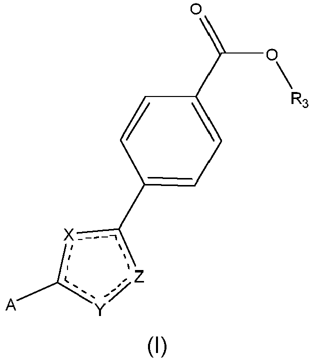 dans laquelle :
A représente 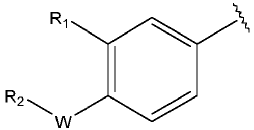
R1 représente un radical alkyle ramifié, ou un radical NR₄R₅;
R2 est un atome d'hydrogène, un radical alkyle linéaire ou ramifié, un radical monohydroxyalkyle ;
R3 est un hydrogène ;
R4 et R5, identiques ou différents, représentent un radical alkyle linéaire ou ramifié ;
R4 et R5, pris ensemble, pouvant également être liés et former un hétérocycle de type pyrrolidine avec l'atome d'azote auquel ils sont liés, cet hétérocycle pouvant éventuellement être substitué ;
W représente O, S, NH ou CH₂;
X, Y, Z, identiques ou différents représentent O, ou N ;
l'hétérocycle central 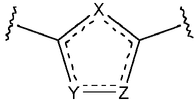 correspond à l'une des structures présentées ci-dessous, les liaisons en pointillée pouvant correspondre à une simple ou double liaison en fonction de la nature des atomes X, Y et Z et de leur covalence 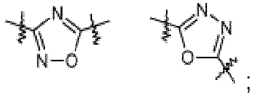
ainsi que leurs sels d'addition avec un acide pharmaceutiquement acceptable, leurs sels d'addition avec une base pharmaceutiquement acceptable et leurs énantiomères.

2. Composé selon la revendication 1, dans lequel le composé est choisi parmi les composés suivants :
acide 4-[3-(3-tert-butyl-4-hydroxy-phényl)-[1,2,4]oxadiazol-5-yl]-benzoique ;
acide 4-[3-(3-tert-butyl-4-méthoxy-phényl)-[1,2,4]oxadiazol-5-yl]-benzoique;
acide 4-[3-(3-tert-butyl-4-éthoxy-phényl)-[1,2,4]oxadiazol-5-yl]-benzoique;
acide 4-[3-(3-tert-butyl-4-propoxy-phényl)-[1,2,4]oxadiazol-5-yl]-benzoique;
acide 4-[3-(3-tert-butyl-4-isobutoxy-phényl)-[1,2,4]oxadiazol-5-yl]-benzoique;
acide 4-{3-[3-tert-butyl-4-(2-hydroxy-éthoxy)-phényl]-[1,2,4]oxadiazol-5-yl}-benzoique;
acide 4-{3-[3-tert-butyl-4-(3-hydroxy-propoxy)-phényl]-[1,2,4]oxadiazol-5-yl}-benzoique;
acide 4-[3-(3-tert-butyl-4-propyl-phényl)-[1,2,4]oxadiazol-5-yl]-benzoique;
acide 4-[5-(3-tert-butyl-4-hydroxy-phenyl)-[1,3,4]oxadiazol-2-yl]-benzoique; et
acide 4-[5-(3-tert-butyl-4-éthoxy-phenyl)-[1,3,4]oxadiazol-2-yl]-benzoique.

3. Composé selon la revendication 1, dans lequel le composé est choisi parmi les composés suivants :
Acide 4-[3-(3-tert-butyl-4-hydroxy-phenyl)-[1,2,4]oxadiazol-5-yl]-benzoique; et
Acide 4-[3-(3-tert-butyl-4-éthoxy-phenyl)-[1,2,4]oxadiazol-5-yl]-benzoique.

4. Composé selon la revendication 1, dans lequel le composé est l'acide 4-[3-(3-tert-butyl-4-hydroxy-phenyl)-[1 ,2,4 ]oxadiazol-5-yl]-benzoique.

5. Composé selon la revendication 1, dans lequel le composé est l'acide 4-[3-(3-tert-butyl-4-méthoxy-phenyl)-[1 ,2,4 ]oxadiazol-5-yl]-benzoique.

6. Composé selon la revendication 1, dans lequel le composé est l'acide 4-[3-(3-tert-butyl-4-éthoxy-phenyl)-[1 ,2,4 ]oxadiazol-5-yl]-benzoique.

7. Composé selon la revendication 1, dans lequel le composé est l'acide 4-[3-(3-tert-butyl-4-propoxy-phenyl)-[1 ,2,4 ]oxadiazol-5-yl]-benzoique.

8. Composé selon la revendication 1, dans lequel le composé est l'acide 4-[3-(3-tert-butyl-4-isobutoxy-phenyl)-[1 ,2,4 ]oxadiazol-5-yl]-benzoique.

9. Composition pharmaceutique comprenant un composé selon l'une quelconque des revendications 1 à 8.

10. Composé selon l'une quelconque des revendications 1 à 8, pour son utilisation à titre de médicament.

11. Composé selon l'une quelconque des revendications 1 à 8, pour son utilisation dans le traitement d'une maladie liée à un récepteur nucléaire de l'acide rétinoïque, dans laquelle la maladie liée à un récepteur nucléaire de l'acide rétinoïque étant choisie parmi :
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose ;
5) les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanée telle que les kératoacanthomes ;
6) les désordres dermatologiques tels que les dermatoses immunes telles le lupus érythémateux, les maladies immunes bulleuses et les maladies du collagène, telle la sclérodermie ;
7) les affections dermatologiques ou générales à composante immunologique ;
8) les troubles ophtalmologiques, notamment les cornéopathies,
9) les stigmates de l'atrophie épidermique et/ou dermique induite par les corticostéroïdes locaux ou systémiques, ou toute autre forme d'atrophie cutanée,
10) le traitement de toute affection d'origine virale au niveau cutané ou général,
11) pour lutter contre les troubles de la fonction sébacée tels que l'hyperséborrhée de l'acné ou la séborrhée simple ;
12) les troubles de la cicatrisation, ou pour prévenir ou pour réparer les vergetures, ou encore pour favoriser la cicatrisation,
13) les désordres de la pigmentation, tel l'hyperpigmentation, le mélasma, l'hypopigmentation ou le vitiligo ;
16) les états cancéreux ou précancéreux ;
17) l'alopécie de différentes origines, notamment l'alopécie due à la chimiothérapie ou aux rayonnements.

## Patentansprüche

1. Verbindung der Formel (I) 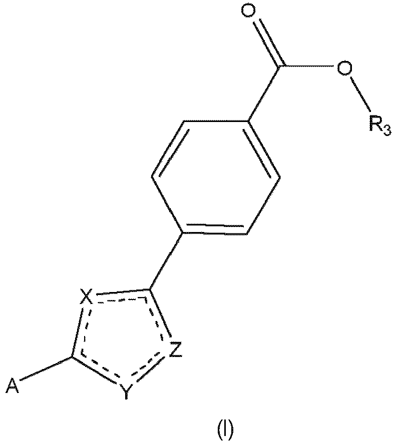 wobei:
A 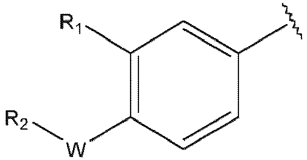 darstellt;
R₁ einen verzweigten Alkylrest oder einen NR₄R₅-Rest darstellt;
R₂ ein Wasserstoffatom, ein linearer oder verzweigter Alkylrest, ein Monohydroxyalkylrest ist;
R₃ Wasserstoff ist;
R₄ und R₅, gleich oder verschieden, einen linearen oder verzweigten Alkylrest darstellen;
R₄ und R₅ zusammengenommen, auch verknüpft sein können und einen Heterocyclus des Pyrrolidin-Typs mit dem Stickstoffatom, an das sie gebunden sind, bilden können, wobei dieser Heterocyclus möglicherweise substituiert ist;
W O, S, NH oder CH₂ darstellt;
X, Y, Z gleich oder verschieden sind, und O oder N darstellen;
der zentrale Heterocyclus 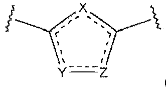 einer der unten dargestellten Strukturen entspricht, wobei die punktierten Bindungen wahlweise einer Einfach- oder Doppelbindung entsprechen, je nach Art der X-, Y- und Z-Atome und ihrer Kovalenz 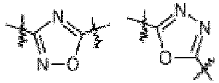
sowie ihre Additionssalze mit einer pharmazeutisch verträglichen Säure, ihre Additionssalze mit einer pharmazeutisch verträglichen Base und ihre Enantiomere.

2. Verbindung nach Anspruch 1, wobei die Verbindung aus den folgenden Verbindungen ausgewählt ist:
4-[3-(3-tert-Butyl-4-hydroxy-phenyl)-[1,2,4]-oxadiazol-5-yl]-benzoesäure;
4-[3-(3-tert-Butyl-4-methoxy-phenyl)-[1,2,4]-oxadiazol-5-yl]-benzoesäure;
4-[3-(3-tert-Butyl-4-ethoxy-phenyl)-[1,2,4]-oxadiazol-5-yl]-benzoesäure;
4-[3-(3-tert-Butyl-4-propoxy-phenyl)-[1,2,4]-oxadiazol-5-yl]-benzoesäure;
4-[3-(3-tert-Butyl-4-isobutoxy-phenyl)-[1,2,4]-oxadiazol-5-yl]-benzoesäure;
4-{3-[3-tert-Butyl-4-(2-hydroxy-ethoxy)-phenyl]-[1,2,4]-oxadiazol-5-yl}-benzoesäure;
4-{3-[3-tert-Butyl-4-(3-hydroxy-propoxy)-phenyl]-[1,2,4]-oxadiazol-5-yl}-benzoesäure;
4-[3-(3-tert-Butyl-4-propyl-phenyl)-[1,2,4]-oxadiazol-5-yl]-benzoesäure;
4-[5-(3-tert-Butyl-4-hydroxy-phenyl)-[1,3,4]-oxadiazol-2-yl]-benzoesäure; und
4-[5-(3-tert-Butyl-4-ethoxy-phenyl)-[1,3,4]-oxadiazol-2-yl]-benzoesäure.

3. Verbindung nach Anspruch 1, wobei die Verbindung aus den folgenden Verbindungen ausgewählt ist:
4-[3-(3-tert-Butyl-4-hydroxy-phenyl)-[1,2,4]-oxadiazol-5-yl]-benzoesäure; und
4-[3-(3-tert-Butyl-4-ethoxy-phenyl)-[1,2,4]-oxadiazol-5-yl]-benzoesäure.

4. Verbindung nach Anspruch 1, wobei die Verbindung 4-[3-(3-tert-Butyl-4-hydroxy-phenyl)-[1,2,4]-oxadiazol-5-yl]-benzoesäure ist.

5. Verbindung nach Anspruch 1, wobei die Verbindung 4-[3-(3-tert-Butyl-4-methoxy-phenyl)-[1,2,4]-oxadiazol-5-yl]-benzoesäure ist.

6. Verbindung nach Anspruch 1, wobei die Verbindung 4-[3-(3-tert-Butyl-4-ethoxy-phenyl)-[1,2,4]-oxadiazol-5-yl]-benzoesäure ist.

7. Verbindung nach Anspruch 1, wobei die Verbindung 4-[3-(3-tert-Butyl-4-propoxy-phenyl)-[1,2,4]-oxadiazol-5-yl]-benzoesäure ist.

8. Verbindung nach Anspruch 1, wobei die Verbindung 4-[3-(3-tert-Butyl-4-isobutoxy-phenyl)-[1,2,4]-oxadiazol-5-yl]-benzoesäure ist.

9. Pharmazeutische Zusammensetzung, umfassend eine Verbindung nach einem der Ansprüche 1 bis 8.

10. Verbindung nach einem der Ansprüche 1 bis 8 zur Verwendung als Arzneimittel.

11. Verbindung nach einem der Ansprüche 1 bis 8 zur Verwendung bei der Behandlung einer Krankheit, die mit einem nukleären Retinsäurerezeptor verbunden ist, wobei die Krankheit, die mit einem nukleären Retinsäurerezeptor verbunden ist, ausgewählt ist aus:
1) dermatologischen Erkrankungen im Zusammenhang mit einer Keratinisierungsstörung in Bezug auf Differenzierung und Zellproliferation, insbesondere zur Behandlung von gewöhnlicher Akne, Komedonen, Polymorphen, Rosacea, nodulozystischer Akne, Konglobata, seniler Akne, sekundärer Akne wie Sonnenakne, medizinisch oder beruflich;
2) Verhornungsstörungen, insbesondere lchthyose, ichthyosiforme Zustände, lamellarer lchthyose, Morbus Darrier, palmoplantare Keratodermie, Leukoplakie und leukoplakiformen Zuständen, Haut- oder Schleimhautflechte (bukkale);
3) dermatologischen Erkrankungen mit entzündlicher immunallergischer Komponente, mit oder ohne Zellproliferationsstörung, insbesondere allen Formen der Psoriasis, ob kutan, schleimig oder ungual, bis hin zu Psoriasis-Arthritis, oder auch Hautatopie, wie Ekzem oder respiratorischer Atopie oder gingivaler Hypertrophie;
4) Hauterkrankungen aufgrund der Exposition gegenüber UV-Strahlung sowie zur Reparatur oder Bekämpfung der Hautalterung, sei es lichtbedingt oder chronologisch oder zur Verringerung von Pigmentierungen und aktinischen Keratosen oder jeglicher Pathologie, die mit der chronologischen oder aktinischen Alterung verbunden ist, wie die Xerose;
5) gutartigen oder bösartigen dermalen oder epidermalen Proliferationen, auch viralen Ursprungs, wie z. B. gewöhnliche Warzen, flache Warzen und verruziforme Epidermodysplasie, orale oder floride Papillomatose, T-Lymphome und Proliferationen, die durch ultraviolette Strahlen induziert werden können, insbesondere in im Fall von basalen und Plattenepitheliomen sowie jeder präkanzerösen Hautläsion wie Keratoakanthome;
6) dermatologischen Erkrankungen wie Immundermatosen wie Lupus erythematodes, bullöse Immunerkrankungen und Kollagenerkrankungen wie Sklerodermie;
7) dermatologischen oder allgemeinen Erkrankungen mit einer immunologischen Komponente;
8) ophthalmologischen Erkrankungen, insbesondere Corneopathien,
9) den Stigmata der epidermalen und/oder dermalen Atrophie, die durch lokale oder systemische Kortikosteroide oder jede andere Form von Hautatrophie induziert wird,
10) der Behandlung von Krankheiten viralen Ursprungs auf kutaner oder allgemeiner Ebene,
11) zur Bekämpfung von Talgdrüsenfunktionsstörungen wie Akne Hyperseborrhoe oder einfacher Seborrhoe;
12) Heilungsstörungen oder zur Vorbeugung oder Reparatur von Dehnungsstreifen oder zur Förderung der Heilung,
13) Pigmentierungsstörungen, wie Hyperpigmentierung, Melasma, Hypopigmentierung oder Vitiligo;
16) krebsartigen oder präkanzerösen Zuständen;
17) Alopezie unterschiedlichen Ursprungs, insbesondere Alopezie durch Chemotherapie oder Bestrahlung.

## Claims

1. A compound of formula (I) 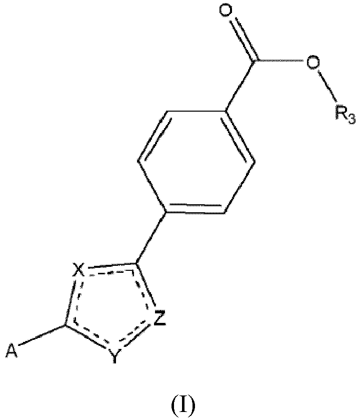 wherein:
A represents 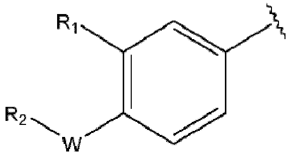
R1 represents a branched alkyl radical, or a radical NR₄R₅;
R2 is a hydrogen atom, a linear or branched alkyl radical, a monohydroxyalkyl radical;
R3 is a hydrogen;
R4 and R5, identical or different, represent a linear or branched alkyl radical;
R4 and R5, taken together, may also be bound and form a pyrrolidine-type heterocycle with the nitrogen atom to which they are bound, this heterocycle which may optionally be substituted;
W represents O, S, NH or CH₂;
X, Y, Z, identical or different, represent O, or N;
the central heterocycle 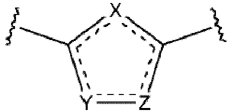 corresponds to one of the structures presented below, the dotted bonds which may correspond to a single or double bond according to the nature of the atoms X, Y and Z and their covalency 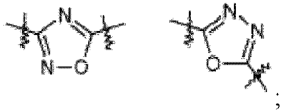
as well as their addition salts with a pharmaceutically acceptable acid, their addition salts with a pharmaceutically acceptable base and their enantiomers.

2. The compound as claimed in claim 1, wherein the compound is selected from the following compounds:
4-[3-(3-tert-butyl-4-hydroxy-phenyl)-[1,2,4]oxadiazol-5-yl]-benzoic acid;
4-[3-(3-tert-butyl-4-methoxy-phenyl)-[1,2,4]oxadiazol-5-yl]-benzoic acid;
4-[3-(3-tert-butyl-4-ethoxy-phenyl)-[1,2,4]oxadiazol-5-yl]-benzoic acid;
4-[3-(3-tert-butyl-4-propoxy-phenyl)-[1,2,4]oxadiazol-5-yl]-benzoic acid;
4-[3-(3-tert-butyl-4-isobutoxy-phenyl)-[1,2,4]oxadiazol-5-yl]-benzoic acid;
4-{3-[3-tert-butyl-4-(2-hydroxy-ethoxy)-phenyl]-[1,2,4]oxadiazol-5-yl}-benzoic acid;
4-{3-[3-tert-butyl-4-(3-hydroxy-propoxy)-phenyl]-[1,2,4]oxadiazol-5-yl}-benzoic acid;
4-[3-(3-tert-butyl-4-propyl-phenyl)-[1,2,4]oxadiazol-5-yl]-benzoic acid;
4-[5-(3-tert-butyl-4-hydroxy-phenyl)-[1,3,4]oxadiazol-2-yl]-benzoic acid; and
4-[5-(3-tert-butyl-4-ethoxy-phenyl)-[1,3,4]oxadiazol-2-yl]-benzoic acid.

3. The compound as claimed in claim 1, wherein the compound is selected from the following compounds:
4-[3-(3-tert-butyl-4-hydroxy-phenyl)-[1,2,4]oxadiazol-5-yl]-benzoic acid; and
4-[3-(3-tert-butyl-4-ethoxy-phenyl)-[1,2,4]oxadiazol-5-yl]-benzoic acid.

4. The compound as claimed in claim 1, wherein the compound is 4-[3-(3-tert-butyl-4-hydroxy-phenyl)-[1,2,4]oxadiazol-5-yl]-benzoic acid.

5. The compound as claimed in claim 1, wherein the compound is 4-[3-(3-tert-butyl-4-methoxy-phenyl)-[1,2,4]oxadiazol-5-yl]-benzoic acid.

6. The compound as claimed in claim 1, wherein the compound is 4-[3-(3-tert-butyl-4-ethoxy-phenyl)-[1,2,4]oxadiazol-5-yl]-benzoic acid.

7. The compound as claimed in claim 1, wherein the compound is 4-[3-(3-tert-butyl-4-propoxy-phenyl)-[1,2,4]oxadiazol-5-yl]-benzoic acid.

8. The compound as claimed in claim 1, wherein the compound is 4-[3-(3-tert-butyl-4-isobutoxy-phenyl)-[1,2,4]oxadiazol-5-yl]-benzoic acid.

9. A pharmaceutical composition comprising a compound as claimed in any one of claims 1 to 8.

10. The compound as claimed in any one of claims 1 to 8, for use as a medicinal product.

11. The compound as claimed in any one of claims 1 to 8, for use in treating a retinoic acid nuclear receptor-related disease, wherein the retinoic acid nuclear receptor-related disease is selected from:
1) dermatological conditions related to a keratinization disorder involving cell differentiation and proliferation, in particular for treating acne vulgaris, comedones, polymorphs, rosacea, nodulocystic acne, conglobata, senile acne, secondary acne such as solar, drug-induced or occupational acne;
2) keratinization disorder, in particular ichthyosis, ichthyosiform states, lamellar ichthyosis, Darier's disease, palmoplantar keratoderma, leukoplakia and leukoplakia-like states, cutaneous or mucosal (oral) lichen;
3) dermatological affections with an inflammatory immuno-allergic component, with or without cell proliferation disorder, and in particular all forms of psoriasis, whether cutaneous, mucous or ungual, and even psoriatic rheumatism, or cutaneous atopy, such as eczema or respiratory atopy, or gingival hypertrophy;
4) skin disorders due to exposure to UV radiation, as well as to repair or combat skin aging, whether photo-induced or chronological, or to reduce pigmentation and actinic keratoses, or any pathologies associated with chronological or actinic aging, such as xerosis;
5) dermal or epidermal proliferations, whether benign or malignant, whether or not of viral origin, such as common warts, flat warts and epidermodysplasia verruciformis, oral or florid papillomatosis, T lymphoma, and proliferations that can be induced by ultraviolet rays, in particular in the case of basal and squamous cell carcinoma, as well as any precancerous skin lesion such as keratoacanthomas;
6) dermatological disorders such as immune dermatoses like lupus erythematosus, immune bullous diseases and collagen diseases, like scleroderma;
7) dermatological or general affections with an immunological component;
8) ophthalmologic disorders, in particular corneal diseases,
9) stigma of epidermal and/or dermal atrophy induced by local or systemic corticosteroids, or any other form of skin atrophy,
10) treatment of any skin or general condition of viral origin,
11) to combat disorders of sebaceous function such as hyperseborrhea associated with acne or simple seborrhea;
12) would healing disorders, or to prevent or repair stretch marks, or to promote wound healing,
13) pigmentation disorders such as hyperpigmentation, melasma, hypopigmentation or vitiligo;
16) cancerous or precancerous conditions;
17) alopecia of various origins, in particular alopecia due to chemotherapy or radiation.
